# EUROPEAN PATENT APPLICATION

(11) **EP 4 458 805 A1**
(43) Date of publication of application: **06.11.2024**
(21) Application number: 23735111.9
(22) Date of filing: 01.01.2023
(51) Int. Cl.: C07C 229/02, C07C 227/08, A61K 31/7088, A61K 39/00, A61K 47/18

(54) **CATIONIC LIPID CONTAINING FUNCTIONAL GROUP ON SIDE CHAIN, AND USE THEREOF**

(30) Priority: 31.12.2021 CN 202111678127
(71) Applicant: Xiamen Sinopeg Biotech Co., Ltd., Xiamen, Fujian 361100 (CN)
(72) Inventor: LIN, Sheng, Xiamen, Fujian 361100 (CN); LIN, Minggui, Xiamen, Fujian 361100 (CN); CHEN, Dandan, Xiamen, Fujian 361100 (CN); WANG, Ailan, Xiamen, Fujian 361100 (CN); LIN, Congming, Xiamen, Fujian 361100 (CN); ZHU, Qi, Xiamen, Fujian 361100 (CN); WENG, Wengui, Xiamen, Fujian 361100 (CN); LIU, Chao, Xiamen, Fujian 361100 (CN); YUAN, Jinchun, Xiamen, Fujian 361100 (CN)
(74) Representative: Algemeen Octrooi- en Merkenbureau B.V.
(86) International application number: PCT/CN2023/070008
(87) International publication number: WO 2023/126006

(57) **Abstract**

The present invention discloses a novel type of cationic lipid and PEGylated derivative thereof, a cationic liposome and a cationic liposome-nucleic acid pharmaceutical composition containing said cationic lipid and formulation thereof. The present invention provides an ionizable lipid compound of the general formula (1). This compound is slightly ionized or neutral at physiological pH but undergoes greater ionization under acidic conditions, exhibiting lower toxicity in the systemic circulation and improved endosomal escape ability. The compound's polar head contains an ionizable tertiary amine group along with a side chain containing functional groups, while the tail chains may include linking groups that are easily degraded. Cationic liposomes containing the compound have a better ability to complex with nucleic acid drugs, higher stability in serum, no apparent cytotoxicity, and high transfection efficiency. Compared with conventional liposomes, the cationic liposomes provided in the present invention has higher efficiency in both nucleic acid delivery and transfection, and can be used as a novel vector or functional reagent for application in nucleic acid drugs and vaccines.

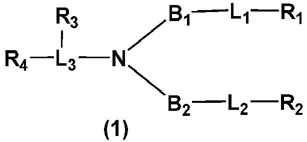

## Description

### CROSS-REFERENCE TO RELATED APPLICATIONS

This application is a U.S. National Phase Application of International Application No. PCT/CN2023/070008, filed Jan. 1, 2023 which claims priority to Chinese Application No. CN202111678127.5, filed Dec. 31, 2021. All of the aforementioned patent applications are hereby incorporated by reference in their entireties.

### TECHNICAL FIELD

The present invention belongs to the field of drug delivery, specifically relates to a cationic lipid that can be used in drug carriers, and particularly relates to a cationic lipid containing side-chain functional groups. The present invention also relates to a lipid composition and a lipid pharmaceutical composition, both containing said cationic lipid, and formulation and application thereof.

### BACKGROUND OF THE INVENTION

Liposomes are widely used for delivering nucleic acid drugs, genetic vaccines, anti-neoplastic drugs, small molecule drugs, peptide drugs, and protein drugs. Especially, as the U.S. Food and Drug Administration (FDA) urgently approved two messenger ribonucleic acid (mRNA) vaccines for vaccination and prevention against the novel coronavirus SARS-CoV-2, in response to the "coronavirus disease 2019" (COVID-19), lipid nanoparticle (LNP) carrying mRNA has become one of the most popular delivery techniques. An LNP can be loaded with mRNA which is negatively charged, while containing also four lipid components including ionizable cationic lipids, neutral co-lipids, steroid lipids, and PEGylated lipids; wherein, the cationic lipids electrostatically interact with the negatively charged mRNA; the neutral co-lipids are usually phospholipids that prevent lipids from oxidation, attach ligands to the surface of liposomes, or reduce the aggregation of lipid particles; steroid lipids have stronger membrane fusogenic properties that facilitate cellular uptake and cytosolic entry of mRNA; PEGylated lipids are located at the surface of lipid nanoparticles to improve hydrophilicity, avoid rapid clearance by the immune system, prevent particle aggregation, and increase stability. Among four lipids used for the preparation of LNP, the ionizable cationic lipid is the most crucial, which is not ionized or slightly ionized under physiological conditions, avoiding too much interference with biofilms in the systemic circulation and thus reducing toxicity. After the cellular uptake, ionizable cationic lipids can be ionized under acidic conditions inside the endosomal lumen to carry a partial positive charge, increasing membrane permeability. Meanwhile, the liposomes are degraded or reorganized, promoting the escape of mRNA from endosomes. The mRNA released into the cytoplasm can be further translated, and thus the delivery and transfection of mRNA is completed.

Lots of LNP pharmaceutical formulations have been approved for market and used in medical fields such as anticancer, antifungals, analgesia, vaccines, etc. Conventional LNP delivery systems have obvious limitations, such as short circulation time in the bloodstream, low stability in vivo, weak complexation with drugs, lower pharmaceutical efficacy, lower transfection efficiency of nucleic acid drugs, etc. Therefore, whether and how to introduce functional groups into the polar head of a cationic lipid and whether the cationic lipid can function synergistically with other lipids are all important questions in solving the aforementioned limitations, especially those on the nanoparticle stability and the transfection efficiency of nucleic acid drugs.

In order to solve the aforementioned problems, a novel cationic lipid and its derivatives are to be developed.

### SUMMARY OF THE INVENTION

The invention provides novel cationic lipids, lipid compositions containing said cationic lipids, lipid pharmaceutical compositions containing said cationic lipids, formulations and applications thereof to be used in the field of drug delivery. Cationic lipids of the present invention have an ionizable polar head group branched via nitrogen; particularly, the polar head group has a side chain that contains functional groups. Lipid compositions prepared with cationic lipids in the present invention have a higher ability to complex with nucleic acid drugs and good stability, which are in favor of sufficient pharmaceutical efficacy of nucleic acid drugs without obvious cytotoxicity and can increase the effectiveness of immunization or therapy.

The above-described purposes of this invention can be realized via embodiments below.

In one embodiment,
provided herein is a cationic lipid of the general formula (1): 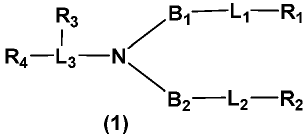
wherein, N is a nitrogen branching center;
L₁ and L₂ are each independently a linking bond or a divalent linking group;
L₃ is a trivalent linking group; L₃ and R₃ form a divalent linking group -L₃(R₃)- with R₃ as the side chain;
B₁ and B₂ are each independently a linking bond or a C₁₋₃₀ alkylene group;
R₁ and R₂ are each independently a C₂₋₃₀ aliphatic hydrocarbon group or a C₂₋₃₀ residue of aliphatic hydrocarbon derivative containing 1-2 O;
R₃ is selected from the group consisting of -OR_{d}, -NR_{d}R_{d}, -SR_{d}, -C(=O)R_{d}, -C(=O)OR_{d}, -OC(=O)R_{d}, -OC(=O)OR_{d}, and 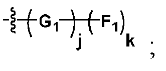 wherein, R_{d} is, at each occurrence, independently a C₁₋₁₂ alkyl group; G₁ is a branching group of valence k+1; j is 0 or 1; F₁ contains the functional group R₀₁; when j is 0, G₁ is absent; when j is 1, G₁ is connected to k F₁, and k F₁ are each independently of the same or different structures, wherein k is an integer in the range of 2-8;
R₄ is selected from the group consisting of a carbocyclyl group, a heterocyclyl group, and R'N(R')-R"-N(R')-; wherein, a heterocyclyl group is a cyclic group having 1, 2, or more heteroatoms in its ring atoms, said heteroatom being B, O, N, Si, P, or S; wherein, R' is, at each occurrence, independently H or a C₁₋₃ alkyl group; R" is a C₂₋₄ alkylene group;
said alkylene group, aliphatic hydrocarbon group, residue of aliphatic hydrocarbon derivative, carbocyclyl group, and heterocyclyl group are each independently substituted or unsub stituted;
or a salt, tautomer, stereoisomer, or solvate thereof.

In another embodiment,
provided herein is a lipid composition containing a cationic lipid of the general formula (1).

In another embodiment,
provided herein is a lipid pharmaceutical composition containing a lipid composition and a drug, said lipid composition containing a cationic lipid of the general formula (1).

In another embodiment,
provided herein is a formulation of lipid pharmaceutical composition containing the aforementioned lipid pharmaceutical composition and pharmaceutically acceptable diluents or excipients.

### Compared with the prior art, the present invention brings the following beneficial effects:

The novel cationic lipid of the present invention is a cationic lipid having a polar head group with a side chain containing functional groups, which enriches the types of cationic lipids, provides more options for constructing lipid nanoparticles, and can be specifically applied in the delivery of nucleic acid drugs, anti-neoplastic drugs, small molecule drugs, peptide drugs, and protein drugs, thus improving the therapeutic and/or diagnostic effects of these drugs as prophylactic agents and/or therapeutic agents. The number, type, and location of functional groups in the side chain of a cationic lipid are all important factors to be considered in regulating the efficiency of cellular uptake, the stability under different physiological conditions, the affinity towards biomolecules, the efficiency of drug delivery, etc., of lipid nanoparticles. In particular, cationic lipids of the present invention can be used synergistically with conventional PEGylated lipids so that lipid nanoparticles can have appropriate sizes, an excellent ability to complex with nucleic acid drugs, high efficiency of drug encapsulation, stability in serum, and low toxicity, therefore realizing safe and highly efficient delivery and transfection of nucleic acid drugs. Moreover, the present invention also provides PEGylated lipids derived from and similar in structure to the cationic lipids of the present invention, which can further enhance the stability of lipid nanoparticles and the efficiency of drug delivery when used synergistically with the cationic lipids of the present invention.

### DETAILED DESCRIPTION OF THE INVENTION

Unless otherwise specified, all technical and scientific terms used herein have the same meaning as is commonly understood by one of skill in the art to which this invention belongs.

In the present invention, unless otherwise specified, any two objects are each independently "selected from" or "preferably selected from" their own groups. When multiple levels are included in a group of selections or preferred selections, those selected for any two objects can be of the same or different levels. For example, the statement "L_{A} and L_{B} are each independently selected from A, B, and C" includes but is not limited to a situation that both L_{A} and L_{B} are A, or that L_{A} is A while L_{B} is B₁ (a lower-level selection of B). For example, "L_{A} is preferably A (a level 1 preferred selection), more preferably selected from A₁~A₃ (level 2 preferred selections), and most preferably selected from A₁₁~A₁₃ (level 3 preferred selections); L_{B} is preferably B (a level 1 preferred selection), more preferably selected from B₁~B₃ (level 2 preferred selections), and most preferably selected from B₁₁~B₁₃ (level 3 preferred selections)" includes but is not limited to a situation that A is selected from A₁~A₃ (level 2 preferred selections) while B is selected from B₁₁~B₁₃ (level 3 preferred selections), or that A and B are both selected from their own level 3 preferred selections.

In the present invention, the phrases "each independently", "each independently selected from", and "preferably, each independently selected from" mean that different objects can be each independently, or each independently selected from, or preferably, each independently selected from the group consisting of selections or preferred selections, which can also be applied to the same object at different locations or occurrences. For example, the statement "a divalent group is selected from the group consisting of -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, -C(R_{c})=N-NR_{c}-, and -NR_{c}-N=C(R_{c})-, wherein R_{c} is, at each occurrence, independently H or a C₁₋₁₂ alkyl group" indicates that two R_{c} groups can be the same or different in -NR_{c}-N=C(R_{c})-(e.g., one is methyl while another is a hydrogen atom or ethyl group), and that any R_{c} in -NR_{c}C(=O)NR_{c}- can be the same as or different from the R_{c} in -NR_{c}C(=O)O-.

In the present invention, when at least two items are listed, the "combination" of said listed items is a combination consisting of two or more of the listed items. The number of any listed item in the combination is not limited, and it can be one or greater than one; when said number is greater than 1, said item can appear in the same or different forms. For example, the statement "L is a linking group, or selected from the group consisting of an alkylene group, a divalent hydrocarbon group containing heteroatoms, -O-, -S-, -S-S-, -C(=O)-, -C(=O)O-, -OC(=O)-, -OC(=O)O-, -NH-, and combinations thereof' indicates that L can be any of the listed, or a combination of any two or more listed items, wherein said combination can be -CH₂-O-CH₂- (i.e., a combination of -O- and alkylene as listed, wherein the alkylene appears twice as two methylene groups) or -CH₂-NH-CH₂CH₂- (i.e., a combination of -NH- and alkylene as listed, wherein the alkylene appears twice as 1 methylene and 1 ethylene). Particularly, a combination of a linking bond and any linking group is still the linking group itself. In the present invention, unless otherwise specified, a "linking group" has at least one atom.

In the present invention, unless otherwise specified, the terms "include", "contain", "cover" and similar expressions in the specification and claims shall be interpreted as "include(s) but is/are not limited to" or "include(s) without limitation" in an open and inclusive manner.

In the present invention, when the expression "includes but is not limited to" is used before a certain range, it means that the items within the range are selectable but the selections are not limited to the range. All items within said range are not necessarily suitable, especially those explicitly unqualified in the present invention, and the criterion is based on successful implementation of the present invention.

In the present invention, a numerical interval can be indicated by a dash (e.g., 1-6), a wavy line (e.g., 1-6), or "to" (e.g., 1 to 6). Unless otherwise specified, a numerical interval represents the group of all integers and non-integers between endpoints included. "The average number of EO units is selected from 22~100", for example, wherein said number is not limited to integers but also any non-integers within the numerical range. For example, "integer in the range of 1-3" represents a group consisting of 1, 2, and 3. For example, -(CH₂)₁₋₄- represents a group consisting of -CH₂-, -(CH₂)₂-, -(CH₂)₃-, and -(CH₂)₄-. For example, 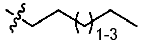 represents a group consisting of

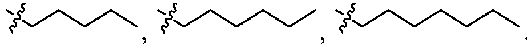

In the present invention, numerical intervals include but are not limited to those of integers, non-integers, percentages, and fractions, including endpoints unless otherwise specified.

In the present invention, with respect to the molecular weight of polymers, the terms "about" and "approximately" generally suggest a ±10% numerical range which in some cases can be increased, e.g., to ±15% but no greater than ±20%, based on the preset numerical value. For example, the deviation between 11 kDa and 10 kDa is 10%, and that between 12 kDa and 10 kDa is 20%; therefore, "approximately 10 kDa" includes but is not limited to 11 kDa and 12 kDa. For example, provided that the molecular weight of a PEG component is about 5 kDa, the corresponding molecular weight or number average molecular weight is allowed to be 5 kDa±10%, i.e., in the range of 4500-5500 Da.

In the present invention, with respect to percentages, the terms "about" and "approximately" generally suggest a±(0.5*0.1^{N})% numerical range when the preset numerical value (without "%") is accurate to the Nth decimal place. For example, approximately 1% means 1±(0.5*0.1⁰)%, i.e., the range of 0.5%-1.5%, approximately 2.2% means 2.2±(0.5*0.1¹)%, i.e., the range of 2.15%-2.25%, and approximately 2.33% means 2.2±(0.5*0.1²)%, i.e., the range of 2.335%-2.325%.

In the present invention, the degree of polymerization is represented by nᵢ, wherein i is an integer selected from 1, 2, 3, ...; different nᵢ in the same structure can interchangeably represent each other when having the same value. For example, when n₂≈n₃, n₂ can be represented by n₃ and n₃ can also be represented by n₂.

In the present invention, unless otherwise specified, a divalent linking group can be connected to another group using either one of its two connecting ends. For example, when an amide bond is used as a divalent linking group between GroupA and GroupB, both GroupA-C(=O)NH-GroupB and GroupA-NHC(=O)-GroupB are acceptable unless any particular connecting end is designated.

In the present invention, " 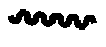 " is used to mark a linking bond in the structural representation of groups. For example, 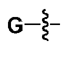 represents the group -G or G-, and 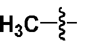 represents the group -CH₃ or CH₃-.

In the present invention, a linking bond or group extending out from a cyclic structure can be connected to any appropriate ring atom, when said linking bond points to the inside of the ring instead of marking any specific ring atom. Moreover, when said ring is part of a fused ring system, said linking bond can be connected to any ring atom of the fused ring system. For example, 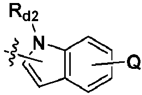 represents a group consisting of any corresponding structure with rational chemical connections, including but not limited to 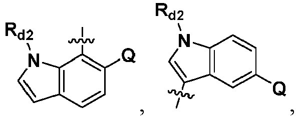 etc.

In the present invention, the number of carbon atoms of a group can be represented by a number or numerical interval as the subscript of "C", unless otherwise specified, excluding the contribution of carbon atoms of substituents. For example, C₁₋₁₂ means "having 1 to 12 carbon atoms"; and, a C_{1-1O} alkylene group represents any alkylene group having carbon atoms of the number in the range indicated by the subscript, i.e., any one selected from the group consisting of C₁, C₂, C₃, C₄, C₅, C₆, C₇, C₈, C₉, and C₁₀ alkylene groups, including but not limited to a linear C₁₋₁₀ alkylene group (e.g., -(CH₂)₆-) and a branched C₁₋₁₀ alkylene group (e.g., -(CH₂)₃-CH(CH₃)-(CH₂)₃-). As another example, "substituted C₁₋₁₂ alkyl group" is selected from the group consisting of C₁, C₂, C₃, C₄, C₅, C₆, C₇, C₈, C₉, C₁₀, C₁₁, and C₁₂ alkyl groups having at least one hydrogen atom substituted with substituent(s), without particular restriction on the number of carbon or hetero atoms in said substituent.

In the present invention, unless otherwise specified, a structure having isomers can be any form of the isomers. For example, when *cis-* and *trans-* isomers are present, the structure can be either a *cis-* or *trans-* isomer; when *E* and *Z* isomers are present, the structure can be either an (*E*)- or (*Z*)- isomer; and, when optical activity is involved, the structure can be either a levo- or dextro- isomer.

In the present invention, if inconsistence exists between a structure depicted and the name of said structure, then the depiction should have the higher priority.

In the present invention, the molecular weight of polyethylene glycol and its derivatives refers to the average molecular weight by default. Unless otherwise specified, "average molecular weight" generally refers to the number average molecular weight (Mₙ) which can be used to describe the molecular weight of either polydisperse blocks or substances, or monodisperse blocks or substances. The unit of measurement for molecular weight is dalton (Da), unless otherwise specified. The molecular weight of polyethylene glycol chain can also be measured in "degree of polymerization" which is, specifically, the number of repeat units (oxyethylene units, i.e., EO units). Accordingly, the average value and the number average value of the number of repeat units are preferably expressed by "average degree of polymerization" and "number average degree of polymerization", respectively.

In the present invention, the term "any appropriate" in "any appropriate linking group", "any appropriate reactive group", etc., indicates that the linking group, the reactive group, etc., can be any structure that follows the basic rules of chemical structures and can facilitate the implementation of preparation methods in the present invention. Chemical structures described in this manner can be regarded as having a clear and defined scope.

In the present invention, the terms "stable" and "degradable" used to describe groups are a pair of opposing concepts.

In the present invention, the term "degradable" means that a chemical bond in the present invention can be cleaved to obtain at least two independent residues. If a linking group with its structure altered after chemical changes still remains a complete linking group, then it is still within the scope of "stable". The conditions for degradation are not particularly limited, which can be physiological conditions in vivo or simulated physiological environments in vitro, etc., preferably physiological conditions in vivo or simulated physiological environments in vitro. Said physiological conditions are not particularly limited, including but not limited to physiological environments of serum, heart, liver, spleen, lung, kidney, bone, muscle, fat, brain, lymph node, small intestine, gonad, etc., which could be intracellular or in the extracellular matrix, in normal tissues or in pathologic tissues (e.g., tumor, inflamed tissue, etc.). Said simulated physiological environment in vitro is not particularly limited, including but not limited to physiological saline, buffer, culture medium, etc. The degradation is not particularly limited with respect to the rate, which can be, for example, rapid degradation via enzymolysis or slow hydrolysis under physiological conditions, etc. Said physiological conditions in vivo include physiological conditions during treatment such as ultraviolet radiation, thermal therapy, etc. The conditions for degradation include but are not limited to light, heat, low temperature, enzyme, oxidation-reduction, acidity, basicity, physiological condition, simulated physiological environment in vitro, etc., preferably light, heat, enzyme, oxidation-reduction, acidity, basicity, etc. The degradation can occur under stimulation from any of the above conditions. Said light includes but is not limited to visible light, ultraviolet light, infrared light, near-infrared light, mid-infrared light, etc. Said heat condition refers to a temperature higher than the normal physiological temperature which generally means 37°C, normally below 45°C, and preferably below 42°C. Said low temperature refers to a temperature below the physiological temperature of human body, preferably below 25°C, and more preferably ≤10°C, with specific examples such as refrigeration temperature, freezer temperature, temperature for liquid nitrogen treatment, 2~10°C, 4~8°C, 4°C, 0°C, -20±5°C, etc. Said enzyme is not particularly limited, and all enzymes that can be physiologically generated are included, e.g., peptidases, proteases, lyases, etc. Said oxidation-reduction is not particularly limited, e.g., oxidation-reduction transformation between a mercapto group and a disulfide bond, and hydrogenation-reduction transformation. Said acidic and basic conditions mainly refer to the pH conditions of internal body parts such as normal tissues, pathologic tissues, organs or tissues in treatment, etc. For example, the stomach has an acidic condition, and tumor sites usually have acidic conditions as well. The degradation described herein can be realized through metabolism in vivo (e.g., physiological effects, enzymatic reactions, oxidation-reduction reactions, etc.), microenvironment stimulations (e.g., acidic conditions and basic conditions) in specific in vivo sites, or clinical therapeutic stimulations (e.g., light, heat, low temperature), etc. What should be noted is that the bonds cleaved only under some conditions such as strong acidity, strong basicity, high temperature (e.g., above 100°C), and others which are common in organic chemistry but extreme for organisms are not considered to be degradable bonds in the present invention. For example, although an ether bond can be cleaved under strongly acidic conditions (e.g., hydrobromic acid), it is always classified as a stable linking group in the present invention.

In the present invention, the term "stable" indicates that a linking group can keep existing as complete (i.e., stably and covalently connected to adjacent groups) and therefore it is defined as "stable", allowing chemical changes without compromising the wholeness of the linking group. Said chemical changes are not particularly limited, including but not limited to isomerization, oxidation, reduction, ionization, protonation, deprotonation, substitution, etc. The conditions for stable existence are not particularly limited, including but not limited to light, heat, low temperature, enzyme, oxidation-reduction, neutrality, acidity, basicity, physiological condition, simulated physiological environment in vitro, etc., preferably light, heat, enzyme, oxidation-reduction, acidity, basicity, etc. The stable existence described herein indicates that, without particular stimulation (e.g., pH conditions in particular areas, and light, heat, low temperature in treatment, etc.), stable connections can be maintained in the metabolic cycle in vivo and the molecular weight is not reduced by chain cleavage as long as the structural integrity is maintained.

In the present invention, "stable" is not an absolute concept with respect to a specific linking group. For example, an amide bond is much more stable than an ester bond under acidic or basic conditions. Accordingly, "stable" linking groups in the present invention include amide bonds. However, the peptide bond, for example, is a special kind of amide bond formed via the dehydration condensation of two amino acids that respectively provide an α-carboxyl group and an α-amino group for the reaction, which can be cleaved under specific enzymatic conditions, and therefore it is also classified as a "degradable" linking group. Similarly, carbamate groups (urethane groups), thiocarbamate groups (thiourethane groups), and the like, can be either stable or degradable linking groups. More commonly, urethane groups, thiourethane groups, and the like, tend to degrade slowly, while non-peptide amide bonds can remain stable in the metabolic cycle in vivo. As another example, common ester bonds can degrade under acidic or basic condition, while the ester bonds contained in special structures can also degrade under UV exposure. As another example, even though some chemical bonds can degrade under specific enzymatic conditions, they can still be regarded as stable if their circulation in clinical use (e.g., site-directed administration) does not or basically not go through said enzymatic conditions.

In the present invention, all compounds of general formula should be regarded as including salts thereof. The "salt" is selected from acid addition salts formed by said compounds and inorganic and/or organic acids, base addition salts formed by said compounds and inorganic and/or organic bases, and combinations of any two or more thereof. When a compound of general formula contains both a basic moiety (such as, but not limited to, a pyridine or imidazole) and an acidic moiety (such as, but not limited to, a carboxylic acid), a zwitterion ("inner salt") can be formed and included in the term "salt". The "salt" can be pharmaceutically acceptable (i.e., non-toxic, physiologically acceptable) or otherwise. Salts of compounds of general formula can be formed by reacting said compounds with an amount (e.g., an equivalent) of acid or base in a medium such as one in which the salt precipitates or in an aqueous medium followed by lyophilization. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecyl sulfates, ethanesulfonates, fumarates, glucoheptanoates, glycerophosphates, hemi sulfates, heptanoates, hexanoates, hydrochlorides, hydrobromides, hydroiodides, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates, sulfonates, tartrates, thiocyanates, toluenesulfonates, undecanoates, and the like. Exemplary base addition salts include ammonium salts, alkali metal salts (e.g., sodium, lithium, and potassium salts), alkaline earth metal salts (e.g., calcium and magnesium salts), salts with organic bases (e.g., organic amines), and salts with amino acids (e.g., arginine, lysine, and the like). Basic nitrogen-containing groups may be quaternized with agents such as lower alkyl halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, tetradecyl, and stearyl chlorides, bromides, and iodides), aralkyl halides (e.g., benzyl and phenethyl bromides), etc. Said acid addition salts and base addition salts are both preferably pharmaceutically acceptable salts, and should be regarded as equivalent to the free forms of corresponding compounds of general formula for the purpose of this disclosure.

In the present invention, hetero atoms are not particularly limited, including but not limited to O, S, N, P, Si, F, Cl, Br, I, B, etc.

In the present invention, relative to a compound, a group formed after losing part of atoms or groups of said compound is also termed a residue.

In the present invention, groups of valence greater than or equal to 2 are collectively referred to as "linking groups". A linking group may have only one atom, such as an ether group (-O-) and a thioether group (-S-). Particularly, when "linking bond" is included in the definition of a group, it means that the group may contain no atoms but only act as a linker.

In the present invention, regarding the valence of a group, the term "multivalent" indicates that the valence is at least 3.

In the present invention, the term "group" for divalent groups can be replaced by the term "bond", without changing the meanings. For example, a divalent ether group can be termed an ether bond (-O-), a divalent ester group can be termed an ester bond (-OC(=O)- or -C(=O)O-), and a divalent carbamate group can be termed a carbamate bond (-OC(=O)NH- or -NHC(=O)O-).

In the present invention, when the number of atoms in a substituent is 1, the substituent can be termed a "substituent atom".

In the present invention, when a compound or group is "substituted", it means that said compound or group contains one or more substituents.

In the present invention, unless otherwise specified, the term "amine group" can be used interchangeably with "amino group", for monovalent, divalent, trivalent, and quadrivalent neutral or cationic groups, substituted or unsubstituted. For example, the -NH₂ in CH₃-NH₂ can be termed an "amino group", "amine group", or "primary amine group". As another example, the -NH- in CH₃-NH-CH₃ can be termed a "secondary amine group" or "secondary amino group", wherein the -NH-CH₃ can also be interpreted as a methyl-substituted amine group.

In the present invention, the term "amine group" includes but is not limited to primary amine groups, secondary amine groups, tertiary amine groups, and quaternary ammonium ions. Exemplary amine groups include -NRₜRₜ and -N⁺RₜRₜRₜ, wherein each Rₜ is independently a hydrogen atom or any hydrocarbon group.

In the present invention, when the valence is undefined, a "hydrocarbon group" could be monovalent, divalent, trivalent, ..., or n-valent, wherein n is the highest possible valence of said hydrocarbon group.

In the present invention, a "hydrocarbylene group" is a divalent hydrocarbon group.

In the present invention, the secondary amine bond (-NH-) and the hydrazine bond (-NH-NH-) are both capped by hydrocarbon groups at both ends, e.g., -CH₂-NH-CH₂- and -CH₂-NH-NH-CH₂-. As a counterexample, -C(=O)-NH- is termed an amide bond instead of one that contains a secondary amino bond.

In the present invention, a "group with functionality" is also termed a "functional group", which is preferably a reactive group, a protected reactive group, or a precursor of a reactive group, etc. The term "poly" indicates that the number of functional group is at least 3; for example, a polyol is a compound having at least 3 hydroxyl groups, a polythiol is a compound having at least 3 thiol groups, etc. What should be noted is that heterofunctional groups of other types are also allowed. For example, tris(hydroxymethyl)aminomethane is a triol containing also an amino group, and citric acid is a tricarboxylic acid containing also a hydroxyl group.

With respect to preparation methods in the present invention, unless otherwise specified, reactive groups also include protected forms thereof; said protected forms may be deprotected in any appropriate step in the actual preparation process to obtain the corresponding reactive forms.

In the present invention, ring atoms are the atoms constituting the ring skeleton.

In the present invention, "bio-related substance" includes but is not limited to the substances described, listed, or referred to in documents including CN104877127A, WO/2016/206540A, CN106967213A, CN108530637A, CN108530617A, and other cited references. In general, bio-related substances include but are not limited to drugs, proteins, polypeptides, oligopeptides, protein mimics, fragments and analogs, enzymes, antigens, antibodies and their fragments, receptors, small molecule drugs, nucleosides, nucleotides, oligonucleotides, antisense oligonucleotides, polynucleotides, nucleic acids, aptamers, polysaccharides, proteoglycans, glycoproteins, steroids, steroid compounds, lipids, hormones, vitamins, phospholipids, glycolipids, dyes, fluorescent substances, targeting factors, targeting molecules, cytokines, neurotransmitters, extracellular matrix substances, plant or animal extracts, viruses, vaccines, cells, vesicles, liposomes, micelles, etc. Said bio-related substances refer to not only themselves but also their precursors, activated states, derivatives, isomers, mutants, analogs, mimics, polymorphs, pharmaceutically acceptable salts, fusion proteins, chemically modified substances, genetically recombined substances, and the corresponding agonists, activating agents, activators, inhibitors, antagonists, modulators, receptors, ligands or ligand groups, antibodies and fragments thereof, acting enzymes (e.g., kinases, hydrolases, lyases, oxoreductases, isomerases, transferases, deaminases, deiminases, invertases, synthetases, etc.), enzyme substrates (e.g., coagulation cascade protease substrates, etc.), etc. Said derivatives include but are not limited to the derivatives of glycosides, nucleosides, amino acids, and polypeptides. Chemically modified products with newly formed reactive groups, i.e., modified products obtained through changing the types of reactive groups via modification or introducing structures such as functional groups, reactive groups, amino acids or derivatives thereof, polypeptides, etc., are all within the scope of chemically modified bio-related substances. Before or after bio-related substances combine with functionalized polyethylene glycols, they are also allowed to be combined with target molecules, appendages, or delivery vectors and form modified bio-related substances or complex bio-related substances. Wherein, said pharmaceutically acceptable salts can be inorganic salts such as hydrochlorides, sulfates, and phosphates, or organic salts such as oxalates, malates, citrates, etc. Wherein, "drugs" in the present invention include any agents, compounds, compositions, and mixtures with physiological or pharmacological effects in vivo or in vitro, and said effects are usually beneficial. Said drugs are not particularly limited with respect to the type, including but not limited to pharmaceuticals, vaccines, antibodies, vitamins, food, food additives, nutritional supplements, nutraceuticals, and other agents providing beneficial effects. Said "drugs" are not particularly limited with respect to the range of physiological or pharmacological effects in vivo, which can be effective for the whole body or only at local sites. Said "drugs" are not particularly limited with respect to the activity, mainly the active substances capable of interacting with other substances or the inert substances which are non-interactive but can be transformed into active forms by in vivo effects or certain stimulations. Wherein, "small molecule drugs" are bio-related substances of molecular weight not exceeding 1000 Da, or small molecule mimics or active fragments of any bio-related substance.

In the present invention, unless otherwise specified, the term "monosaccharide group" refers to the residue of monosaccharide or the skeleton of monosaccharide, including open-chain monosaccharide groups and cyclic monosaccharide groups (e.g., furanose ring and pyranose ring).

Monosaccharide groups in the present invention can be selected from but not limited to the residues of compounds of monosaccharides, sugar alcohols, deoxy sugars, amino sugars, amino sugar derivatives (e.g., amide derivatives), sugar acids, and glycosides, with open-chain or cyclic structures. Exemplary monosaccharide groups include the amino residue formed by removing an amino hydrogen atom of an amino sugar, the acyl group formed by removing a carboxylic hydroxyl group of a sugar acid, etc. Said monosaccharides include but are not limited to aldoses (polyhydroxy aldehydes) and ketoses (polyhydroxy ketones). Said monosaccharides also include alkyl ether derivatives; specifically, e.g., methyl ether derivatives; more specifically, e.g., quebrachitol. Monosaccharide groups in the present invention are not particularly limited with respect to the number of carbon atoms, including but not limited to tetroses, pentoses, hexoses, and heptoses, preferably pentoses and hexoses. Wherein, exemplary tetroses, pentoses, hexoses, heptoses, sugar alcohols, deoxy sugars, amino sugars, amide derivatives of amino sugars, sugar acids, glycosides, and the like include but are not limited to the structures disclosed in CN106967213A.

In the present invention, "micro-modification" refers to a chemical modification process that can be realized by simple chemical reactions. Said simple chemical reactions mainly include protection, deprotection, salt complexation, decomplexation, ionization, protonation, deprotonation, changing leaving groups, etc.

In the present invention, "micro-modified form" is according to "micro-modification", referring to a structural form that can be transformed into the target reactive group after simple chemical reactions such as protection, deprotection, salt complexation, decomplexation, ionization, protonation, deprotonation or changing leaving groups, etc. Said changing leaving groups, i.e., transformation of leaving groups, includes but is not limited to the transformation of an ester form to an acyl chloride form.

In some embodiments, the reaction process also involves "protection" and "deprotection" of relevant groups. To avoid the influence of a certain reactive group on said reaction, the protection of the reactive group is usually needed. In some embodiments, when 2 or more reactive groups are present, the target reactive group should be selectively used for the reaction, and therefore the other reactive groups should be protected. Protecting groups not only remain stable during the target reaction process, but can also be removed by conventional technical means in the art as needed.

In the present invention, the "protection" of a reactive group refers to a strategy for reversibly transforming the reactive group in need of protection to an inert group (i.e., non-reactive group) using particular reagents. Within a protected group, the moiety that distinguishes the protected form from the unprotected form is regarded as the "protecting group". For example, -OTBS is a protected form of hydroxyl group (-OH), wherein -TBS is the protecting group for hydroxyl group.

In the present invention, the terms "deprotection" and "removal of protection" have the same meaning, both referring to the process of transforming a protected group to its unprotected from.

In the present invention, "hydroxyl protecting group" includes all the hydroxyl protecting groups commonly used in the art. Hydroxyl protecting groups are preferably alkanoyl (e.g., acetyl, butyryl), aromatic alkanoyl (e.g., benzoyl), benzyl, triphenylmethyl, trimethylsilyl, t-butyldimethylsilyl, allyl, acetal, or ketal. The removal of acetyl groups is generally carried out under basic conditions, most commonly by the ammonolysis with NH₃/MeOH or by the methanolysis catalyzed by methanol anions. Benzyl groups can be easily removed via palladium-catalyzed hydrogenolysis in a neutral solution at room temperature, or via reduction reaction with metallic sodium in ethanol or liquid ammonia. Triphenylmethyl groups are generally removed via catalytic hydrogenolysis. Trimethylsilyl groups are generally removed using reagents containing fluoride ions (e.g., tetrabutylammonium fluoride/anhydrous THF, etc.). The *t*-butyldimethylsilyl ether is relatively stable, which can withstand ester hydrolysis conditions with alcoholic potassium hydroxide as well as mild reduction conditions (e.g., Zn/CH₃OH and the like), but can be removed by fluoride ions (e.g., Bu₄N⁺F⁻) in THF solution or by aqueous acetic acid at room temperature. The protection of diols preferably forms dioxolanes, dioxanes, cyclic carbonates, or cyclic borates.

In the present invention, "thiol protecting group" includes all the thiol protecting groups commonly used in the art. Similar to hydroxyl groups, thiol groups can be protected in the form of thioethers or thioesters. Thiol protecting groups are preferably tert-butyl, benzyl, substituted benzyl, benzhydryl, substituted benzhydryl, trityl, acetyl, benzoyl, tert-butoxycarbonyl, benzyloxycarbonyl, thioacetal, or thioketal. The deprotection of thioether can be realized by acid-catalyzed reduction using Na/NH₃, or by reactions with heavy metal ions (e.g., Ag⁺, Hg⁺) followed by treating with hydrogen sulfide. Groups such as S-diphenylmethyl, S-triphenylmethyl thioether, S-2-tetrahydropyranyl, and S-isobutoxymethyl groups in hemi-thioacetals can be oxidized to disulfides by (SCN)₂, iodine, or thionyl chloride, and further reduced to thiols. The formation of thioester and the corresponding deprotection methods are the same as those of carboxylates.

In the present invention, the term "carboxyl protecting group" refers to a protecting group that can be transformed into a carboxyl group via hydrolysis or via the deprotection of the carboxyl protecting group. Carboxyl protecting group is preferably alkyl (e.g., methyl, ethyl, and butyl) or aralkyl (e.g., benzyl), and more preferably butyl (tBu), methyl (Me), or ethyl (Et). In the present invention, the "protected carboxyl group" is a group protected by an appropriate carboxyl protecting group, preferably a methoxycarbonyl group, an ethoxycarbonyl group, a *t*-butyloxycarbonyl group, or a benzyloxycarbonyl group. Said carboxyl protecting groups can be removed through hydrolysis catalyzed by acids or alkalis, or through pyrolysis reactions occasionally. For example, *t*-butyl groups can be removed under mild acidic conditions, and benzyl groups can be removed by hydrogenolysis. The reagent used for the removal of a carboxyl protecting group is selected from the group consisting of TFA, H₂O, LiOH, NaOH, KOH, MeOH, EtOH, and combinations thereof, preferably the combination of TFA and H₂O, the combination of LiOH and MeOH, or the combination of LiOH and EtOH. The deprotection of a protected carboxyl group can produce the corresponding free acid, which could be carried out in the presence of an alkali that forms pharmaceutically acceptable salts with said free acid generated during said deprotection.

In the present invention, the term "amino protecting group" is equivalent to "amine protecting group", including all the groups commonly used as amino/amine protecting groups in the art, such as aryl C₁₋₆ alkyl groups, C₁₋₆ alkoxy C₁₋₆ alkyl groups, C₁₋₆ alkoxycarbonyl groups, aryloxycarbonyl groups, C₁₋₆ alkylsulfonyl groups, arylsulfonyl groups, methylsilyl groups, etc. An amino protecting group is preferably selected from the group consisting of a *t*-butoxycarbonyl (Boc) group, a *p*-methoxybenzyloxycarbonyl (Moz) group, and a 9-fluorenylmethoxycarbonyl (Fmoc) group. The reagent used for the removal of an amino protecting group is selected from the group consisting of TFA, H₂O, LiOH, MeOH, EtOH, and combinations thereof, preferably the combination of TFA and H₂O, the combination of LiOH and MeOH, or the combination of LiOH and EtOH. The reagent used for the removal of Boc can be TFA or HCl/EA, preferably TFA. The reagent used for the removal of Fmoc can be the solution of 20% piperidine in *N*,*N-*dimethylformamide (DMF).

In the present invention, "alkynyl protecting group" includes all the groups commonly used as alkynyl protecting groups in the art, preferably trimethylsilyl (TMS), triethylsilyl, tert-butyldimethylsilyl (TBS), or biphenyldimethylsilyl. TMS-protected alkynyl groups can be easily deprotected under basic conditions, for example, K₂CO₃/MeOH or KOH/MeOH. TBS-protected alkynyl groups can be deprotected in the solution of tetra-n-butylammonium fluoride in tetrahydrofuran (TBAF/THF).

In the present invention, hydroxyl groups that can be protected by hydroxyl protecting groups are not particularly limited, e.g., alcoholic hydroxyl groups and phenolic hydroxyl groups. Amino/amine groups that can be protected by amino protecting groups are not particularly limited, e.g., amino groups of primary amines, secondary amines, hydrazines, and amides. Amine groups in the present invention are not particularly limited, including but not limited to primary amine groups, secondary amine groups, tertiary amine groups, and quaternary ammonium ions.

In the present invention, the repeat unit of polyethylene glycols is the oxyethylene unit (i.e. -CH₂CH₂O- or -OCH₂CH₂-), also termed the "EO" unit. The number of repeat units is also termed the number of EO units. The average number of repeat units is also termed the average number of EO units, which is preferably the number-averaged mean value.

In the present invention, for polydisperse cases, the term "identical" or "same" or "equal" (including other forms of equivalent expressions) with respect to the molecular weight/degree of polymerization of a single compound molecule and the number average molecular weight/number average degree of polymerization of a compound component in macroscopic matter, unless otherwise specified, does not indicate a strict equality but a proximity or approximate equality of values, wherein said proximity or approximate equality preferably corresponds to a deviation within ±10% and the preset value is generally used as the base value.

In the present invention, for monodisperse cases, the same or equal numbers of oxyethylene units with respect to a single compound molecule or a general formula are strictly equal in value. For example, provided that the number of EO units of a certain PEG component is 11, the value 12 is not within the preset scope. However, in order to obtain a compound component having the preset number of EO units, particular preparation methods might be used, and therefore the macroscopic matter obtained could also contain components having other numbers of EO units besides the component having the target number of EO units due to the limitations of preparation or purification methods; in this case, if the deviation between the average number of EO units and the preset number of EO units is within ±5% (base value≥10) or within ±0.5 (base value<10), it is considered to have obtained a monodisperse macroscopic matter containing the target component. Moreover, if the content of components having the number or average number of EO units within the allowed deviation reaches specific percentages (preferably ≥90%, more preferably >95%, more preferably >96%, more preferably >98%, and more preferably 99-100%), it is also considered to have obtained the monodisperse macroscopic matter containing the target component; even if the aforementioned content in percentage is not met, the product as well as the component in the form of main product, co-product or by-product, which are insufficient in content, are all within the scope of the present invention as long as they are prepared using the preparation methods of the present invention or any similar methods adopting basically the same ideas, with or without isolation or purification.

In the present invention, when Da, kDa, the number of repeat units, or the number of EO units is used to describe the molecular weight of a compound of general formula for polydisperse components, the value of said molecular weight is allowed to be within a certain given range (with endpoints included, and preferably within ±10%) with respect to a single compound molecule. When the number of oxyethylene units is used to describe the preset molecular weight of a compound of general formula for monodisperse components, said molecular weight is not a variable value in a range but a discrete point while the average number of EO units could be variable in a certain range (within ±10% or ±1, preferably within ±5% or ±0.5) due to non-uniform molecular weights of components in the prepared product. For example, provided that the molecular weight of an mPEG (methoxy polyethylene glycol) is 5 kDa, the molecular weight of a single molecule of general formula is in the range of 4500-5500 Da and the average molecular weight of the corresponding components in the product is 5 kDa; i.e., the product with the average molecular weight in the range of 4500-5500 Da is regarded as the target product, and only the components with molecular weight in said range contribute to the content of the target component. As another example, provided that an mPEG is designed to have 22 oxyethylene units, all the compound molecules of general formula should have the number of EO units being strictly 22 while the prepared product could be a mixture of compounds having different numbers of EO units selected from 20, 21, 22, 23, and 24; meanwhile, if the average number of EO units is within the range of 22±2.2 (preferably 22±1.1), then the target component is considered obtained and all the components with molecular weight within said range can be regarded as the target component for calculation of purity.

In the present invention, unless otherwise specified, "mPEG" is a polyethylene glycol chain capped with a methoxy group, having the structure of 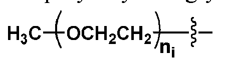 or 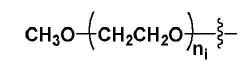 wherein nᵢ is the degree of polymerization of said polyethylene glycol chain.

In the present invention, a product with PDI<1.005 is regarded as monodisperse (PDI=1).

In the present invention, the number of repeat units in a "single-chain component" is at least 4.

In the present invention, the "lipid" includes but is not limited to the esters of fatty acids, and is generally characterized by poorer solubility in water and better solubility in many nonpolar organics. Although lipids usually have poorer solubility in water, lipids of particular types (e.g., lipids modified with polar groups, such as DMG-PEG2000) have limited water solubility, and can be dissolved in water under certain conditions. The known types of lipids include biomolecules such as fatty acids, waxes, sterols, fat-soluble vitamins (e.g., vitamins A, D, E, and K), monoglycerides, diglycerides, triglycerides, and phospholipids.

In the present invention, lipids include simple esters, compound esters, and derived lipids. Said simple esters are esters formed by fatty acids and alcohols, which can also be classified into three subcategories: fats, oils, and waxes. Said compound esters are "lipoid compounds" which are also termed "lipoids", including phospholipids, sphingolipids, glycolipids, steroids, sterols, and lipoproteins. Said derived lipids include derivatives of simple and compound lipids, having the general properties of lipids.

In the present invention, lipids can be synthetic or derived (separated or modified) from natural sources or compounds.

In the present invention, the term "lipid nanoparticle" or "LNP" refers to a nano-sized (e.g., 1 to 1000 nm) particle that contains one or more types of lipid molecules. The LNP provided herein can further contain at least one type of non-lipid payload molecule (e.g., one or more types of nucleic acid molecules). In some embodiments, the LNP contains a non-lipid payload molecule either partially or completely encapsulated inside a lipid shell. Particularly, in some embodiments, the payload is a negatively charged molecule (e.g., mRNA encoding a viral protein), and the lipid components of the LNP include at least one type of cationic lipid and at least one type of PEGylated lipid. It can be expected that the cationic lipids can interact with the negatively charged payload molecules and facilitate the incorporation and/or encapsulation of the payload into the LNP during LNP formation. As provided herein, other lipids that can be part of a LNP include but are not limited to neutral lipids and steroid lipids.

In the present invention, the term "cation" refers to a corresponding structure that is positively charged either permanently or non-permanently in response to certain conditions (e.g., pH). Therefore, cations include not only permanent cations but also those cationisable. Permanent cations refer to the corresponding compounds, groups, or atoms that are positively charged under conditions of any pH value or hydrogen ion activity of its environment. As a typical example, the presence of a quaternary nitrogen atom results in a positive charge. When a compound carries multiple positive charges, it can also be termed a permanent cation. A cationisable substance refers to a compound, group, or atom that is positively charged at a lower pH and uncharged at a higher pH of its environment. Moreover, in non-aqueous environments where the pH cannot be determined, a cationisable compound, group, or atom is positively charged at a high concentration of hydrogen ions and uncharged at a low concentration or activity of hydrogen ions. It depends on the individual properties of the cationisable or polycationisable compound, in particular the pKa of the respective cationisable group or atom, at which pH or hydrogen ion concentration said compound is charged or uncharged. In diluted aqueous environments, the fraction of cationisable compounds, groups, or atoms that are positively charged may be estimated using the so-called Henderson-Hasselbalch equation which is well-known to a person skilled in the art. In some embodiments, a cationisable compound or its moiety is positively charged at the physiological pH (e.g., about 7.0-7.4). In some preferred embodiments, a cationisable compound or its moiety is neutral at the physiological pH (e.g., about 7.0-7.4), but becomes positively charged at a lower pH (e.g., about 5.5-6.5). In some embodiments, the preferred range of pKa of the cationisable compound or its moiety is from about 5 to about 7.

In the present invention, the term "cationic lipid" refers to a lipid that is positively charged at any pH or hydrogen ion activity of its environment, or a cationisable lipid which can be positively charged in response to the pH or hydrogen ion activity of its environment (e.g., the expected environment for its use). Therefore, the term "cationic" includes the scope of both "permanently cationic" and "cationisable". In some embodiments, the positive charge of a cationic lipid is due to the existence of a quaternary nitrogen atom. In some embodiments, cationic lipids include zwitterionic lipids that can be positively charged in the expected environment for their use (e.g., at the endosomal pH). In some embodiments, if a liposome contains cationisable lipids, it is preferred that about 1% to 100% cationisable lipids are cationized at a pH of about 1 to 9, preferably 4 to 9, 5 to 8, or 6 to 8, and more preferably at the endosomal pH (e.g., about 5.5 to 6.5). Cationic lipids include but are not limited to *N,*N-dioleyl-*N*,*N*-dimethylammonium chloride (DODAC), *N*,*N*-distearyl-*N*,*N*-dimethylammonium bromide (DDAB), *N*-[1-(2,3-dioleoyloxy)propyl]-*N*,*N*,*N*-trimethylammonium chloride (DOTAP), *N*-[1-(2,3-dioleyloxy)propyl]-*N*,*N*,*N*-trimethylammonium chloride (DOTMA) *N*,*N*-dimethyl-2,3-dioleyloxy-1-(dimethylamino)propane (DODMA), 3-(didodecylamino)-*N1*,*N1*,4-tridodecyl-1-piperazineethanamine (KL10), *N1*-[2-(didodecylamino)ethyl]-*N1*,*N4*,*N4*-tridodecyl-1,4-piperazinediethanamine (KL22), 14,25-ditridecyl-15,18,21,24-tetraaza-octatriacontane (KL25), 1,2-dilinoleyloxy-*N*,*N*-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA), 2,2-dilinoleyl-4-(2-dimethylaminoethyl)-[1,3]-dioxolane (DLin-KC2-DMA), the cationic lipids disclosed in CN113402405A, and mixtures thereof.

In the present invention, the term "PEGylated lipid" refers to a molecule containing both lipid and polyethylene glycol moieties, which can be classified into linear PEGylated lipids and non-linear PEGylated lipids according to the structure. PEGylated lipids not only include those provided in the present invention, but also include but are not limited to polyethylene glycol-1,2-dimyristoyl-sn-glycerol (PEG-DMG), polyethylene glycol-1,2-distearoyl-sn-glycero-3-phosphoethanolamine (PEG-DSPE), PEG-cholesterol, polyethylene glycol-diacylglycamide (PEG-DAG), polyethylene glycol-dialkyloxypropyl (PEG-DAA), and specifically PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, PEG2000-2,3-distearoylglycerol (PEG-DMG), etc.

In the present invention, the term "neutral lipid" refers to any of the many types of lipid substances existing in the form of uncharged species or neutral zwitterionic species at a chosen pH, preferably phospholipid. Neutral lipids include but are not limited to 1,2-dilinoleoyl-sn-glycero-3-phosphocholine (DLPC), 1,2-dimyristoyl-sn-glycero-phosphocholine (MPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3 -phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3 -phosphoethanolamine, 1,2-dioleoyl-sn-glycero-3-phospho-rac-(1-glycerol) sodium salt (DOPG), dioleoylphosphatidylserine (DOPS), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE), and compositions thereof. A neutral lipid can be synthetic or naturally sourced.

In the present invention, a "steroid lipid" is selected from cholesterol, coprostanol, sitosterol, ergosterol, campesterol, stigmasterol, brassicasterol tomatidine, ursolic acid, α-tocopherol, and mixtures thereof.

In the present invention, a liposome can be termed a "PEGylated liposome" when containing PEGylated lipids, a "cationic liposome" when containing cationic lipids, and either a "PEGylated liposome" or a "cationic liposome" when containing both PEGylated lipids and cationic lipids.

In the present invention, a "cationic liposome" refers to a liposome containing cationic lipids and optionally other types of lipids (including but not limited to PEGylated lipids, neutral lipids, steroid lipids, etc.).

In the present invention, the term "N/P ratio" refers to the molar ratio of the nitrogen atoms in cationic lipids to the phosphate groups in nucleic acids.

In the present invention, the term "nucleic acid" refers to DNA, RNA, or a modified form thereof, including purine or pyrimidine bases in DNA (adenine "A", cytosine "C", guanine "G", thymine "T"), and purine or pyrimidine bases in RNA (adenine "A", cytosine "C", guanine "G", uracil "U").

In the present invention, the term "RNA" refers to a ribonucleic acid that may be naturally or non-naturally existing. For example, an RNA may include modified and/or non-naturally existing components such as one or more nucleobases, nucleosides, nucleotides, and linkers. An RNA may include a cap structure, a chain terminating nucleoside, a stem loop, a polyadenylation sequence and/or a polyadenylation signal. An RNA may have a nucleotide sequence encoding a polypeptide of interest. For example, an RNA may be a messenger RNA (mRNA). Translation of an mRNA encoding a particular polypeptide, for example, the in vivo translation of an mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA (messenger RNA), single guide RNA (sgRNA), cas9 mRNA, and mixtures thereof.

In the present invention, an antisense oligonucleotide or small interfering RNA (siRNA) can inhibit the expression of the target gene and the target protein in vitro or in vivo.

In the present invention, FLuc mRNA can express the luciferase protein which emits bioluminescence in the presence of a fluorescein substrate, and therefore FLuc is commonly used in the culture of mammalian cells to measure gene expression and cell activity.

In the present invention, the term "inhibiting the expression of a target gene" refers to the ability of nucleic acids to silence, reduce, or inhibit the expression of a target gene. To examine the extent of gene silencing, a test sample (e.g., a sample of cells in culture expressing the target gene) is contacted with nucleic acids that inhibit the expression of the target gene. The expression of the target gene in the test sample or test animal is compared to that in a control sample (e.g., a sample of cells in culture expressing the target gene) which is not contacted with or administered the nucleic acids. The expression of the target gene in the control sample may be assigned a value of 100%. In particular embodiments, inhibition of the expression of the target gene is achieved when the level of target gene expression in the test sample relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

In the present invention, assays for determining the level of target gene expression include but are not limited to dot blots, northern blots, in situ hybridization, ELISA, immunoprecipitation, enzyme function, and phenotypic assays.

In the present invention, the term "transfection" refers to the introduction of a species (e.g., RNA) into a cell. Transfection may occur, for example, in vitro, ex vivo, or in vivo.

In the present invention, the term "antigen" typically refers to a substance that can be recognized by the immune system, preferably recognized by the adaptive immune system, and can trigger an antigen-specific immune response, for example, forming antibodies and/or antigen-specific T cells as a part of the adaptive immune response. Typically, the antigen may be or may contain a peptide or a protein that can be presented to T cells by MHC. In the present invention, the antigen may be a translation product of the provided nucleic acid molecule (preferably mRNA as defined herein). In this context, fragments, variants, and derivatives of peptides and proteins containing at least one epitope are also defined as antigens.

In the present invention, the term "delivery" refers to providing an entity to the target, for example, delivering drugs and/or therapeutic agents and/or prophylactic agents to subjects, said subjects being tissues and/or cells of human and/or other animals.

In the present invention, the term "pharmaceutically acceptable carrier" refers to a diluent, adjuvant, excipient, or vehicle administered together with the therapeutic agent, which is, within the scope of sound medical judgement, suitable for contact with tissues of human and/or other animals without causing any excessive toxicity, irritation, allergic reaction, or other problems or complications corresponding to rational benefit/risk ratios. Pharmaceutically acceptable carriers that can be used in the pharmaceutical compositions in the present invention include but are not limited to sterile liquids, such as water and oil, including oils from petroleum, animals, vegetables, or synthetics, e.g., peanut oil, soybean oil, mineral oil, sesame oil, etc. When said pharmaceutical composition is administered intravenously, an exemplary carrier is water. Physiological saline, glucose, and aqueous glycerol solution can also be used as liquid carriers, especially for injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. Said compositions may also contain a small amount of humectant, emulsifier, or pH buffer as needed. Oral preparations may contain standard carriers, such as pharmaceutical grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and water for hydration. More specifically, excipients include but are not limited to butylated hydroxytoluene (BHT), calcium carbonate, calcium phosphate, calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, phenyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present invention, pharmaceutical compositions can act systematically or locally. For this purpose, they can be administered via appropriate routes such as injection (e.g., intravenous, intraarterial, subcutaneous, intraperitoneal, and intramuscular injections, including instillation) and transdermal delivery, or via oral, buccal, transnasal, transmucosal, or topical routes, or in the form of ophthalmic preparation, or by inhalation. Regarding these routes of administration, the pharmaceutical compositions of the present invention can be administered in suitable dosage forms. Said dosage forms include but are not limited to tablets, capsules, lozenges, hard sugar agents, powders, sprays, creams, ointments, suppositories, gels, pastes, lotions, ointments, aqueous suspensions, injectable solutions, elixirs, and syrups.

In the present invention, vaccines are preventive or therapeutic materials that provide at least one antigen or antigenic function. An antigen or antigenic function can stimulate the body's adaptive immune system to provide an adaptive immune response.

In the present invention, treatment refers to patient management and care in order to resist diseases, obstacles, or symptoms, which is intended to delay the development of diseases, obstacles, or symptoms, reduce or alleviate symptoms and complications, and/or cure or eliminate diseases, obstacles, or symptoms. The patients to be treated are preferably mammals, especially humans.

### 1. Cationic lipids

In one embodiment,
provided herein is a cationic lipid of the general formula (1): 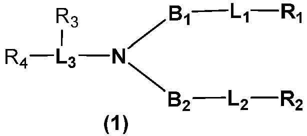
wherein, N is a nitrogen branching center;
L₁ and L₂ are each independently a linking bond or a divalent linking group;
L₃ is a trivalent linking group; L₃ and R₃ form a divalent linking group -L₃(R₃)- with R₃ as the side chain;
B₁ and B₂ are each independently a linking bond or a C₁₋₃₀ alkylene group;
R₁ and R₂ are each independently a C₂₋₃₀ aliphatic hydrocarbon group or a C₂₋₃₀ residue of aliphatic hydrocarbon derivative containing 1-2 O;
R₃ is selected from the group consisting of -OR_{d}, -NR_{d}R_{d}, -SR_{d}, -C(=O)R_{d}, -C(=O)OR_{d}, -OC(=O)R_{d}, -OC(=O)OR_{d}, and 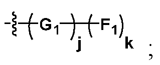 wherein, R_{d} is, at each occurrence, independently a C₁₋₁₂ alkyl group; G₁ is a branching group of valence k+1; j is 0 or 1; F₁ contains the functional group R₀₁; when j is 0, G₁ is absent; when j is 1, G₁ is connected to k F₁, and k F₁ are each independently of the same or different structures, wherein k is an integer in the range of 2-8;
R₄ is selected from the group consisting of a carbocyclyl group, a heterocyclyl group, and R'N(R')-R"-N(R')-; wherein, a heterocyclyl group is a cyclic group having 1, 2, or more heteroatoms in its ring atoms, said heteroatom being B, O, N, Si, P, or S; wherein, R' is, at each occurrence, independently H or a C₁₋₃ alkyl group; R" is a C₂₋₄ alkylene group;
said alkylene group, aliphatic hydrocarbon group, residue of aliphatic hydrocarbon derivative, carbocyclyl group, and heterocyclyl group are each independently substituted or unsubstituted;
or a salt, tautomer, stereoisomer, or solvate thereof.

### 1.1. Stable divalent linking groups and degradable divalent linking groups

In the present invention, unless otherwise specified, any divalent linking group itself or one consisting of said divalent linking group and adjacent heteroatom-containing groups thereof can be either a stable linking group (STAG) or a degradable linking group (DEGG).

### (1) Stable divalent linking groups (STAG)

The conditions for stable existence of STAG are not particularly limited, including but not limited to light, heat, low temperature, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The type of STAG is not particularly limited, including but not limited to the stable divalent linking group selected from the group consisting of alkylene group, divalent heteroalkyl group, carbon-carbon double bond, carbon-carbon triple bond, divalent dienyl group, divalent cycloalkyl group, divalent cycloalkenyl group, divalent cycloalkenyl hydrocarbon group, divalent cycloalkynyl hydrocarbon group, arylene group, aliphatic heterocyclic group, heterophenylene group, aromatic-fused heterocyclic group, hetero-fused heterocyclic group, substituted alkylene group, substituted heteroalkyl group, substituted heteroalkylene group, substituted double bond, substituted triple bond, substituted divalent dienyl group, substituted divalent cycloalkyl group, substituted divalent cycloalkenyl group, substituted divalent cycloalkenyl hydrocarbon group, substituted divalent cycloalkynyl hydrocarbon group, substituted arylene group, substituted aliphatic heterocyclic group, substituted phenylene-derived heterocyclic group, substituted aromatic-fused heterocyclic group, substituted hetero-fused heterocyclic group, ether bond, thioether bond, urea bond, thiourea bond, carbamate bond, thiocarbamate bond, -P(=O)-, divalent silyl group without active hydrogen atoms, boron-containing divalent linking group, secondary amino group, tertiary amino group, carbonyl group, thiocarbonyl group, amido group, thioamide group, sulphonamide group, enamine group, triazole, 4,5-dihydroisoxazole, skeleton of an amino acid or its derivative, and combinations of any two or more thereof.

Specifically, a STAG includes but is not limited to the structures described or listed in the references CN104530413A, CN104530415A, and CN104530417A. Taking CN104530417A as an example, the relevant paragraphs are [0627]~[0704]. The manner in which two or more stable divalent linking groups are combined into a STAG is not particularly limited, including but not limited to those disclosed in the paragraph [0704] of CN104530417A.

### (2) Degradable divalent linking groups (DEGG)

The conditions for degradation of DEGG are not particularly limited, including but not limited to light, heat, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The divalent linking group formed by the combination of any DEGG and any STAG is a degradable linking group. Degradable divalent linking groups containing aryl rings may also be formed by the combination of aryl rings and degradable divalent linking groups.

The type of DEGG is not particularly limited, including but not limited to the degradable divalent linking group selected from the group consisting of disulfide bond, vinylether bond, ester group, thioate group, thioester group, dithioester group, carbonate group, thiocarbonate group, dithiocarbonate group, trithiocarbonate group, carbamate group, thiocarbamate group, dithiocarbamate group, acetal group, cycloacetal group, thioacetal group, azaacetal group, azacycloacetal group, azathiaacetal group, dithioacetal group, hemiacetal group, thiohemiacetal group, azahemiacetal group, ketal group, thioketal group, azaketal group, azacycloketal group, azathiaketal group, imine bond, hydrazone bond, acylhydrazone bond, oxime bond, thiooxime ether bond, semicarbazone bond, thiosemicarbazone bond, hydrazino group, hydrazide group, thiocarbohydrazide group, azocarbohydrazide group, azothiocarbohydrazide group, hydrazino formate group, hydrazino thioformate group, carbohydrazide group, thiocarbohydrazide group, azo group, isourea group, isothiourea group, allophanate group, thioallophanate group, guanidino group, amidino group, aminoguanidino group, carbamimidamido group, imino acid group, thioimidate group, sulfonate group, sulfinate group, sulfonyl hydrazide group, sulfonylurea group, maleimide group, orthoester group, phosphate group, phosphirate group, phosphinate group, phosphonate group, phosphosilicate group, silicate group, amide group, thioamide group, sulfonamide bond, polyamide group, phosphamide group, phosphoramidite group, pyrophosphamide group, cyclophosphamide group, ifosfamide group, thiophosphamide group, aconityl group, peptide fragment, skeleton of a nucleotide or its derivative, skeleton of a deoxynucleotide or its derivative, and combinations of any two or more thereof.

Herein, said carbamate group, thiocarbamate group, amide group, phosphamide group, and the like, can be regarded as either a stable linking group or a degradable linking group, depending on the characteristics of the environment in which they are used.

Specifically, a DEGG includes but is not limited to the structures described or listed in the references CN104530413A, CN104530415A, and CN104530417A. Taking CN104530417A as an example, the relevant paragraphs are [0705]~[0725].

### 1.2. L₁, L₂

In one specific embodiment, L₁ and L₂ belong to any of the following cases:
Case (1): one of L₁ and L₂ is a linking bond, and the other is a divalent linking group;
Case (2): both L₁ and L₂ are linking bonds;
Case (3): both L₁ and L₂ are divalent linking groups, and L₁ and L₂ are of the same or different structures;

in any said case, the divalent linking group is selected from the group consisting of -O-, -S-, -S-S-, -OC(=O)-, -C(=O)O-, -OC(=O)O-, -C(=O)-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, -C(R_{c})=N-NR_{c}-, -NR_{c}-N=C(R_{c})-, and a combination of any two thereof and a C_{1-1O} alkylene group; wherein, R_{c} is, at each occurrence, independently H or methyl, preferably H;
preferably, L₁ and L₂ are each independently selected from the group consisting of -O-, -OC(=O)-, -C(=O)O-, -OC(=O)O-, -C(=O)-, -NHC(=O)-, -C(=O)NH-, -C(=O)O(CH₂)_{y}OC(=O)-, -OC(=O)(CH₂)_{y}OC(=O)-, -C(=O)O(CH₂)_{y}C(=O)O-, and -OC(=O)(CH₂)_{y}C(=O)O-, wherein y is an integer in the range of 2-8.

In one specific embodiment, L₁ and L₂ are each independently selected from the group consisting of -OC(=O)-, -C(=O)O-, and -OC(=O)O-.

In one specific embodiment, one of L₁ and L₂ is -C(=O)-, and the other is selected from the group consisting of -OC(=O)-, -C(=O)O-, -OC(=O)O-, and -O-.

In one specific embodiment, one of L₁ and L₂ is -OC(=O)O-, and the other is -OC(=O)- or -C(=O)O-.

In one specific embodiment, L₁ and L₂ are both -OC(=O)- or both -C(=O)O-.

In one specific embodiment, L₁ and L₂ are both -OC(=O)O-.

In one specific embodiment, L₁ and L₂ are both -O-.

In one specific embodiment, L₁ and L₂ are each independently selected from the group consisting of -NHC(=O)-, -C(=O)NH-, -OC(=O)-, and -C(=O)O-.

In one specific embodiment, L₁ and L₂ are both -C(=O)O(CH₂)_{y}OC(=O)-, both -OC(=O)(CH₂)_{y}OC(=O)-, both -C(=O)O(CH₂)_{y}C(=O)O-, or both -OC(=O)(CH₂)_{y}C(=O)O-, wherein y is an integer in the range of 2-8.

### 1.3. L₃, R₃

In one specific embodiment, the divalent linking group -L₃(R₃)- is selected from the group consisting of -L₄-, -Z-L₄-, -L₅-Z-L₄-, -L₄-Z-L₅-, -Z-L₄-Z-L₅-, -L₅-Z-L₄-Z-L₅-, -L₄-Z-L₅-Z-L₅-, -Z-L₅-Z-L₄-, and -L₅-Z-L₅-Z-L₄-, and its right end is connected to the nitrogen branching center; wherein, L₄ is -(CH₂)ₓ-CR₃H-(CH₂)ₓ-, L₅ is -(CH₂)ₓ-, and x is, at each occurrence, independently an integer in the range of 0-3; wherein, Z is, at each occurrence, independently selected from the group consisting of -C(=O)-, -OC(=O)-, -C(=O)O-, -OC(=O)O-, -O-, -S-, -S-S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; and wherein, R_{c} is, at each occurrence, independently H or methyl, preferably H;
-L₃(R₃)- is preferably -(CH₂)ₓ-CR₃H-(CH₂)ₓ-.

In one specific embodiment, the structure of the aforementioned -L₃(R₃)- is 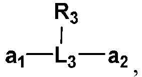 wherein a₁ is a linking bond connected to R₄, and a₂ is a linking bond connected to the nitrogen branching center of the general formula (1); said 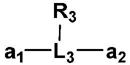 is preferably selected from the group consisting of the following structures:

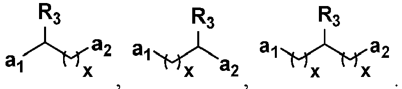

In one specific embodiment, the aforementioned 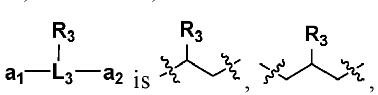 or

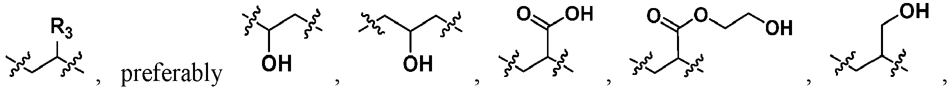

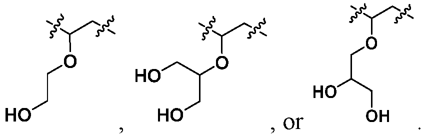

In one specific embodiment, the divalent linking group -L₃(R₃)- is a linear combination of 1 L₄, 0-4 Z, and 0-4 L₅, and the arrangement of L₄, Z, and L₅ is not particularly limited; wherein, L₄ is -(CH₂)ₓ-CR₃H-(CH₂)ₓ-, L₅ is -(CH₂)ₓ-, and x is, at each occurrence, independently an integer in the range of 0-3; wherein, Z is, at each occurrence, independently selected from the group consisting of -C(=O)-, -OC(=O)-, -C(=O)O-, -OC(=O)O-, -O-, -S-, -S-S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; and wherein, R_{c} is, at each occurrence, independently H or methyl. -L₃(R₃)- is preferably selected from the group consisting of the following structures:

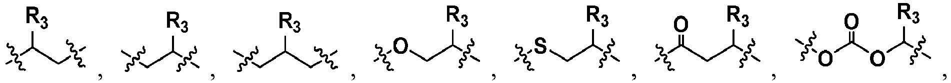

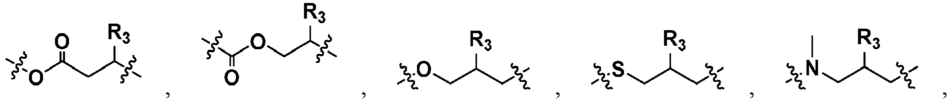

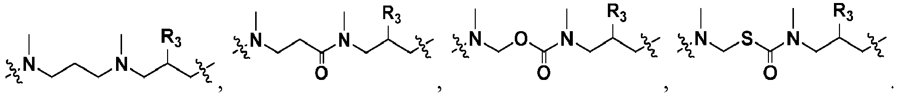

### 1.4. B₁, B₂

In one specific embodiment, B₁ and B₂ are each independently a linking bond or C₁₋₂₀ alkylene group, preferably belonging to any of the following cases:
Case (1): one of B₁ and B₂ is a linking bond, and the other is a C₁₋₂₀ alkylene group;
Case (2): both B₁ and B₂ are linking bonds;
Case (3): B₁ and B₂ are each independently a C₁₋₂₀ alkylene group; said C₁₋₂₀ alkylene group is substituted or unsubstituted, and is selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, an eicosylene group, a divalent linking group consisting of 1 -CR_{q}H- and 0-19 -CH₂-, and a divalent linking group consisting of 2 -CR_{q}H- and 0-18 -CH₂-; said R_{q} is, at each occurrence, independently selected from the group consisting of -OH, -(CH₂)_{tq}OH, -(CH₂)_{tq}CH₃, -(CH₂)_{tq}OCH₃, -SH, -(CH₂)_{tq}SH, -(CH₂)_{tq}SCH₃, -NH₂, -N((CH₂)_{tq}CH₃)₂, -(CH₂)_{tq}NH₂, and -(CH₂)_{tq}N(CH₃)₂, wherein tq is an integer in the range of 0-4; R_{q} is preferably -OH.

In one specific embodiment, the aforementioned B₁ and B₂ are each independently selected from the group consisting of a linking bond, an ethylene group, a propylene group, a butylene group, a pentylene group, a hexylene group, a heptylene group, an octylene group, a nonylene group, a decylene group, a -OH substituted butylene group, a -OH substituted pentylene group, a -OH substituted hexylene group, a -OH substituted heptylene group, and a -OH substituted octylene group;
it is preferred that both B₁ and B₂ are ethylene groups, both B₁ and B₂ are butylene groups, both B₁ and B₂ are hexylene groups, both B₁ and B₂ are heptylene groups, B₁ is a butylene group while B₂ is a heptylene group, B₁ is a pentylene group while B₂ is a hexylene group, B₁ is a pentylene group while B₂ is a heptylene group, B₁ is a linking bond while B₂ is a hexylene group, B₁ is a pentylene group while B₂ is a -OH substituted hexylene group, both B₁ and B₂ are -OH substituted hexylene groups, or B₁ is a hexylene group while B₂ is a -OH substituted hexylene group.

In one specific embodiment, B₁ and B₂ are each independently selected from the group consisting of a butylene group, a pentylene group, a hexylene group, and a heptylene group.

In one specific embodiment, one of B₁ and B₂ is a linking bond, and the other is selected from the group consisting of a pentylene group, a hexylene group, and a heptylene group.

In one specific embodiment, one of B₁ or B₂ is selected from the group consisting of a pentylene group, a hexylene group, and a heptylene group, and the other is

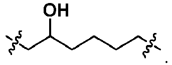

In one specific embodiment, B₁ and B₂ are both ethylene groups, both propylene groups, both butylene groups, both pentylene groups, both hexylene groups, both heptylene groups, or both

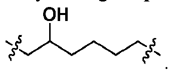

In one specific embodiment, B₁ is a pentylene group, and B₂ is a heptylene group.

### 1.5. R₁, R₂

In one specific embodiment, R₁ and R₂ are each independently R_{L}, R_{B}, or Rr; said R_{L}, R_{B}, and Rr each independently contains 0-4 Rₘ substituents; said Rₘ is, at each occurrence, independently a linear C₁₋₁₂ hydrocarbon group or branched C₁₋₃ alkyl group, preferably a methyl group;
said R_{L} is a linear C₂₋₃₀ aliphatic hydrocarbon group containing 0-4 carbon-carbon double bonds, preferably any of the following structures or any *cis*-/*trans*- isomer thereof 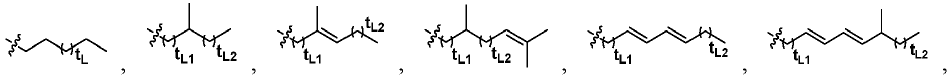 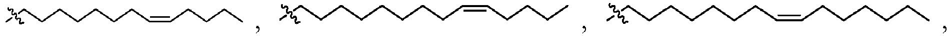 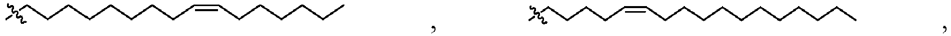 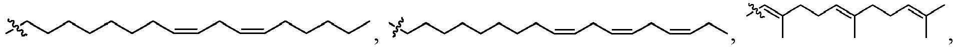 and 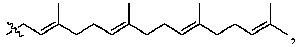 wherein t_{L} is an integer in the range of 0-20, and wherein t_{L1} and t_{L2} are each independently an integer in the range of 2-10;
said R_{B} has the structure of 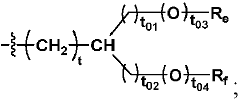 wherein, t is 0, 1, or 2, and t₀₁, t₀₂, t₀₃, and t₀₄ are each independently 0 or 1; Rₑ and R_{f} are each independently selected from the group consisting of C₁₋₁₂ alkyl, C₃₋₁₂ alkenyl, and C₃₋₁₂ alkynyl; R_{B} is preferably selected from the group consisting of the following structures: 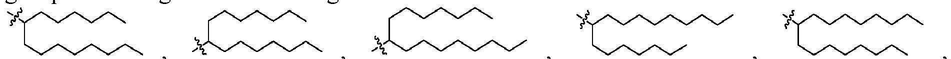 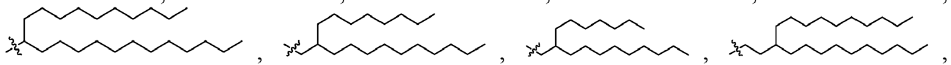 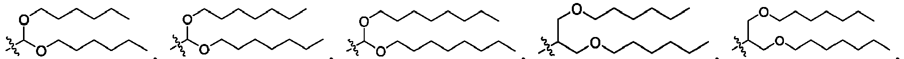 and 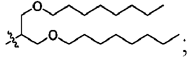
said Rr is a C₃₋₃₀ aliphatic hydrocarbon group containing cyclic structures; said cyclic structure is a C₃₋₂₀ carbocycle, preferably a benzene ring or a residue of C₃₋₂₀ cycloalkane; Rr is preferably selected from the group consisting of the following structures: 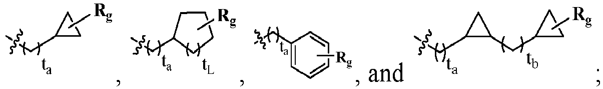 wherein, tₐ is, at each occurrence, independently an integer in the range of 0-12, t_{b} is an integer in the range of 1-12, t_{L} is an integer in the range of 0-20, and R_{g} is, at each occurrence, independently a substituent at any position on the ring, said substituent being selected from the group consisting of H, C₁₋₁₂ alkyl, C₂₋₁₂ alkenyl, and C₂₋₁₂ alkynyl;
Rr is more preferably selected from the group consisting of the following structures:

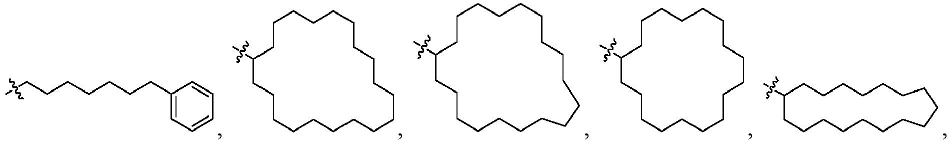

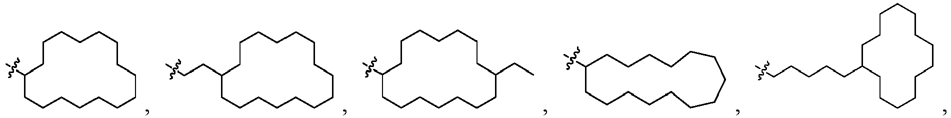

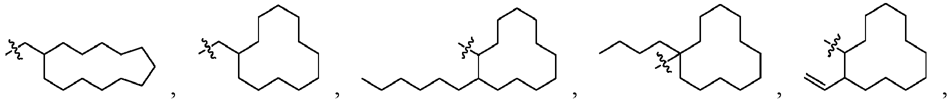

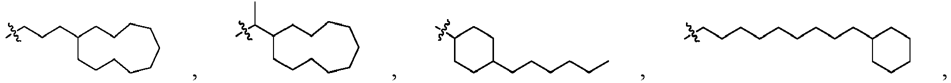

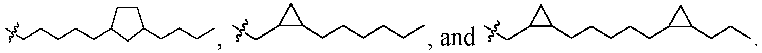

In one specific embodiment, R₁ and R₂ each independently contains 0-8 carbon-carbon double bonds and/or 0-8 carbon-carbon triple bonds; R₁ and R₂ are preferably each independently a linear alkyl group, a branched alkyl group, a linear alkenyl group, a branched alkenyl group, a linear alkynyl group, or a branched alkynyl group; said alkenyl group preferably contains 1-4 carbon-carbon double bonds, and said alkynyl group preferably contains 1-4 carbon-carbon triple bonds.

### 1.6. R₃, G₁, F₁, R₀₁

In one specific embodiment, R₃ is selected from the group consisting of -O(CH₂)ᵣCH₃, -S(CH₂)ᵣCH₃, -C(=O)(CH₂)ᵣCH₃, -C(=O)O(CH₂)ᵣCH₃, -OC(=O)(CH₂)ᵣCH₃, -OC(=O)O(CH₂)ᵣCH₃, and -(CH₂)ᵣN(CH₃)₂, wherein r is an integer in the range of 0-3 and preferably 0 or 1.

In one specific embodiment, R₃ is 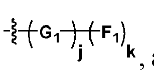 and F₁ has the structure of -(CH₂)ₕ-R₀₁ or -(CH₂)_{f}-Z₃-(CH₂)_{g}-R₀₁; wherein, h is an integer in the range of 0-6, f is 0, 1, or 2, and g is 2, 3, or 4; wherein, Z₃ is selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; and wherein, R_{c} is, at each occurrence, independently H or methyl, preferably H;

F₁ is preferably selected from the group consisting of -R₀₁, -CH₂-R₀₁, -(CH₂)₂-R₀₁, -O-(CH₂)₂-R₀₁, -C(=O)O-(CH₂)₂-R₀₁, -OC(=O)-(CH₂)₂-R₀₁, and -OC(=O)O-(CH₂)₂-R₀₁. In one specific embodiment, j is 1, R₃ is 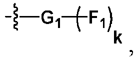 and G₁ has the structure of -(CH₂)_{f}-(Z₀)_{q}-(CH₂)_{f}-G₀₁; wherein, q is 0 or 1; wherein, f is, at each occurrence, independently 0, 1, or 2; wherein, G₀₁ is a trivalent or quadrivalent branching core selected from the group consisting of -OCH<, -N<, and 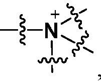 and its left end is connected to -(CH₂)_{f}-; wherein, Z₀ is Z₁, Z₂, or -(Z₂)_{q}-(CH₂)_{g}-(Z₁)_{q}-, wherein Z₁ and Z₂ are each independently selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is, at each occurrence, independently H or methyl, preferably H; and wherein, g is 2, 3, or 4;

G₁ is preferably -(CH₂)_{f}O(CH₂)_{f}CH<, more preferably -OCH₂CH<.

In one specific embodiment, R₀₁ is selected from the group consisting of an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a carbamate group, a succinimidyl group, a maleimide group, a protected maleimide group, a dimethylamino group, an alkenyl group, an alkenoate group, an azido group, an alkynyl group, a folate group, a rhodamine group, and a biotinyl group.

In one specific embodiment, R₀₁ is selected from the group consisting of functional groups, modified forms of functional groups, therapeutically targeting functional groups, and fluorescent functional groups; wherein, said modified forms are selected from the group consisting of the precursors of functional groups, the active forms to which functional groups are precursors, the active forms in which functional groups are substituted, and the inactive forms in which functional groups are protected; wherein, said precursors of functional groups refer to the structures which can be transformed into said functional groups through at least one process among oxidation, reduction, hydration, dehydration, electronic rearrangement, structural rearrangement, salt complexation and decomplexation, ionization, protonation, and deprotonation.

In one specific embodiment, the aforementioned R₀₁ is selected from the group consisting of the functional groups in the following classes A~I and modified forms thereof:
Class A: active ester group, analogous structure of active ester group; wherein, said active ester group is selected from the group consisting of a succinimidyl active ester group, a *p*-nitrophenyl active ester group, an *o*-nitrophenyl active ester group, a 1,3,5-trifluorophenyl active ester group, a 1,3,5-trichlorophenyl active ester group, a 1,3,5-tribromophenyl active ester group, a 1,3,5-triiodophenyl active ester group, a pentafluorophenyl active ester group, an imidazole active ester group, a benzotriazole active ester group, a thiazolidine-2-thione active ester group, a pyrrolidine-2-thione active ester group, a 2-mercaptobenzothiazole active ester group, and a 1-oxo-3-thioxoisoindoline active ester group; and wherein, said analogous structure of active ester group is an active carboxylate group or active acyl group;
Class B: carboxyl group, protected carboxyl group, sulfonic acid group, sulfonate group, sulfinic acid group, sulfinate group, sulfenic acid group, ester group, thioester group, dithioester group, carbonate group, thiocarbonate group, dithiocarbonate group, trithiocarbonate group, xanthate group, tetrathiodiester group, sulfone group, sulfoxide group, methacryloyl group, hydroxamic acid group, thiohydroxamic acid group, sulfonyl halide group, thiocarboxy group;
Class C: aldehyde group, hydrated aldehyde group, thioaldehyde group, acyl halide group, ketone group, hydrated ketone group, thione group, hydrated thione group, glyoxal group, acetal group, monothioacetal group, dithioacetal group, ketal group, monothioketal group, dithioketal group, hemiacetal group, thiohemiacetal group, hemiketal group, orthoacid group, protected orthoacid group, orthoester group, cyanate group, thiocyanate group, isocyanate group, isothiocyanate group, oxazoline group, isoxazoline group;
Class D: primary amino group, secondary amino group, protected amino group, hydroxylamine group, thiol group, disulfide group, halogen atom, haloacetamide group, ammonium salt, hydrazino group, tetramethylpiperidinyloxy group, dioxapiperidinyloxy group, O-carbonyl hydroxylamine group, amide group, imide group, hydrazide group, sulfonyl hydrazide group, hydrazone group, imine group, enamino group, alkynylamine group, carbamate group, thiocarbamate group, dithiocarbamate group;
Class E: urea group, thiourea group, guanidino group and its protonated form, amidine group and its protonated form, anhydride group, squaric acid group, squarate group, semi-squaric acid group, semi-squarate group, imidazole-1-carboxamide group, imidate group, nitrone group, aldoxime group, ketoxime group;
Class F: maleimide group, furan-protected maleimide group, acrylate group, *N*-acrylamide group, *N*-methacrylamide group, methacrylate group, maleamic acid group, 1,2,4-triazoline-3,5-dione group, linear azo compound group, cyclic azo compound group;
Class G: alkenyl group, alkenylhydrocarbon group, cycloalkenyl group, alkynyl group, alkynylhydrocarbon group, protected alkynyl group, cycloalkynl group, linear conjugated diene group, cyclic conjugated diene group, heteroatom-containing cyclic conjugated diene group, epoxy group, 1,2,4,5-tetrazine group, azido group, nitrile oxide group, cyano group, isocyano group, diazo group, diazonium ion , azo oxide group, nitrilimine group, aldimine *N*-oxide group, tetrazolyl group, 4-acetyl-2-methoxy-5-nitrophenoxy group and its diazotized form, imidazole group, indolyl group; wherein, the cycloalkenyl group is selected from the group consisting of a cyclooctenyl group, a norbornenyl group, a norbornadienyl group, an oxa norbornenyl group, and an oxa norbornadienyl group;
Class H: hydroxyl group, protected hydroxyl group, protected dihydroxyl group, siloxy group, trihydroxysilyl group, protected trihydroxysilyl group; wherein, the hydroxyl group is selected from the group consisting of an alcoholic hydroxyl group, a phenolic hydroxyl group, an enolic hydroxyl group, and a hemiacetal hydroxyl group;
Class I: monosaccharide group, selected from the group consisting of the residues of allose, altrose, arabinose, cladinose, erythrose, erythrulose, fructose, fucitol, fucosamine, fucose, fuculose, galactosamine, galactosaminitol, N acetyl-galactosamine, galactose, glucosamine, *N*-acetyl-glucosamine, glucosaminitol, glucose, glucose-6-phosphate, gulose glyceraldehyde, *L*-glycero-*D*-manno-heptose, glycerol, glyceraldehyde, dihydroxyacetone, gulose, idose, lyxose, mannosamine, mannose, mannose-6-phosphate, mannoheptulose, allulose, quinolose, quinosamine, rhamnitol, rhamnosamine, rhamnose, ribose, ribulose, deoxyribose, sedoheptulose, sorbose, tagatose, talose, tartaric acid, threose, xylose, xylulose, and functional derivatives thereof; said monosaccharide group is in *D*-configuration or *L*-configuration, cyclic or linear, substituted or unsubstituted;
wherein, said protected hydroxyl group is preferably selected from the group consisting of an ether group, a silicon ether group, an ester group, a carbonate group, and a sulfonate group; said protected amino group is preferably selected from the group consisting of a carbamate group, an amide group, an imide group, a *N*-alkylamine group, a *N*-arylamine group, an imine group, an enamine group, an imidazole group, a pyrrole group, and an indole group; said protected thiol group is preferably selected from the group consisting of a thioether group, a disulfide group, a silicon sulfide group, and a thioester group; said protected carboxyl group is preferably the carboxyl group protected by a group selected from the group consisting of a methyl group, an ethyl group, a tert-butyl group, and a benzyl group; said protected alkynyl group is preferably the alkynyl group protected by a silyl group; said protected dihydroxyl group preferably has the structure where the protecting group and two oxygen atoms form a five-membered or six-membered cyclic acetal structure; said dihydroxyl protecting group is preferably a methylene or substituted methylene group, and more preferably selected from the group consisting of methylene, 1-methylmethylene, 1,1-dimethylmethylene, 1,1-cyclopentylene, 1,1-cyclohexylene, 1-phenylmethylene, and 3,4-dimethylphenylmethylene groups.

In one specific embodiment, R₀₁ is selected from the group consisting of the functional groups of the following classes A~I and modified forms thereof:
Class A: 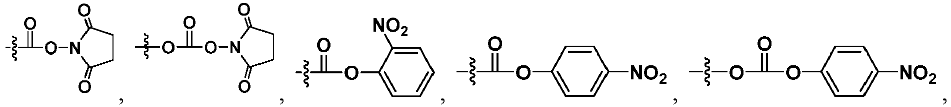 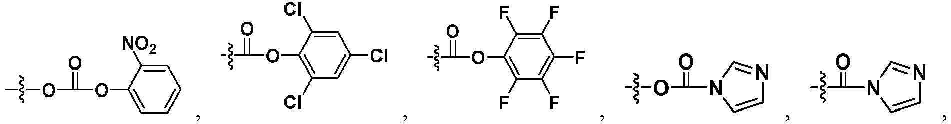 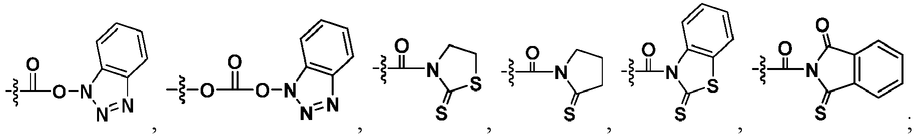
Class B: 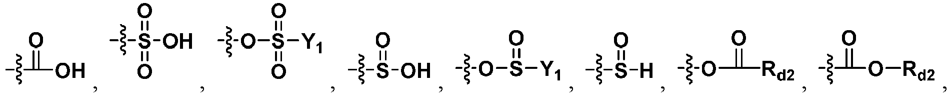 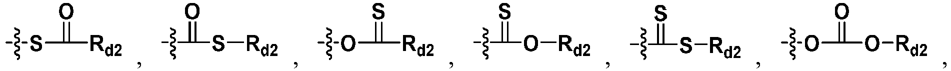 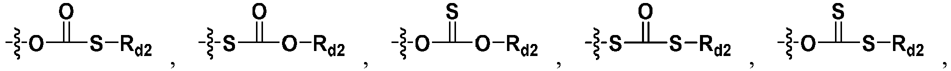 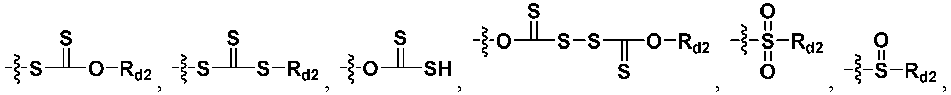 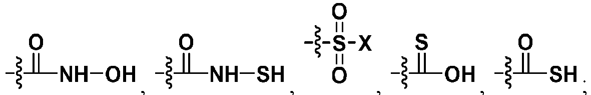
Class C: 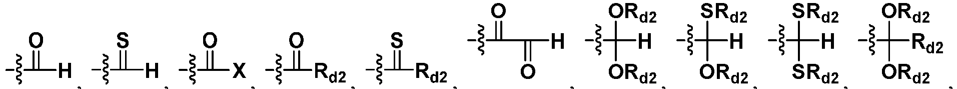 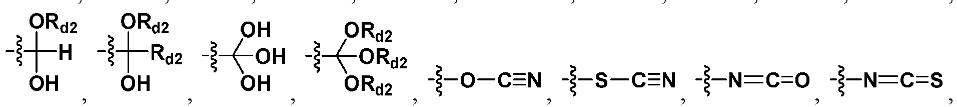 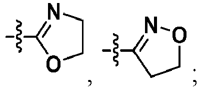
Class D: 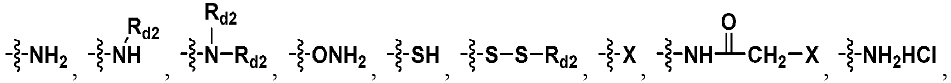 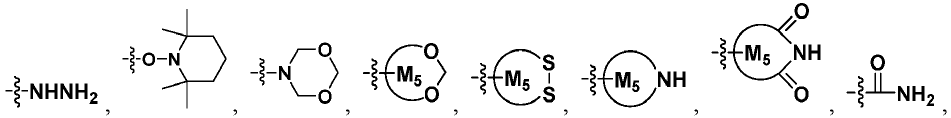 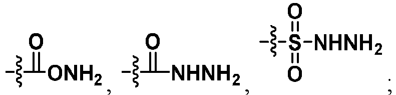
Class E: 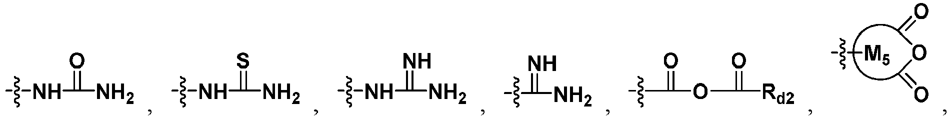 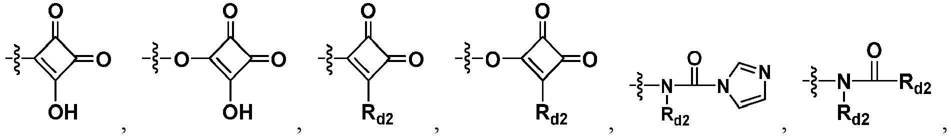 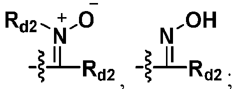
Class F: 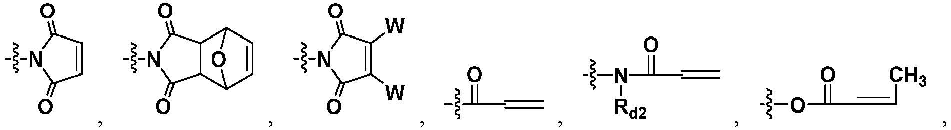 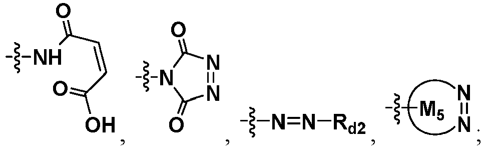
Class G: 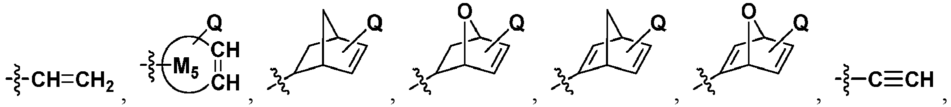 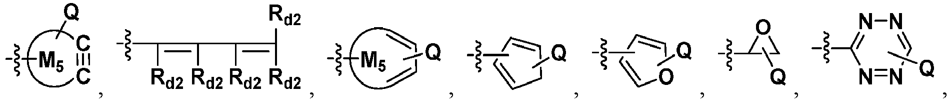 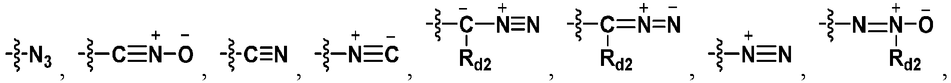 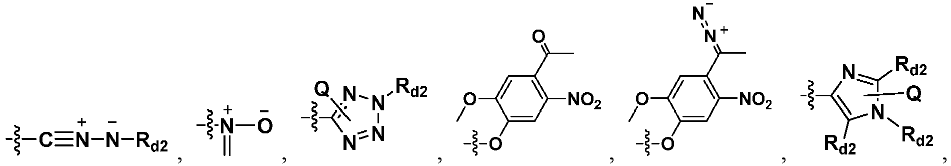 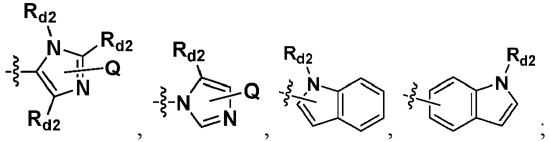
Class H: 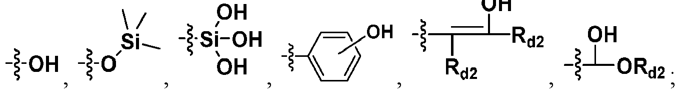
Class I:

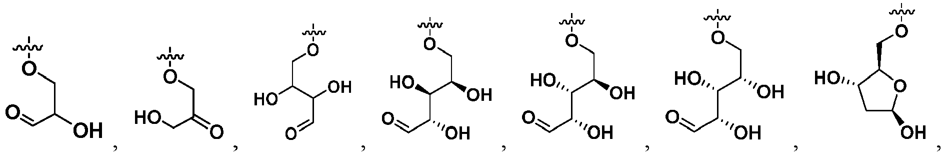

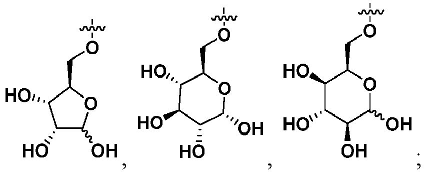

wherein, X is a halogen atom selected from the group consisting of a fluorine atom, a chlorine atom, a bromine atom, and an iodine atom;
wherein, Y₁ is selected from the group consisting of C₁₋₅ alkyl, vinyl, phenyl, benzyl, *p*-methylphenyl, 4-(trifluoromethoxy)phenyl, trifluoromethyl, and 2,2,2-trifluoroethyl groups;
wherein, R_{d2} is an organic group and is preferably, at each occurrence, independently selected from the group consisting of a C₁₋₅ alkyl group, a C₂₋₅ alkenyl group, a C₂₋₅ alkynyl group, and a phenyl group; said alkyl group, alkenyl group, alkynyl group and phenyl group are each independently substituted or unsubstituted;
wherein, W is a leaving group selected from the group consisting of -F, -Cl, -Br, -I, and -SPh;
wherein, M₅ is a ring atom selected from the group consisting of a carbon atom, a nitrogen atom, a phosphorus atom, and a silicon atom; the cyclic structure containing M₅ is a 3-30 membered ring, preferably a 3-20 membered ring, more preferably a 3-16 membered ring, and more preferably a 5-16 membered ring; said cyclic structure is preferably selected from the group consisting of cyclohexane, furanose ring, pyranose ring, benzene, tetrahydrofuran, pyrrolidine, thiazolidine, cyclohexene, tetrahydropyran, piperidine, 1,4-dioxane, pyridine, pyridazine, pyrimidine, pyrazine, 1,3,5-triazine, 1,4,7-triazacyclononane, cyclotripeptide, indene, indane, indole, isoindole, purine, naphthalene, dihydroanthracene, xanthene, thioxanthene, dihydrophenanthrene, 10,11 -dihydro-5H-dibenzo[a,d]cycloheptane, dibenzocycloheptene, 5-dibenzosuberenone, quinoline, isoquinoline, fluorene, carbazole, iminodibenzyl, acenaphthene, dibenzocyclooctyne, aza-dibenzocyclooctyne, substituted forms thereof, and heterosubstituted forms thereof;
wherein, 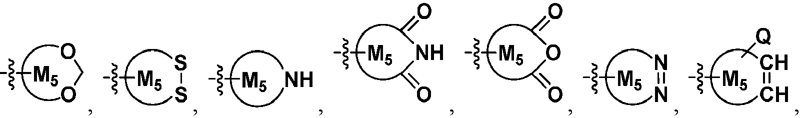 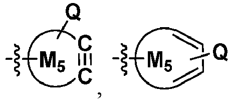 are cyclic structures of which the ring skeletons contain an acetal group, a disulfide bond, an amine group, an imide group, an anhydride group, an azo group, a carbon-carbon double bond, a carbon-carbon triple bond, and a conjugated diene, respectively; said cyclic structure is selected from the group consisting of a carbocycle, a heterocycle, a benzoheterocycle, a substituted carbocycle, a substituted heterocycle, and a substituted benzoheterocycle;
wherein, Q is an atom or substituent that promotes the inductive or conjugate effect of electrons of unsaturated bonds; when Q is connected to the ring, the number of Q may be one or greater; when said number is greater than one, the structures of Q may be the same or a combination of two or more different structures; when Q is a substituent, the structure of which is linear, branched with a pendant group, or ring-containing;
said modified form is selected from the group consisting of the precursors of functional groups, the active forms to which functional groups are precursors, the active forms in which functional groups are substituted, and the inactive forms in which functional groups are protected; wherein, said precursors of functional groups refer to the structures which can be transformed into said functional groups through at least one process among oxidation, reduction, hydration, dehydration, electronic rearrangement, structural rearrangement, salt complexation and decomplexation, ionization, protonation, and deprotonation.

In one specific embodiment, R₀₁ is selected from the group consisting of hydroxyl, carboxyl, aldehyde, amino, and thiol groups, and protected forms thereof; or, from the group consisting of a halogen atom, an ester group, a sulfonate group, and an active ester group; R₀₁ is preferably selected from the group consisting of -OH, -COOH, and -C(=O)OCH₃.

### 1.7. R₄

In one specific embodiment, R₄ is R'N(R')-R"-N(R')-, selected from the group consisting of 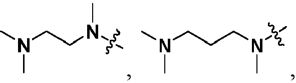 and 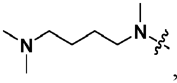 preferably

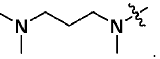

In one specific embodiment, R₄ is a carbocyclyl or heterocyclyl group containing 0-5 substituents; said substituents are each independently selected from the group consisting of -F, -Cl, -Br, -I, -R_{d}', -OR_{d}', -NRₐ'Rₐ', -SR_{d}', -C(=O)R_{d}', -C(=O)OR_{d}', -OC(=O)R_{d}', and -OC(=O)OR_{d}', wherein R_{d}' is a C₁₋₃ alkyl group;
R₄ is preferably selected from the group consisting of the following structures: 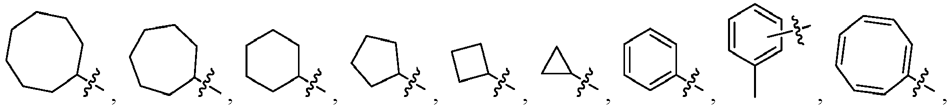 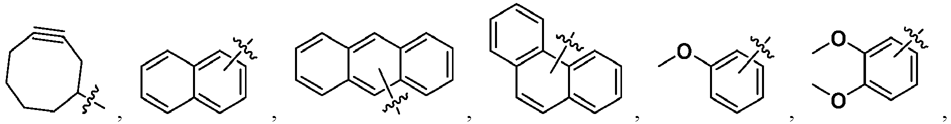 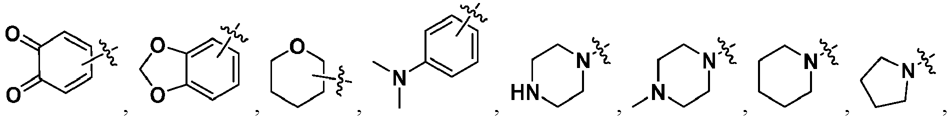 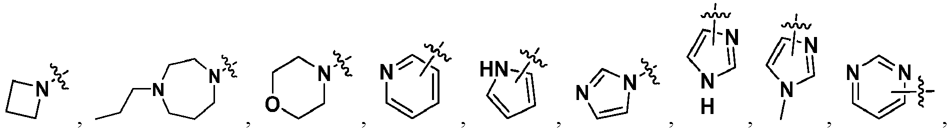 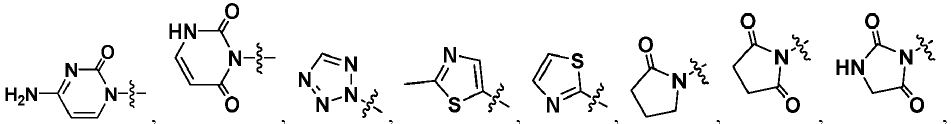 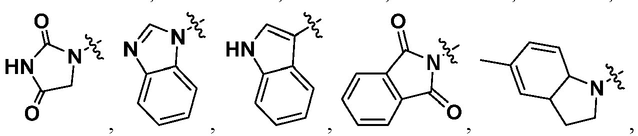
and more preferably selected from the group consisting of the following structures:

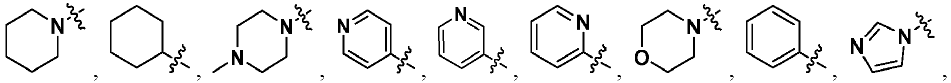

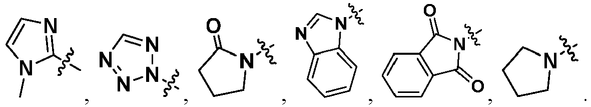

In one specific embodiment, R₄ is selected from the group consisting of phenyl, benzyl, naphthyl, phenanthrenyl, anthracenyl, biphenyl, oxyheterocycloalkyl, epoxyalkyl, furyl, dihydrofuryl, tetrahydrofuryl, pyranyl, dihydropyranyl ,tetrahydropyranyl, thiophenyl, dihydrothiophenyl, tetrahydrothiophenyl, thiopyranyl, dihydrothiopyranyl, tetrahydrothiopyranyl, aziridinyl, 2-azirinyl, azetidinyl, pyrrolidinyl, pyrrolidonyl, piperidinyl, piperazinyl, homopiperazinyl, morpholinyl, pyridyl, dihydropyridyl, tetrahydropyridyl, pyrrolyl, pyrrolinyl, hydantoinyl, imidazolyl, triazolyl, tetrazolyl, pyrimidinyl, dihydropyrimidinyl, tetrahydropyrimidinyl, purinyl, dihydropurinyl, tetrahydropurinyl, thiazolyl, lactam, succinimidyl, indolyl, and isoindolyl groups, etc., and benzo derivatives thereof; R₄ is unsubstituted or substituted by one or more Rₙ groups; when the number of Rₙ is greater than one, the structures of Rₙ are the same or a combination of two or more different structures; each Rₙ is independently selected from the group consisting of a C_{1-1O} alkyl group, a C₂₋₁₀ alkenyl group, a C₂₋₁₀ alkynyl group, a phenyl group, =O, an acetal group, -NH₂, -ORⱼ, -NRⱼRⱼ, -SRⱼ, -C(=O)Rⱼ, -C(=O)ORⱼ, -OC(=O)Rⱼ, -OC(=O)ORⱼ, a piperidinyl group, a piperazinyl group, and a morpholinyl group, wherein Rⱼ is a C₁₋₁₀ alkyl group and said alkyl, alkenyl and alkynyl groups are linear or ring-containing; preferably, each Rₙ is independently C₃₋₈ cycloalkyl, C₃₋₈ cycloalkenyl, or cyclooctynyl.

### 1.8. Examples of specific structures

In one specific embodiment, the structure of cationic lipid is selected from the group consisting of the following structures:

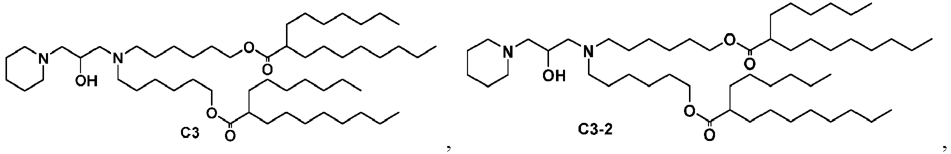

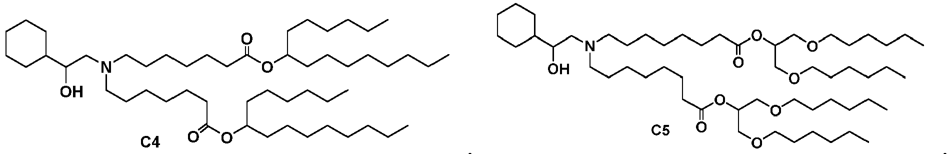

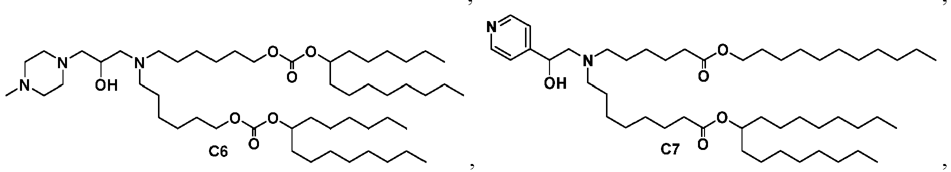

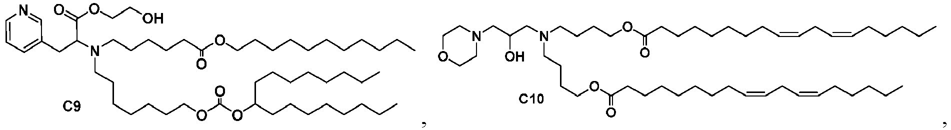

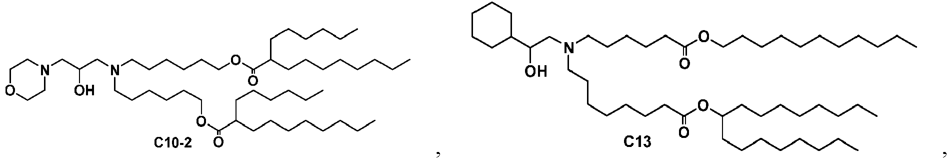

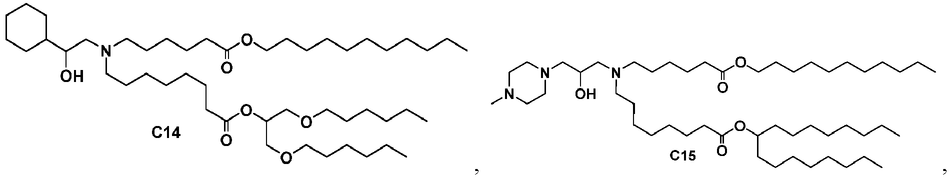

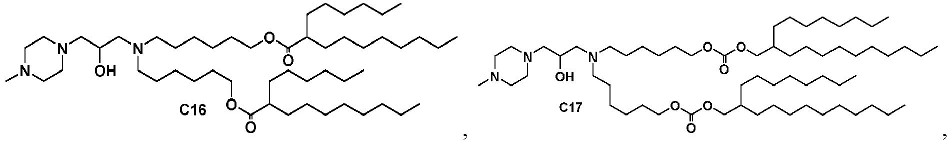

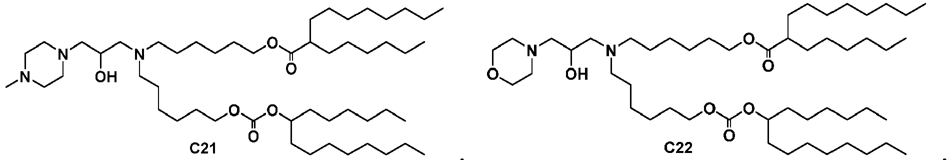

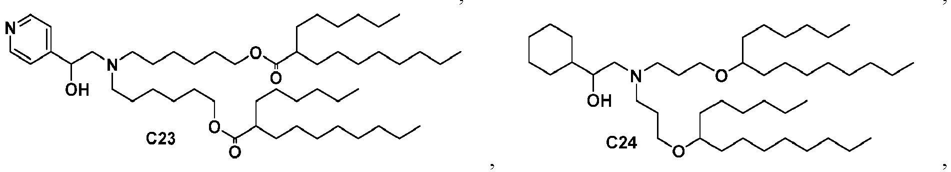

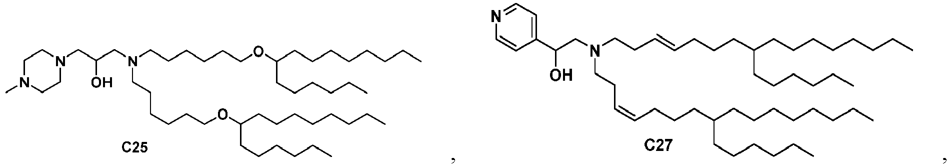

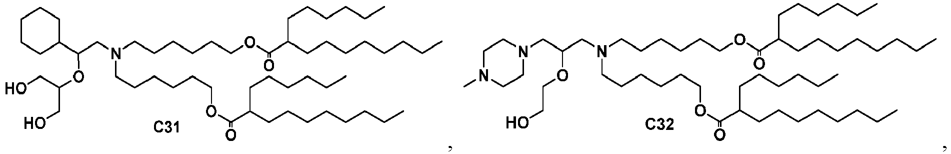

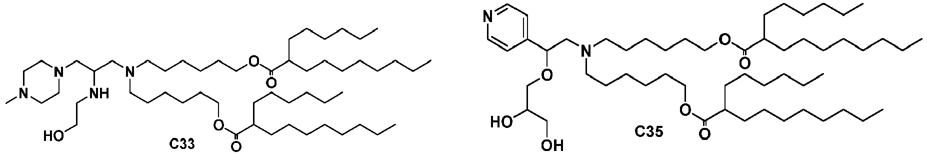

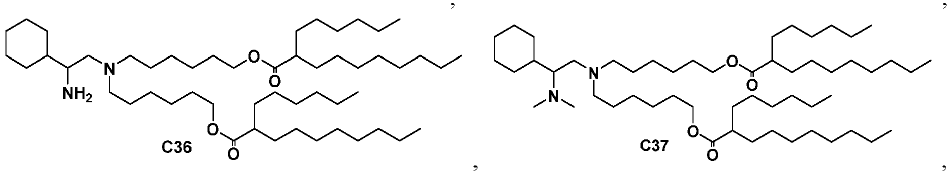

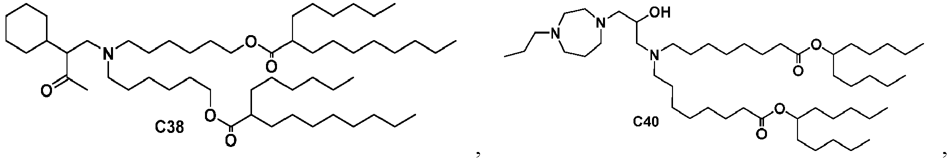

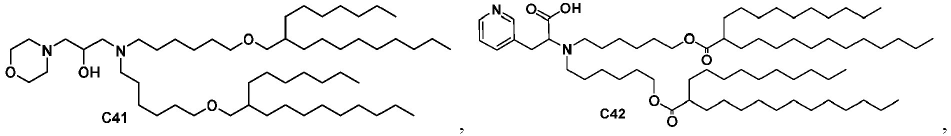

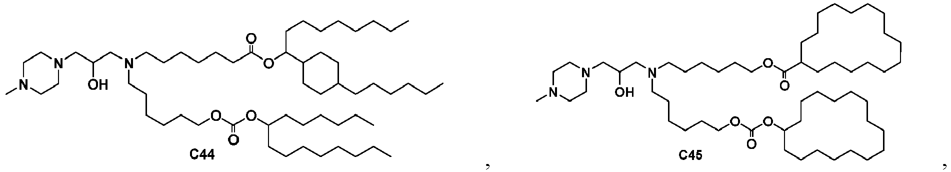

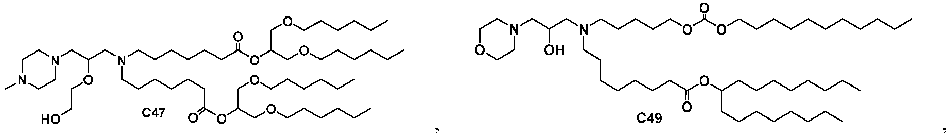

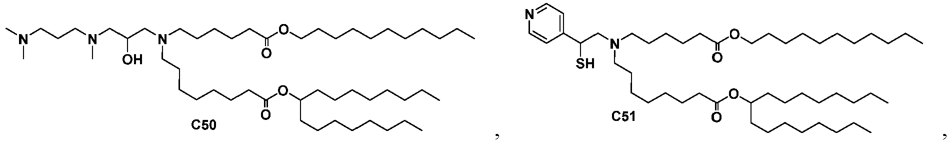

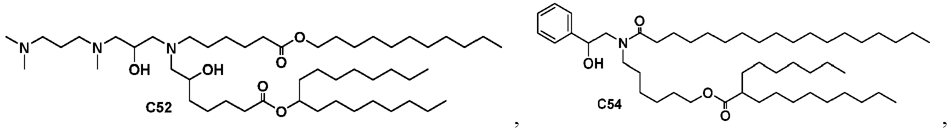

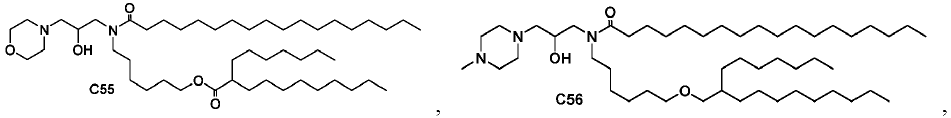

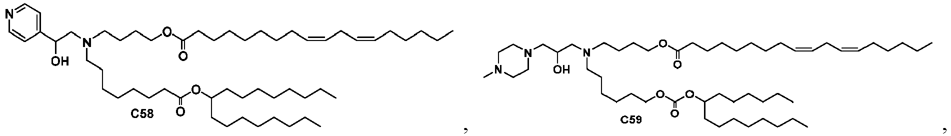

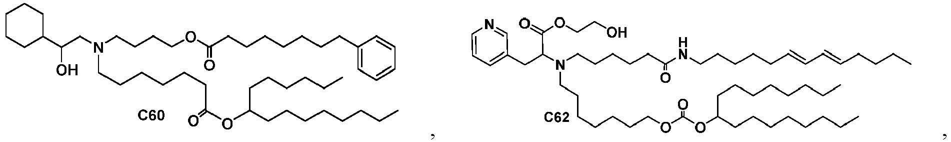

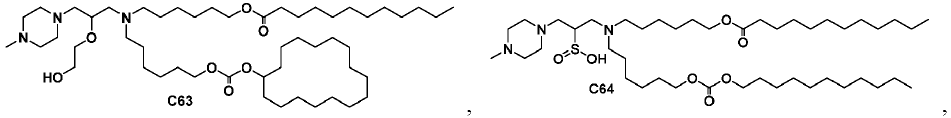

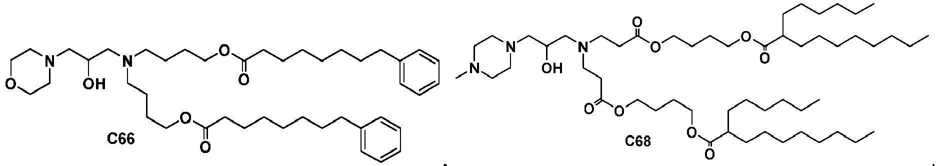

### 2. PEGylated lipids

In one embodiment,
provided herein is a PEGylated lipid derived from the aforementioned cationic lipid, having the structure of the general formula (2): 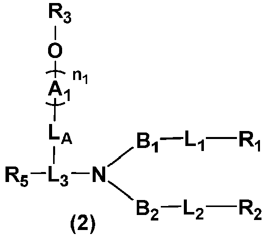
wherein, N is the nitrogen branching center;
L₁ and L₂ are each independently a linking bond or a divalent linking group;
L₃ is a trivalent linking group; L₃ and -L_{A}(A₁)ₙₗOR₃ form a divalent linking group -L₃[L_{A}(A₁)ₙ₁OR₃]- with -L_{A}(A₁)ₙ₁OR₃ as the side chain;
B₁ and B₂ are each independently a linking bond or a C₁₋₃₀ alkylene group;
R₁ and R₂ are each independently a C₂₋₃₀ aliphatic hydrocarbon group or a C₂₋₃₀ residue of aliphatic hydrocarbon derivative containing 1-2 O;
R₃ is selected from the group consisting of H, -R_{d}, -(CH₂)ₕNR_{d}R_{d}, -(CH₂)ₕSR_{d}, -C(=O)R_{d}, -C(=O)OR_{d}, and 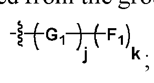 wherein, R_{d} is, at each occurrence, independently a C₁₋₁₂ alkyl group; h is an integer in the range of 0-6; G₁ is a branching group of valence k+1; j is 0 or 1; F₁ contains the functional group R₀₁; when j is 0, G₁ is absent; when j is 1, G₁ is connected to k F₁, and k F₁ are each independently of the same or different structures, wherein k is an integer from 2 to 8;
R₅ is selected from the group consisting of a C₃₋₁₄ alkyl group, a carbocyclyl group, a heterocyclyl group, and R'N(R')-R"-N(R')-; wherein, a heterocyclyl group is a cyclic group having 1, 2, or more heteroatoms in its ring atoms, said heteroatom being B, O, N, Si, P, or S; wherein, R' is, at each occurrence, independently H or a C₁₋₃ alkyl group; R" is a C₂₋₄ alkylene group;
L_{A} is -(CH₂)ₕ- or -(CH₂)_{f}-Z₃-(CH₂)_{g}-; wherein, h is an integer in the range of 0-6, f is 0, 1, or 2, and g is 2, 3, or 4; wherein, Z is selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is, at each occurrence, independently H or methyl, preferably H;
A₁ is -OCH₂CH₂-, and is connected to L_{A} by its left end;
n₁ is the number of repetitions of A₁, and is an integer in the range of 20-250;
said alkyl group, alkylene group, aliphatic hydrocarbon group, residue of aliphatic hydrocarbon derivative, carbocyclyl group, and heterocyclyl group are each independently substituted or unsubstituted.

In one specific embodiment, the structure of any aforementioned PEGylated lipid is selected from the group consisting of the following structures:

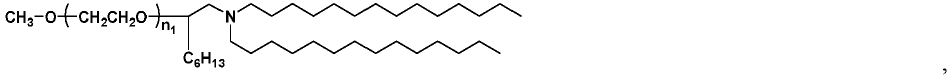

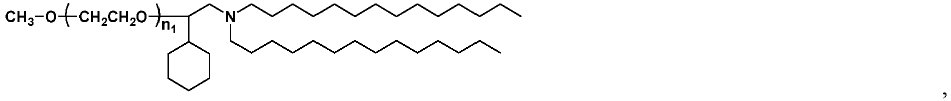

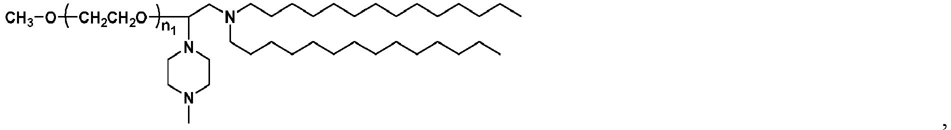

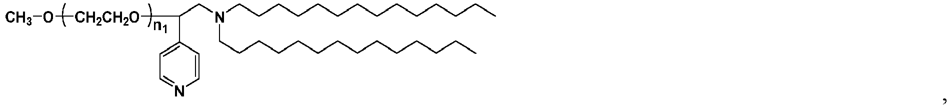

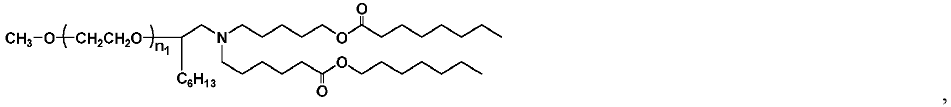

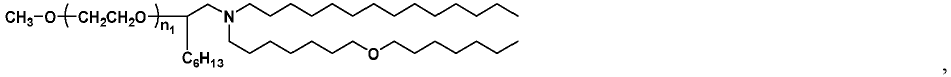

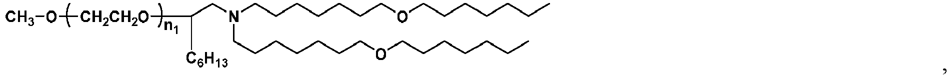

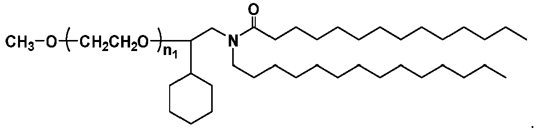

### 3. Methods of lipid preparation

In the present invention, the starting materials used for the preparation of lipids can be purchased or obtained by synthetic means.

In the present invention, two identical or different reactive groups may form a divalent linking group via reaction. The reaction condition is relevant to the type of the divalent linking group formed, which may include the prior art. For example, amino groups can react with active esters, active formates, sulfonate esters, aldehydes, α,β-unsaturated bonds, carboxyl groups, epoxides, isocyanates, and isothiocyanates, respectively, to obtain divalent linking groups such as amide groups, carbamate groups, amino groups, imine groups (which can be further reduced to secondary amine groups), amino groups, amide groups, alkanolamine groups, ureido bonds, thioureido bonds, etc.; thiol groups can react with active esters, active formates, sulfonate groups, thiol groups, maleimide groups, aldehyde groups, α,β-unsaturated bonds, carboxyl groups, iodoacetamide groups, and anhydride groups, respectively, to obtain divalent linking groups such as thioester groups, thiocarbonate groups, thioether groups, disulfide groups, thioether groups, thiohemiacetal groups, thioether groups, thioester groups, thioether groups, imide groups, etc.; unsaturated bonds can react with thiol groups to obtain thioether groups; carboxyl groups or acyl halides can respectively react with thiol groups and amino groups to obtain groups such as thioester groups, amide groups, etc.; hydroxyl groups can react with carboxyl groups, isocyanates, epoxides, and chlorocarbonyloxy groups to obtain divalent linking groups such as ester groups, carbamate groups, ether bonds, carbonate groups, etc.; carbonyl groups or aldehyde groups can react with amino groups, hydrazines, and hydrazides to obtain divalent linking groups such as imine bonds, hydrazone bonds, acylhydrazone bonds, etc.; reactive groups such as azido groups, alkynyl groups, alkenyl groups, thiol groups, azido groups, diene groups, maleimide groups, 1,2,4-triazoline-3,5-dione groups, dithioester groups, hydroxylamine groups, hydrazide groups, acrylate groups, allyloxy groups, isocyanate groups, tetrazole groups, and the like, can undergo click reactions to form various divalent linking groups including but not limited to triazole groups, isoxazole groups, thioether bonds, etc.

In the present invention, the type of coupling reaction is not particularly limited, as long as two identical or different reactive groups can form a covalent linking group through the reaction; single-step or multi-step coupling reactions can be included in the preparation process, while every step of said coupling reactions are preferably each independently selected from the group consisting of alkylation reaction, condensation reaction, amidation reaction, esterification reaction, thioesterification reaction, ring-opening reaction, ring-closing condensation reaction, addition reaction, cycloaddition reaction, addition reaction to α,β-unsaturated bonds, addition reaction of alkynes, Schiff base reaction-reduction reaction, click reaction, azide-alkyne addition reaction, 1,3-dipolar cycloaddition reaction, Diels-Alder addition reaction, thiol-yne reaction, thiol-ene reaction, thiol-vinyl reaction, and condensation reaction. The reaction conditions for said coupling reactions are relevant to the type of the covalent linking groups formed in the reactions, which may include the prior art. The valence state of a covalent linking group obtained from said coupling reactions could be divalent or trivalent, and preferably divalent. Groups obtained from said coupling reactions could be stable groups or degradable groups.

In the present invention, PEGylated lipids are obtained via preparation processes that contain coupling reactions and/or polymerization reactions. Said polymerization reaction includes at least the following two steps: deprotonation of small molecule initiator, and polymerization of ethylene oxide; said small molecule initiator can be a readily available starting materials, or an intermediate in the preparation process. When coupling and polymerization reactions are both present in a preparation process, the order in which said coupling and polymerization reactions take place is not particularly limited.

In the present invention, with respect to the method for preparing monodisperse PEGylated lipids, any monodisperse starting material containing polyethylene glycol components can be replaced by a polydisperse starting material containing the same components, therefore obtaining the corresponding polydisperse product; similarly, with respect to the method for preparing polydisperse PEGylated lipids, any polydisperse starting material containing polyethylene glycol components can be replaced by a monodisperse starting material containing the same components, therefore obtaining the corresponding monodisperse product.

In the present invention, when changing only the number average degree of polymerization and/or the PDI of a polyethylene glycol or its derivative as starting material, without changing structural formula thereof, the reactions can be successfully implemented without any obvious variations on the reaction conditions, said variations not including the change in the amount of solvent used.

The intermediates and end-products prepared in the present invention can be purified by a purification means including but not limited to extraction, recrystallization, adsorption treatment, precipitation, reverse precipitation, membrane dialysis, supercritical extraction, etc. The characterization of the structure and molecular weight of the end-products can use methods including but not limited to NMR, electrophoresis, UV-visible spectrophotometer, FTIR, AFM, GPC, HPLC, MALDI-TOF, circular dichroism spectroscopy, etc.

In the present invention, stepwise/multi-step reactions can be completed in multiple or a single practical operation by a person skilled in the art.

### 3.1. Preparation of cationic lipids

In the present invention, any aforementioned cationic lipid can be prepared using the following general reaction schemes which do not represent the complete routes of preparation in practice. The actual preparation process can also include any necessary procedures such as micro-modification, protection/deprotection, intermediate preparation, workup, purification, etc., which are familiar to a person skilled in the art. 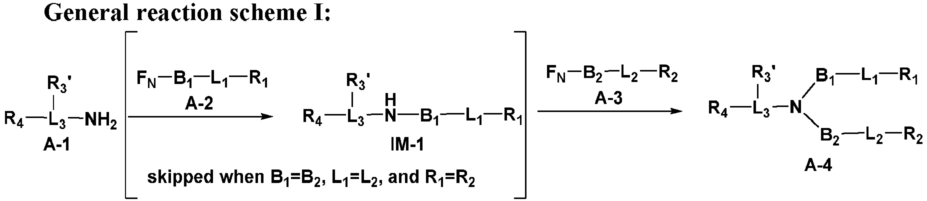
wherein, the structures of R₃' and R₃ can be the same or different; when the specific structures of R₃' and R₃ are different, R₃' can be transformed into R₃ by a micro-modification before or after any reaction step; said micro-modification is selected from the group consisting of deprotection, salt complexation and decomplexation, ionization, protonation, deprotonation, changing leaving groups, and combinations of any two or more thereof; R₃ is preferably a deprotected form of R₃', for example, -OH transformed from -OTBS through deprotection reaction;
wherein, F_{N} is a reactive group capable of reacting with amino or secondary amino groups, preferably selected from the group consisting of -OMs, -OTs, -CHO, -F, -Cl, -Br, -COOH, -COCl, and an activated carboxyl group; said activated carboxyl group refers to one obtained from the activation of a carboxyl group by a carboxyl activating agent;
wherein, the definitions of L₁, L₂, L₃, B₁, B₂, R₃, R₄, R₁, and R₂ are in line with those of the general formula (1), without further descriptions.
wherein, **A-2** and **A-3** can be prepared using the following method: 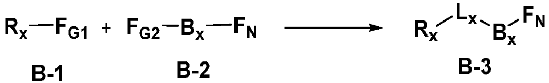
wherein, x is 1 or 2; when x is 1, **B-3** is **A-2;** when x is 2, **B-3** is **A-3;**
wherein, F_{G1} and F_{G2} are reactive groups and form the divalent linking group L₁ or L₂; F_{G1} and F_{G2} are preferably each independently selected from the group consisting of -OH, -COOH, -F, -Cl, Br, -OMs, -OTs, -CHO, -COCl, -NH₂, and an active ester; for example, **B-1** is 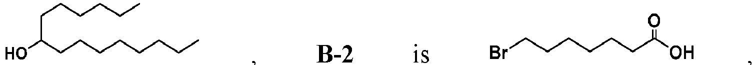 and **B-3** 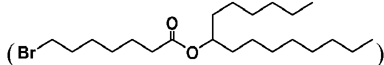 can be obtained from the esterification reaction between **B-1** and **B-2,** wherein F_{N} is -Br. 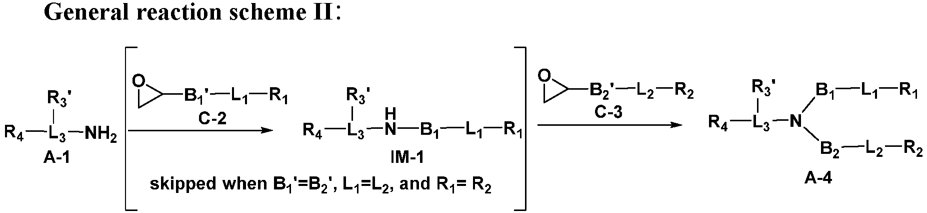 or 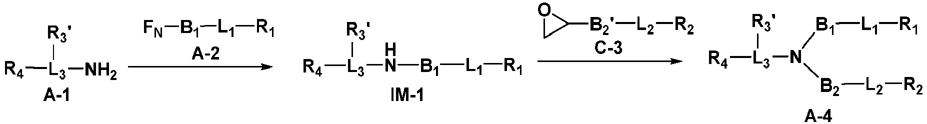 or 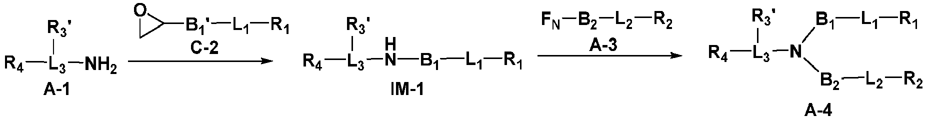
wherein, the transformation between R₃' and R₃ and the definition of Frr refer to those in the **general reaction scheme I;** the B₁ obtained from B₁' contains one hydroxyl group, and the B₂ obtained from B₂' contains one hydroxyl group;
for example, 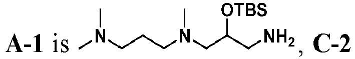 is 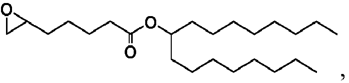 and the structure of IM-1 obtained from the reaction between A-1 and C-2 is 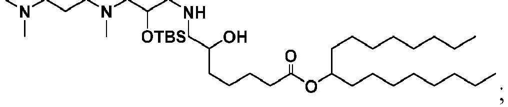 wherein, B₁' is a butylene group, and B₁ is 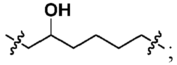
wherein, the definitions of L₁, L₂, L₃, B₁, B₂, R₃, R₄, R₁, and R₂ are in line with those of the general formula (1). 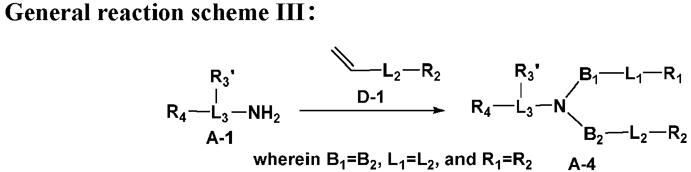
wherein, B₁ and B₂ are both ethylene groups;
wherein, the transformation between R₃' and R₃ and the definition of Frr refer to those in the **general reaction scheme I;**
wherein, L₁ and L₂ are both -C(=O)O(CH₂)_{y}OC(=O)-, and y is an integer in the range of 2-8
for example, the structure of **D-1** is 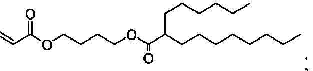
wherein, the definitions of L₁, L₂, L₃, B₁, B₂, R₃, R₄, R₁, and R₂ are in line with those of the general formula (1).

### 3.2. Specification for relevant starting materials and/or procedures in the preparation process

### 3.2.1. Carboxyl activating agents, condensing agents, oxidizing agents, and reducing agents

In the present invention, the term "activation of carboxyl group" refers to the activation of carboxyl groups with carboxyl activating agents. The activated carboxyl groups can promote condensation reactions, for example, by inhibiting the generation of racemic impurities, by accelerating the reactions through catalysis, etc. The "carboxyl activating group" is a residue of a carboxyl activating agent. Said carboxyl activating agent is selected from the group consisting of *N*-hydroxysuccinimide (NHS), 1-ethyl-3-(3-dimethylaminopropyl)carbodiimide hydrochloride (EDCI), *N*-hydroxy-5-norbornene-2,3-dicarboximide (HONb), *N*,*N*'-dicyclohexylcarbodiimide (DCC), and combinations thereof, preferably the combination of NHS/EDCI, NHS/DCC, or HONb/DCC.

In the present invention, the condensing agents used in reactions are not particularly limited, preferably selected from the group consisting of *N*,*N'*-dicyclohexylcarbodiimide (DCC), 1-ethyl-3-(3-dimethylaminopropyl)carbodiimide hydrochloride (EDC·HCl), *o*-(7-azabenzotriazol-1-yl)-*N*,*N*,*N'*,*N'*-tetramethyluronium hexafluorophosphate (HATU), and *o*-(benzotriazol-1-yl)-*N*,*N*,*N'*,*N'*-tetramethyluronium hexafluorophosphate (HBTU), most preferably DDC. Generally, the molar equivalent of the condensing agent used is 1 to 20 times that of carboxylic acids, preferably 5 to 10 times. Appropriate catalysts (e.g., 4-dimethylaminopyridine (DMAP)) can be added in the reaction.

In the present invention, the oxidizing agent used in the reaction is not particularly limited, as long as it is a compound or a combination of various compounds that can increase the valence of the substrate, preferably selected from the group consisting of phenyliodine(III) bis(trifluoroacetate), 1,4-benzoquinone, benzyl trimethyl ammonium tribromide, pyridinium dichromate, potassium dichromate, ozone, oxygen, hydrofluoric acid, sodium hypochlorite, cobaltic acetate, cobalt acetate, manganous acetate, palladium(II) acetate, cupric acetate, monoperoxyphthalic acid, iodine, *N*-iodosuccinimide, iodylbenzene, 2-iodylbenzoic acid, dimethyldioxirane, dimethyl sulfoxide-oxalyl chloride, DDQ, dichlorotris(triphenylphosphine)ruthenium, manganese dioxide, (diacetoxyiodo)benzene, periodic acid, sodium periodate, sodium periodate-osmium tetraoxide, potassium permanganate, sodium perborate, perbenzoic acid, dibenzoyl peroxide, nickel peroxide, hydrogen peroxide, cumyl hydroperoxide, *t*-butyl hydroperoxide, peracetic acid, *m*-chloroperbenzoic acid, *N*-chlorosuccinimide, pyridinium chlorochromate, palladium chloride-cupric chloride, urea hydrogen peroxide adduct, triphenylcarbenium tetrafluoroborate, tributyltin oxide, cobalt trifluoride, vanadium oxytrifluoride, chromium trioxide, manganese triacetate, TEMPO, diammonium cerium nitrate, bromine, pyridine *N*-oxide, silver oxide, *O*-ethylperoxycarbonic acid, manganese acetylacetonate, vanadyl acetylacetonate, aluminum isopropoxide, potassium peroxymonosulfate, dichloroiodobenzene, etc., and combinations thereof, and more preferably selected from the group consisting of oxygen, sodium hypochlorite, hydrogen peroxide, dichloroiodobenzene, potassium peroxymonosulfate, etc., and combinations thereof. The molar equivalent of the oxidizing agent used is 1 to 50 times that of the hydroxyl groups in intermediate compounds, preferably 1 to 20 times, and more preferably 5 to 10 times.

In the present invention, the reducing agent used in the reaction is not particularly limited, as long as the Schiff base formed via the reaction between an amine and an aldehyde or ketone can be reduced to an amino group; said reducing agent is preferably selected from the group consisting of sodium borohydride, sodium cyanoborohydride, lithium aluminum hydride, borane, diborane, diisobutylaluminum hydride, diisopinocampheylborane, lithium borohydride, zinc borohydride, borane-pyridine, borane-methyl sulfide, borane-tetrahydrofuran, etc., and combinations thereof, and more preferably sodium cyanoborohydride. The molar equivalent of the reducing agent used is 1 to 50 times that of the amino groups to be modified, preferably 1 to 20 times, and more preferably 5 to 10 times.

In the present invention, the reaction temperature is 0 to 200°C, preferably 0 to 100°C, and more preferably 0 to 25°C. The reaction time is preferably 10 min to 48 h, and more preferably 30 min to 24 h. The obtained product can be purified by a purification means such as extraction, recrystallization, adsorption treatment, precipitation, reverse precipitation, membrane dialysis, supercritical extraction, etc.

In the present invention, reactions may be solvent-free or with aprotic solvents; said aprotic solvent includes toluene, benzene, xylene, acetonitrile, ethyl acetate, diethyl ether, tert-butyl methyl ether, tetrahydrofuran, chloroform, dichloromethane, dimethylsulfoxide, dimethylformamide, and dimethylacetamide, and is preferably tetrahydrofuran, dichloromethane, dimethylsulfoxide, or dimethylformamide.

In the present invention, the bases used in reactions can be organic bases (e.g., triethylamine, pyridine, 4-dimethylaminopyridine, imidazole, or *N*,*N*-diisopropylethylamine), preferably triethylamine or pyridine. The molar equivalent of the base used is 1 to 50 times that of carboxylic acids, preferably 1 to 10 times, and more preferably 2 to 3 times. Said bases can also be inorganic bases such as potassium carbonate (K₂CO₃).

### 3.2.2. "Protection" and "deprotection" of relevant groups involved in the reaction process

In the present invention, a reaction process may involve processes of "protection" and "deprotection" of relevant groups. In order to avoid effects of a certain functional group in a reaction, said functional group is usually protected. In addition, when there are two or more functional groups and selectively only the target functional group needs to react, the rest functional groups should therefore be protected. The protecting group not only protects the functional group stably, but also needs to be removed easily as needed. Therefore, in organic synthesis, it is important to remove, under appropriate conditions, only the protecting group that is bonded to the designated functional group.

In the present invention, a "carboxyl protecting group" refers to the protecting group which can be transformed into a carboxyl group via hydrolysis or deprotection. A carboxyl protecting group is preferably an alkyl group (e.g., methyl, ethyl, tert-butyl) or an aralkyl group (e.g., benzyl), and more preferably a tert-butyl group (tBu), a methyl group (Me), or an ethyl group (Et). In the present invention, a "protected carboxyl group" refers to the group formed via the protection of a carboxyl group with an appropriate carboxyl protecting group, and is preferably a methoxycarbonyl group, an ethoxycarbonyl group, a *t*-butoxycarbonyl group, or a benzyloxycarbonyl group. Said carboxyl protecting group can be removed through hydrolysis catalyzed by acids or alkalis, or through pyrolysis reactions occasionally; for example, *t*-butyl groups can be removed under mild acidic conditions, and benzyl groups can be removed by hydrogenolysis. The reagents used for the removal of carboxyl protecting groups are selected from the group consisting of TFA, H₂O, LiOH, NaOH, KOH, MeOH, EtOH, and combinations thereof, preferably the combination of TFA and H₂O, the combination of LiOH and MeOH, or the combination of LiOH and EtOH. A protected carboxyl group can undergo deprotection and then produce the corresponding free acid; said deprotection can be conducted in the presence of an alkali, and said alkali forms pharmaceutically acceptable salts with said free acid obtained from said deprotection.

In the present invention, hydroxyl groups protected by hydroxyl protecting groups are not particularly limited, which can be, for example, alcoholic hydroxyl groups, phenolic hydroxyl groups, etc. Amino/amine groups protected by amino protecting groups are not particularly limited, which can be, for example, those from primary amines, secondary amines, hydrazines, amides, etc. Amine groups in the present invention are not particularly limited, including but not limited to primary amine groups, secondary amine groups, tertiary amine groups, and quaternary ammonium ions.

In the present invention, the deprotection of protected hydroxyl groups is related to the type of hydroxyl protecting group. Said type of hydroxyl protecting group is not particularly limited; for example, benzyl, silyl ether, acetal, or tert-butyl groups can be used to protect terminal hydroxyl groups, and the corresponding deprotection methods include but are not limited to the follows:

### A: Deprotection of benzyl groups

The deprotection of benzyl groups can be achieved via hydrogenation using hydrogenation reducing agents and hydrogen donors. The water content in the reaction system should be less than 1% in order to facilitate the reaction.

The catalyst for hydrogenation reduction is not particularly limited, preferably palladium or nickel. The catalyst support is not particularly limited, preferably aluminum oxide or carbon, and more preferably carbon. The amount of palladium used is 1 to 100 wt% relative to the compounds containing protected hydroxyl groups, preferably 1 to 20% wt%.

The reaction solvent is not particularly limited, as long as both the starting materials and the products can be dissolved, but is preferably methanol, ethanol, ethyl acetate, tetrahydrofuran, or acetic acid, more preferably methanol. The hydrogen donor is not particularly limited, but is preferably gaseous hydrogen, cyclohexene, 2-propanol, or ammonium formate, etc. The reaction temperature is preferably in the range of 25 to 40°C. The reaction time is not particularly limited, which is negatively correlated with the amount of catalyst used and preferably in the range of 1 to 5 hours.

### B: Deprotection of acetals and ketals

The compounds used for protecting hydroxyl groups in the forms of acetals or ketals are preferably ethyl vinyl ether, tetrahydropyran, acetone, 2,2-dimethoxypropane, or benzaldehyde, etc. The deprotection of such acetals or ketals can be realized under an acidic condition wherein the pH of the solution is preferably 0 to 4. The acid used is not particularly limited, but is preferably acetic acid, phosphoric acid, sulfuric acid, hydrochloric acid, or nitric acid, and more preferably hydrochloric acid. The reaction solvent is not particularly limited, as long as the reactants and the products can be dissolved, but is preferably water. The reaction temperature is preferably 0 to 30°C.

### C: Deprotection of silyl ethers

The protected hydroxyl groups in the forms of silyl ethers include trimethylsilyl ether, rimethylsilyl ether, tert-butyldimethylsilyl ether, tert-butyldiphenylsilyl ether, etc. The deprotection of such silyl ethers uses compounds containing fluoride ions; wherein, said compounds are preferably selected from the group consisting of tetrabutylammonium fluoride, tetraethylammonium fluoride, hydrofluoric acid, and potassium fluoride, and more preferably tetrabutylammonium fluoride or potassium fluoride. The molar equivalent of the fluorine-containing compound used is 5 to 20 times that of the protected hydroxyl group, preferably 8 to 15 times that of the initiator. When the molar equivalent of the fluorine-containing compound used is less than 5 times that of the protected hydroxyl group, the deprotonation might be incomplete. When the molar equivalent of the deprotection reagent used exceeds 20 times that of the protected hydroxyl group, the excess reagents or compounds will bring difficulty in the purification and possibly cause side reactions in subsequent steps. The reaction solvent is not particularly limited, as long as the reactants and the products can be dissolved, but is preferably an aprotic solvent and more preferably tetrahydrofuran or dichloromethane. The reaction temperature is preferably 0 to 30°C; when the temperature is lower than 0°C, the reaction rate is relatively slow, and the protecting group cannot be completely removed.

### D: Deprotection of tert-butyl groups

The deprotection of tert-butyl groups is carried out under an acidic condition wherein the pH of the solution is preferably 0 to 4. The acid used is not particularly limited, but is preferably acetic acid, phosphoric acid, sulfuric acid, hydrochloric acid, or nitric acid and more preferably hydrochloric acid. The reaction solvent is not particularly limited, as long as the reactants and the products can be dissolved, but is preferably water. The reaction temperature is preferably 0 to 30°C.

With respect to the methods for end-functionalization, it is preferred that q is 0, q₁ is 1, and Z₁ is a 1,2-methylene group. When q is not 0 and a linking group (e.g., amino acid group, succinyl group, etc.) is present between A and R₀₁, the prior art (including but not limited to alkylation, condensation, click reactions, etc.) capable of generating Z₂ or Z₁ can be used, and the preparation can be carried out referring to the following linear functionalization methods.

### 3.2.3. Alkylation reactions

In the present invention, alkylation reactions are preferably those based on hydroxyl groups, thiol groups, or amino groups, corresponding to the formation of ether bonds, thioether bonds, and secondary or tertiary amino groups, respectively. Examples are as follows:

### (1) Alkylation reaction of alcohol substrates with sulfonates or halides

An amine intermediate can be obtained via the nucleophilic substitution with a sulfonate derivative or halide on the alcohol substrate under basic conditions. Wherein, the molar equivalent of the sulfonate or halide is 1 to 50 times that of the alcohol substrate, preferably 1 to 5 times. When the molar equivalent of the sulfonate or halide is less than 1 fold of that of the alcohol substrate, the substitution might be incomplete and cause difficulty in the purification process. When the molar equivalent of the sulfonate or halide exceeds 50 times that of the alcohol substrate, the excess sulfonate or halide tends to bring difficulty in the purification process, and might be brought into the subsequent step and therefore cause more side reactions which further increases difficulty in the purification.

The resulting product is a mixture of ether intermediate and excess sulfonate or halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or organic solvents. Said organic solvent is not particularly limited, as long as the product can be dissolved within, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base used can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50, preferably 1 to 10, more preferably 3 to 5 times that of the sulfonate or halide.

### (2) Alkylation reaction of amine substrates with aldehyde derivatives

The amine substrate reacts with an aldehyde derivative to obtain an imine intermediate, which is followed by obtaining an intermediate using reducing agents. Wherein, the molar equivalent of the aldehyde derivative is 1 to 20, preferably 1 to 2, more preferably 1 to 1.5 times that of the amine substrate. When the molar equivalent of the aldehyde derivative exceeds 20 times that of the amine substrate, the excess reagent tends to cause difficulty in the purification process, and might be brought into the subsequent step and therefore increase difficulty in the purification. When the molar equivalent of the aldehyde derivative is less than 1 fold of that of the amine substrate, the reaction might be incomplete, causing further difficulty in the purification. Wherein, the resulting product can be obtained after purification by means such as cation exchange resin, osmosis, ultrafiltration, etc. Said cation exchange resin is not particularly limited, as long as it can undergo ion-exchange with quaternary ammonium cations and realize the isolation. The solvents used for osmosis or ultrafiltration treatment are not limited, generally water or organic solvents. Said organic solvent is not particularly limited, as long as the product can be dissolved within, but is preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited, as long as the imine can be reduced to an amine, but is preferably sodium borohydride, lithium aluminum hydride, sodium cyanoborohydride, or Zn/AcOH, etc., and more preferably sodium cyanoborohydride. The amount of the reducing agent used is generally 0.5 to 50 times, preferably 1 to 10 times that of the aldehyde derivative.

### 3.2.4. Linear end-functionalization of the side-chain R₃

The method for linear end-functionalization of the side-chain R₃ is not particularly limited, but is related to the type of the final functional group or its protected from. An R₃ modified by linear end-functionalization still belongs to the scope of R₃ of the present invention.

### (1) Functionalization of terminal hydroxyl groups

The terminal hydroxyl group of F₁ can be functionalized to obtain a functional group selected from the aforementioned classes A~I or protected forms thereof. An exemplary transformation is from -(CH₂)ₕ-R₀₁ to -(CH₂)_{f}-Z₃-(CH₂)_{g}-R₀₁, wherein h is an integer in the range of 0-6, f is 0, 1, or 2, and g is 2, 3, or 4; wherein, Z₃ is selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is, at each occurrence, independently H or methyl, preferably H. Specific methods for the preparation include but are not limited to those disclosed in the paragraphs [0960] to [1205] of the document CN104530417A.

### (2) Transformation of reactive groups into target functional groups or protected forms thereof

Method 1: direct modification. The target functional group or its protected form can be obtained via the direct modification of a reactive group. Exemplary direct modifications include the transformation of a carboxyl group into an acyl halide group, a hydrazide group, an ester group, a thioester group, or a dithioester group, the transformations of a hydroxyl group, a thiol group, an alkynyl group, an amino group, a carboxyl group, etc., into the corresponding protected forms, and the modifications of a hydroxyl group, an amino group, etc., with anhydrides.

Method 2: coupling reaction of two reactive groups. A heterofunctional reagent is used as a starting material containing 1 type of reactive group as well as the target functional group, and said reactive group reacts with the terminal reactive group of F₁ to introduce the target functional group or its protected form. The reaction of two reactive groups is not particularly limited with respect to the manner or method, and the reaction conditions are relevant to the type of the divalent linking group formed in the reaction. Prior arts such as alkylation, addition of alkenes, addition of alkynes, Schiff base reaction-reduction reaction, condensation, etc., can be used herein. Wherein, the alkylation reaction is preferably based on thiol group or amino group, corresponding to the formation of thioether bond and secondary or tertiary amino group, respectively. Wherein, the condensation reaction includes but is not limited to those producing ester groups, thioester groups, amide groups, imine bonds, hydrazone bonds, carbamate groups, etc. The target functional group or its protected form can also be introduced via click reactions using starting materials including a heterofunctional reagent containing not only a reactive group selected from the group consisting of an azido group, an alkynyl group, an alkenyl group, a tri-thioester group, a thiol group, a diene group, a furyl group, a 12,4,5-tetrazinyl group, a cyanate group, etc., but also the target functional group or its protected form. The reaction of two reactive groups is accompanied by the formation of new bonds. Typical representatives of newly formed divalent linking groups include amide bond, urethane bond, ester group, secondary amine bond, thioether bond, triazole group, etc.

Method 3: combination of direct modifications and coupling reactions. The target functional group or its protected form can be obtained via said combination.

### 4. Lipid compositions, lipid pharmaceutical compositions and formulations thereof

### 4.1. Lipid compositions

In one specific embodiment, lipid compositions form a liposome which is preferably a lipid nanoparticle (LNP).

Lipid compositions of the present invention can be used to deliver bioactive substances to one or more of the following physiological sites of a patient: liver or liver cells (such as hepatocytes), kidney or kidney cells, tumor or tumor cells, CNS (central nervous system, such as brain and/or spinal cord) or CNS cells, PNS (peripheral nervous system) or PNS cells, lung or lung cells, blood vessels or blood vessel cells, skin or skin cells (such as dermal cells and/or follicular cells), eye (such as macula, fovea, cornea, retina) or eye cells, ear or ear cells (such as inner ear, middle ear and/or outer ear cells).

In one embodiment,
provided herein is a lipid composition containing any aforementioned cationic lipid.

In one specific embodiment, the aforementioned lipid composition contains also one or more types of lipids selected from the group consisting of phospholipid, steroid lipid, and PEGylated lipid, belonging to any of the following cases:
Case (1): contains also a phospholipid;
Case (2): contains also a steroid lipid;
Case (3): contains also a PEGylated lipid;
Case (4): contains also a phospholipid and a steroid lipid;
Case (5): contains also a phospholipid and a PEGylated lipid;
Case (6): contains also a steroid lipid and a PEGylated lipid;
Case (7): contains also a phospholipid, a steroid lipid, and a PEGylated lipid;
and preferably contains also three types of lipids consisting of phospholipid, steroid lipid, and PEGylated lipid, simultaneously.

In one specific embodiment, the phospholipid in the aforementioned lipid composition is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-*O*-octadecenyl-sn-glycero-3-phosphatidylcholine, 1-oleoyl-2-cholesterylhemisuccinyl-sn-glycero-3-phosphocholine, 1-*O*-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoylphosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof.

In one specific embodiment, the steroid lipid in the aforementioned lipid composition is selected from the group consisting of cholesterol, coprostanol, sitosterol, ergosterol, campesterol, stigmasterol, brassicasterol tomatidine, ursolic acid, α-tocopherol, and mixtures thereof.

In one specific embodiment, the PEGylated lipid in the aforementioned lipid composition is selected from the group consisting of 1,2-dimyristoyl-sn-glycerol-methoxypolyethylene glycol, 1,2-distearoyl-sn-glycero-3-phosphoethanolamine-*N*-[amino(polyethylene glycol)], PEG-cholesterol, polyethylenglycol-diacylgricerol conjugate, and polytheylyene-dialkyloxypropyl conjugate, and specifically the group consisting of PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, and PEG2000-2,3-distearoylglycerol.

In one specific embodiment, the PEGylated lipid in the aforementioned lipid composition is any PEGylated lipid of the general formula (2).

In one specific embodiment, the structure of the PEGylated lipid in the aforementioned lipid composition is selected from the group consisting of the following: 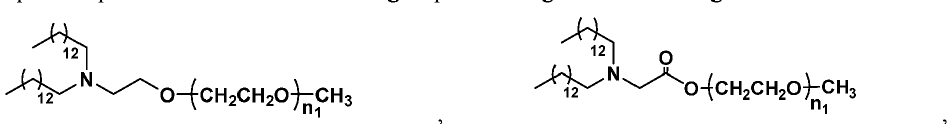 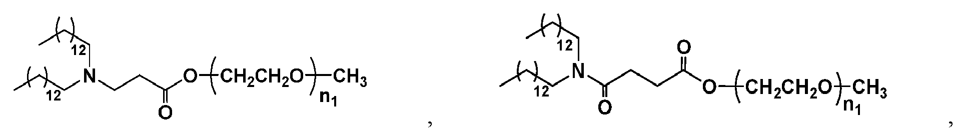 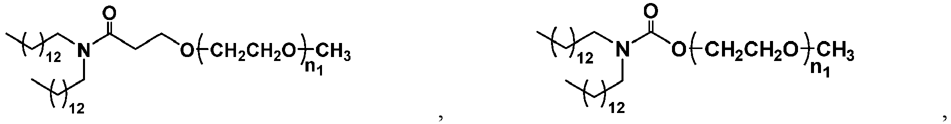 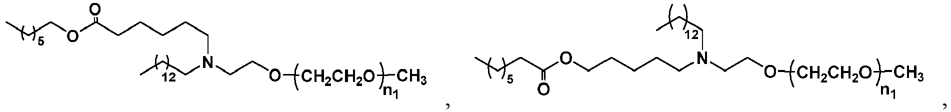 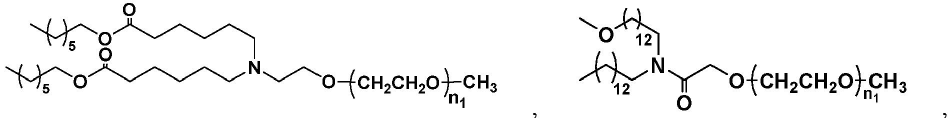 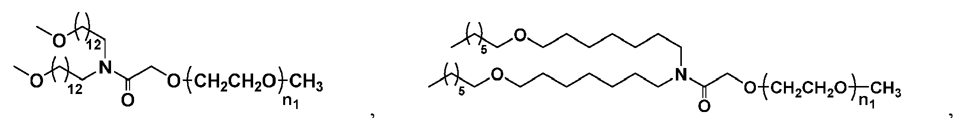 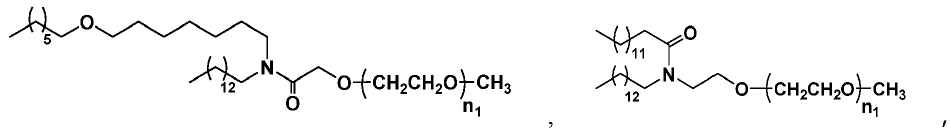 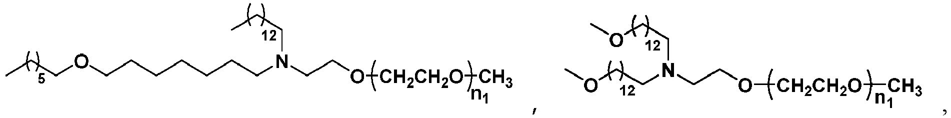 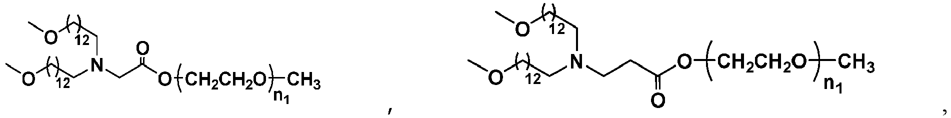 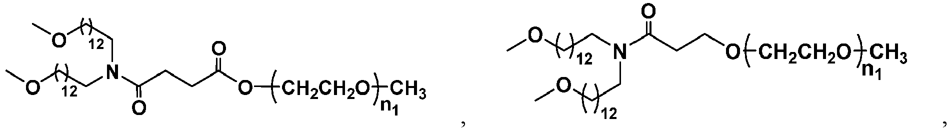 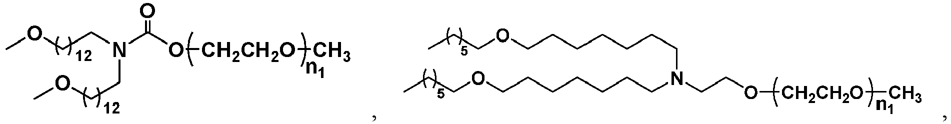 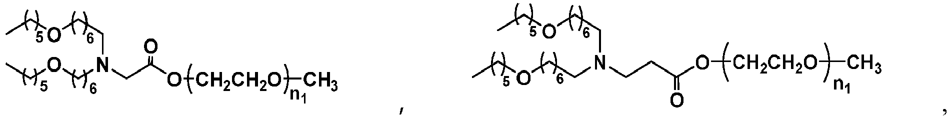 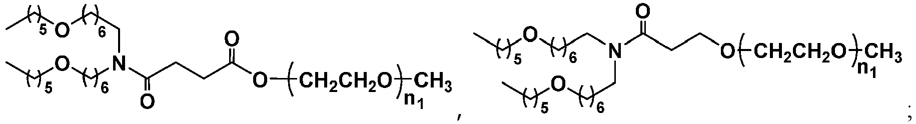 wherein, n₁ is an integer in the range of 20-250.

In one specific embodiment, the molar percentages of PEGylated lipid, cationic lipid, neutral lipid and steroid lipid in total lipids of the aforementioned lipid compositions are not particularly limited; preferably,
the molar percentage of PEGylated lipid in total lipids is 0.5-5%, preferably 1-3%, and more preferably 1.5%, 1.6%, 1.7%, 1.8%, or 1.9%;
the molar percentage of cationic lipid in total lipids is 30-65%, preferably 35%, 40%, 45%, 46%, 47%, 48%, 49%, 50%, or 55%;
the molar percentage of phospholipid in total lipids is 7.5-13%, preferably 8%, 9%, 10%, 11%, or 12%;
the molar percentage of steroid lipid in total lipids is 35-50%, preferably 40%, 41%, 42%, 43%, 44%, 45%, 46%, 47%, 48%, 49%, or 50%.

In one specific embodiment, the aforementioned lipid composition contains 20-80% cationic lipids of the general formula (1), 5-15% neutral lipids, 25-55% steroid lipids, and 0.5-10% PEGylated lipids, said percentages being the molar percentages of each type of lipids in total lipids.

### 4.2. Lipid pharmaceutical compositions and formulations thereof

In one embodiment,
provided herein is a lipid pharmaceutical composition containing any aforementioned lipid composition and a drug selected from the group consisting of nucleic acid drug, genetic vaccine, anti-neoplastic drug, small molecule drug, peptide drug, and protein drug.

In one specific embodiment, the drug contained in the aforementioned lipid pharmaceutical composition is a nucleic acid drug selected from the group consisting of DNA, RNA, antisense nucleic acid, plasmid, interfering nucleic acid, aptamer, antagomir, and ribozyme, wherein said RNA is selected from the group consisting of mRNA, saRNA, circRNA, miRNA, and siRNA; said nucleic acid drug is preferably selected from the group consisting of DNA, mRNA, miRNA, and siRNA.

In one specific embodiment, the aforementioned lipid pharmaceutical composition is used as a medicine selected from the group consisting of a drug for treating cancer, an anti-infective agent, an antibiotic agent, an antiviral agent, an antifungal agent, and a vaccine.

In one specific embodiment, the lipid composition in the aforementioned lipid pharmaceutical composition forms an LNP; said lipid pharmaceutical composition is an LNP pharmaceutical composition, preferably an LNP-nucleic acid pharmaceutical composition, and more preferably an LNP-mRNA composition.

In one specific embodiment, the drug contained in the aforementioned lipid pharmaceutical composition includes but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, maytansinoid, and oxaliplatin.

In one specific embodiment, the drug contained in the aforementioned lipid pharmaceutical composition is a nucleic acid drug, and the N/P ratio is preferably (0.1~100):1, more preferably (0.2~30):1, and most preferably (0.5~20):1.

In one embodiment,
provided herein is a formulation of lipid pharmaceutical composition which contains any aforementioned lipid pharmaceutical composition and a pharmaceutically acceptable diluent or excipient; said diluent or excipient is preferably deionized water, ultrapure water, phosphate buffer, or physiological saline, more preferably phosphate buffer or physiological saline, and most preferably physiological saline.

In one specific embodiment, the ratio of lipid composition to working solution in the aforementioned formulation of lipid pharmaceutical composition is not particularly limited, preferably (0.05~20) g:100 mL, more preferably (0.1~10) g:100 mL, and most preferably (0.2~5)g:100 mL.

### 4.3. Preparation methods

In one specific embodiment, the preparation of the formulation of lipid pharmaceutical composition includes the following steps:
(1) equilibrate the lipid components in the diluent or excipient;
(2) add the drug to the mixture containing the equilibrated liposome and the diluent or excipient for complexation;
wherein, the equilibration time is not particularly limited, preferably 0.1~12 h, more preferably 0.2-6 h, and most preferably 0.5-3 h; wherein, the complexation time is not particularly limited, preferably 0.1-12 h, more preferably 0.2-5 h, and most preferably 0.5-2 h.

In one specific embodiment, the preparation of the LNP-nucleic acid pharmaceutical composition includes the following steps:
(1) dissolve the lipid components with an organic solvent to obtain an organic phase solution;
(2) add the nucleic acid drug to a buffer solution to obtain an aqueous phase solution;
(3) mix the organic phase solution with the aqueous phase solution to obtain the LNP-nucleic acid pharmaceutical composition, wash the mixture using ultrafiltration to remove the organic solvent and free molecules, and finally perform a filtration using a sterile filter for further use;
wherein, said organic solvent is preferably selected from the group consisting of methanol, ethanol, propanol, tert-butanol, acetonitrile, dimethyl sulfoxide, *N*,*N*-dimethylformamide, *N*,*N*-dimethylacetamide, *N*-methylpyrrolidone, and mixtures thereof; said buffer solution is preferably a citrate buffer which further preferably has the concentration of 5-80 mM and the pH of 2-6, and more preferably the concentration of 10-50 mM and the pH of 3-5; the volumetric ratio of organic phase solution to aqueous phase solution is preferably 1:1-10, more preferably 1:2.

In one specific embodiment, the particle size of lipid nanoparticle is controlled by ultrasonic, extrusion, or microfluidic devices; said particle size is preferably 1-1000 nm, more preferably 20-500 nm, more preferably 60-200 nm, and most preferably 60-150 nm.

### 5. Specific embodiments

The following specific examples are for further description of the preparation of cationic lipids, lipid compositions, and lipid pharmaceutical compositions, and the biological activity assays for LNP-nucleic acid pharmaceutical compositions. Said specific examples are for further detailed illustration of the present invention, not limiting the scope of the present invention.

### 5.1. Preparation of lipid compounds

### Example 1: cationic lipid E1-1

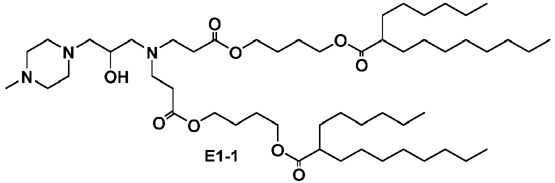

**E1-1** corresponds to the general formula (1); wherein, R₁ and R₂ are both 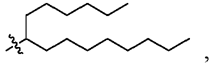 B₁ and B₂ are both ethylene groups, L₁ and L₂ are both -C(=O)O-(CH₂)₄-OC(=O)-, -L₃(R₃)- is 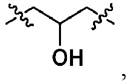 R₃ is -OH, and R₄ is 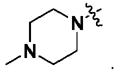 The molecular weight is approximately 938 Da.

The preparation process is as follows:
**S1-1** (2.53 g, 6.6 mmol, prepared via the condensation reaction of 2-hexyldecanoic acid and 4-hydroxybutyl acrylate) was dissolved with 40 mL methanol, added with the derivative of 1-amino-3-(4-methyl-1-piperazinyl)-2-propanol containing a TBS-protected hydroxyl group **(S1-2,** 0.86 g, 3.0 mmol), and reacted overnight at 35°C while stirring. After the reaction was over, the reaction solution was dried over anhydrous sodium sulfate, filtered, concentrated under reduced pressure, and then added with 20 mL tetrahydrofuran and further with 20 mL 1 M solution of tetra-*n*-butylammonium fluoride (TBAF) in tetrahydrofuran (THF). The deprotection reaction was continued overnight and the TBS was removed. After the reaction was over, the reaction solution was concentrated, extracted, and then the organic phases were combined, dried over anhydrous sodium sulfate, filtered and concentrated. The crude product was purified by silica gel column chromatography to obtain **E1-1** (2.59 g). The main data of the ¹H-NMR spectrum of **E1-1** are as follows: ¹H NMR (400 MHz, CDCl₃) δ: 4.10-4.06 (m, 8H), 3.88-3.81 (m, 1H), 2.79-2.24 (m, 25H) 1.71-1.19 (m, 56H), 0.85 (t, 12H). MS (ESI): m/z = 938.82 [M+H]⁺.

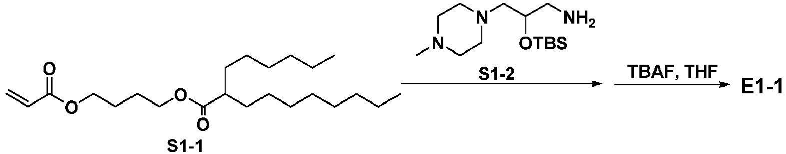

### Example 2: cationic lipid E2-1

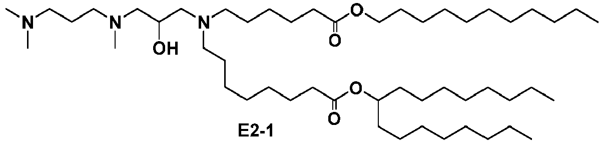

**E2-1** corresponds to the general formula (1); wherein, R₁ is an undecyl group, R₂ is 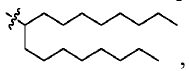 B₁ is a pentylene group, B₂ is a heptylene group, L₁ and L₂ are both ester groups (-C(=O)O-), -L₃(R₃)- is 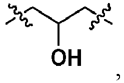 R₃ is -OH, and R₄ is 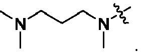 The molecular weight is approximately 838 Da.

The preparation process is as follows:
Step a: The derivative of 1-amino-3-chloro-2-propanol containing a TBS-protected hydroxyl group and a Boc-protected amino group (**S2-1**, 3.23 g, 10.0 mmol) was dissolved with dried THF (50 mL), followed by slowly adding NaH (60%, 3.98 g, 100.0 mmol) in an ice bath and reacting for 1 h in an ice bath. After the reaction was over, trimethyl-1,3-propanediamine **(S2-2,** 1.39 g, 12.0 mmol) was added and the reaction was continued for 1 h while stirring in an ice bath, followed by slowly restoring the temperature of reaction solution to room temperature and then reacting overnight. After the reaction was over, an ice bath was used and then 3 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL), dried over anhydrous sodium sulfate, filtered, concentrated, and further dissolved with dichloromethane. TFA was added until its concentration reached 0.1 M. After reaction for 4 h, the pH was adjusted to neutral, and then the reaction solution was concentrated, added with purified water, and extracted with dichloromethane. The extract was dried over anhydrous sodium sulfate, filtered, concentrated, and recrystallized to obtain the intermediate **S2-3** (2.29 g) with a free amino group.
Step b: **S2-4** (3.31 g, 7.2 mmol, prepared via the condensation reaction of 8-bromooctanoic acid and 9-heptadecanol) was dissolved with 50 mL DMF, added with **S2-3** (1.82 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phases were combined and filtered, then concentrated under reduced pressure, and finally purified by silica gel column chromatography to obtain **S2-5** (3.24 g).
Step c: The above described compound **S2-5** (2.74 g, 4.0 mmol) was dissolved with anhydrous THF (50 mL), and slowly added with NaH (60%, 1.59 g, 40.0 mmol) in an ice bath. After reaction for 1 h in the ice bath, **S2-6** (1.67 g, 4.8 mmol, prepared via the condensation reaction of 6-bromohexanoic acid and 1-undecanol) was added and reacted for 1 h in the ice bath while stirring. Then, the temperature of the solution was slowly restored to room temperature, and the reaction was continued overnight. After the reaction was over, the reaction solution was placed in an ice bath, and then added slowly with 2 mL water to quench the reaction. After stirring for 30 min, water (50 mL) was added and mixed while stirring, followed by extraction twice with dichloromethane (25 mL*2). The organic phases were collected and combined, and then washed with a saturated solution of sodium chloride (50 mL). The organic phase was concentrated and added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E2-1** which was further purified by column chromatography, concentrated, and dried with an oil pump to obtain the cationic lipid **E2-1** (2.41 g). The main data of the ¹H-NMR spectrum of **E2-1** are as follows: ¹H NMR (400 MHz, CDCl₃) δ: 4.83-4.77 (m, 1H), 4.06 (t, 2H), 3.83-3.77 (m, 1H), 2.66-2.29 (m, 16H), 2.26-2.20 (m, 9H), 1.69-1.21 (m, 64H), 0.86 (t, 9H). MS (ESI): m/z = 838.83 ([M+H]⁺).

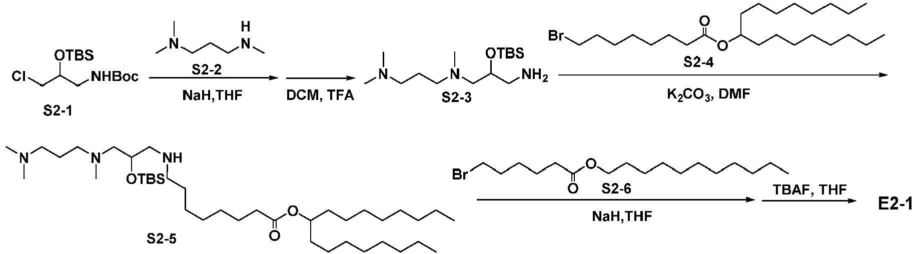

### Example 3: cationic lipids E3-1 and E3-2

### Example 3.1: preparation of the cationic lipid E3-1

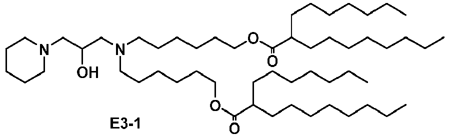

**E3-1** corresponds to the general formula (1); wherein, R₁ and R₂ are both 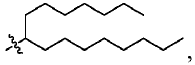 B₁ and B₂ are both hexylene groups, L₁ and L₂ are both ester groups (-OC(=O)-), -L₃(R₃)- is 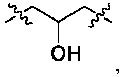 R₃ is -OH, and R₄ is 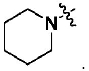 The molecular weight is approximately 863 Da.

The preparation process is as follows:
2-Heptyldecanoic acid-6-bromohexyl ester (**S3-1**, 2.85 g, 6.6 mmol, prepared via the condensation reaction of 6-bromohexanol and 2-heptyl decanoic acid) was dissolved with 50 mL DMF, added with the derivative of 1-amino-3-(1-piperidinyl)-2-propanol containing a TBS-protected hydroxyl group and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and added with 25 mL tetrahydrofuran, which was followed by adding 25 mL solution of TBAF in tetrahydrofuran (1M) and reacting overnight to remove the TBS protecting group. After the reaction was over, the reaction solution was concentrated and extracted, and the organic phases were combined, dried over anhydrous sodium sulfate, filtered and concentrated. The crude product was purified by silica gel column chromatography to obtain **E3-1** (2.10 g). The main data of the ¹H-NMR spectrum of **E3-1** are as follows: ¹H NMR (400 MHz, CDCl₃) δ: 4.08-4.01 (m, 4H), 3.84-3.75 (m, 1H), 2.62-2.19 (m, 12H), 1.73-1.51 (m, 12H), 1.46-1.20 (m, 64H), 0.86 (t, 12H). MS (ESI): m/z = 863.88 ([M+H]⁺).

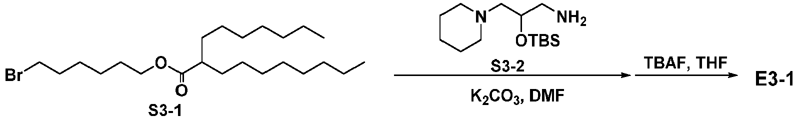

### Example 3.2: preparation of the cationic lipid E3-2

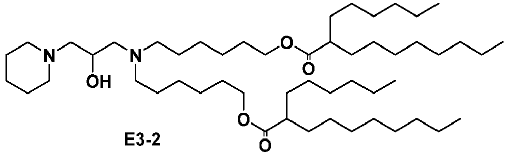

**E3-2** corresponds to the general formula (1); wherein, R₁ and R₂ are both 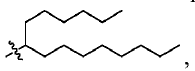 B₁ and B₂ are both hexylene groups, L₁ and L₂ are both ester groups (-OC(=O)-), -L₃(R₃)- is 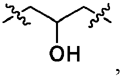 R₃ is -OH, and R₄ is 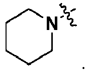 The molecular weight is approximately 835 Da.

Referring to the preparation of **E3-1**, **E3-2** was obtained via replacing **S3-1** with **S3-3** (prepared via the condensation reaction of 6-bromohexanol and 2-hexyldecanoic acid) while the rest steps were kept the same. The main data of the ¹H-NMR spectrum are as follows: ¹H NMR (400 MHz, CDCl₃) δ: 4.07-4.01 (m, 4H), 3.84-3.75 (m, 1H), 2.62-2.20 (m, 12H), 1.72-1.50 (m, 12H), 1.45-1.20 (m, 60H), 0.86 (t, 12 H). MS (ESI): m/z = 835.80 ([M+H]⁺).

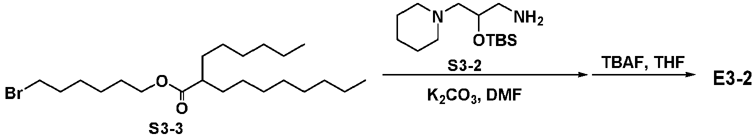

### Example 4: cationic lipid E4-1

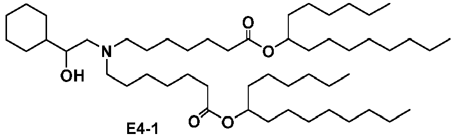

**E4-1** corresoonds to the seneral formula (1); wherein. R₁ and R₂ are both 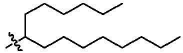 , B₁ and B₂ are both hexylene groups, L₁ and L₂ are both ester groups (-C(=O)O-), -L₃(R₃)- is 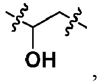 R₃ is -OH, and R₄ is 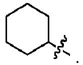 The molecular weight is approximately 820 Da.

The preparation process is as follows:
Step a: Under argon atmosphere, DCC (3.09 g, 15.0 mmol) was added into a round-bottom flask containing 7-bromoheptanoic acid (**S4-1**, 2.08 g, 10.0 mmol), 7-pentadecanol (**S4-2**, 2.51 g, 11.0 mmol) and DMAP (0.31 g, 2.5 mmol) which were dissolved in 50 mL dichloromethane, and reacted for 16 h at room temperature. After the reaction was over, the precipitates were removed by filtration. The filtrate was concentrated and the residue was purified by silica gel column chromatography to obtain 1-hexylnonyl 7-bromoheptanoate (**S4-3**, 3.49 g).
Step b: **S4-3** (2.76 g, 6.6 mmol) was dissolved with 50 mL DMF, added with the derivative of 2-amino-1-cyclohexylethanol containing a TBS-protected hydroxyl group (**S4-4**, 0.77 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and added with 25 mL tetrahydrofuran and further with 25 mL solution of TBAF in tetrahydrofuran (1M). The reaction was continued overnight to remove the TBS protecting group. After the reaction was over, the reaction solution was concentrated and extracted, and the organic phases were combined, dried over anhydrous sodium sulfate, filtered and concentrated. The crude product was purified by silica gel column chromatography to obtain **E4-1** (1.99 g). The main data of the ¹H-NMR spectrum of **E4-1** are as follows: ¹H NMR (400 MHz, CDCl₃) δ: 4.89-4.82 (m, 2H), 3.35-3.28 (m, 1H), 2.60- 2.20 (m, 10H), 1.90 (d, 1H), 1.77-1.39 (m, 20H), 1.32-0.96 (m, 54H), 0.86 (t, 12H). MS (ESI): m/z = 820.58 ([M+H]⁺).

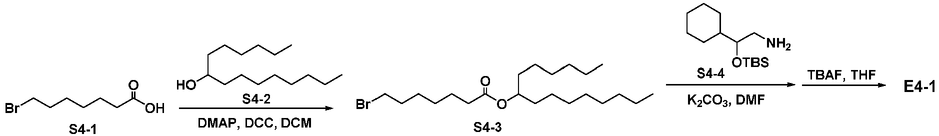

### Example 5: cationic lipid E5-1

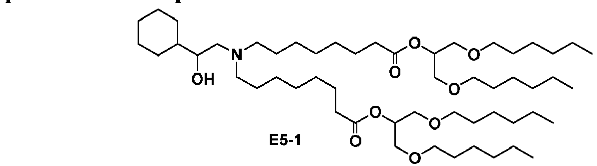

**E5-1** corresponds to the general formula (1); wherein, R₁ and R₂ are both 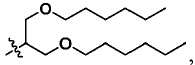 B₁ and B₂ are both heptylene groups, L₁ and L₂ are both ester groups (-C(=O)O-), -L₃(R₃)- is 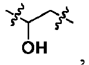 R₃ is -OH and R₄ is 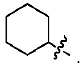 The molecular weight is approximately 912 Da.

The preparation process is as follows:
Step a: Under argon atmosphere, the derivative of glycerol containing a TBS-protected hydroxyl group (**S5-1**, 4.12 g, 20.0 mmol), K₂CO₃ (8.28 g, 60.0 mmol), and 1-bromohexane (**S5-2**, 3.61 g, 22.0 mmol) were dissolved with 100 mL DMF, and the mixture was stirred at 110°C for 16 h. After the reaction was confirmed complete by thin layer chromatography, the reaction solution was added into water (100 mL) to precipitate. The solid obtained after filtration was dissolved with 50 mL tetrahydrofuran, further added with 50 mL solution of TBAF in tetrahydrofuran (1M), and reacted overnight to remove the TBS protecting group. After the reaction was over, the reaction solution was concentrated and extracted, and the organic phases were combined, dried over anhydrous sodium sulfate, filtered, concentrated, and purified by silica gel column chromatography to obtain **S5-3** (3.02 g).
Step b: Under argon atmosphere, DCC (2.78 g, 13.5 mmol) was added into a round-bottom flask containing 8-bromooctanoic acid (**S5-4**, 2.00 g, 9.0 mmol), **S5-3** (2.57 g, 9.9 mmol) and DMAP (0.27 g, 2.3 mmol) dissolved in dichloromethane (50 mL) and reacted at room temperature for 16 h. After the reaction was over, the precipitates were removed by filtration. The filtrate was concentrated and the residue obtained was purified by silica gel column chromatography to obtain **S5-5** (3.49 g).
Step c: **S5-5** (3.06 g, 6.6 mmol) was dissolved with 50 mL DMF, added with the derivative of 2-amino-1-cyclohexylethanol containing a TBS-protected hydroxyl group (**S4-4**, 0.77 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran, and further added with 25 mL solution of TBAF in tetrahydrofuran (1M). The deprotection reaction was continued overnight to remove the TBS. After the reaction was over, the reaction solution was concentrated and extracted, and the organic phases were combined, dried over anhydrous sodium sulfate, filtered, and concentrated. The crude product was purified by silica gel column chromatography to obtain **E5-1** (2.19 g). The main data of the ¹H-NMR spectrum of **E5-1** are as follows: ¹H NMR (400 MHz, CDCl₃) δ: 4.97-4.92 (m, 2H), 3.59-3.27 (m, 17H), 2.60-2.19 (m, 10H), 1.90 (d, 1H), 1.79-1.40 (m, 20H), 1.33-0.95 (m, 42H), 0.86 (t, 12H). MS (ESI): m/z = 912.84 ([M+H]⁺).

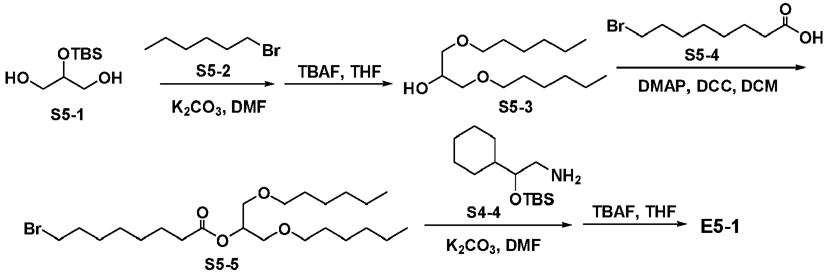

### Example 6: cationic lipid E6-1

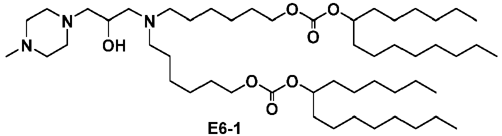

**E6-1** corresponds to the general formula (1); wherein, R₁ and R₂ are both 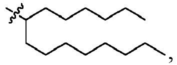 B₁ and B₂ are both hexylene groups, L₁ and L₂ are both carbonate groups (-OC(=O)O-), -L₃(R₃)- is 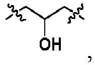 R₃ is -OH, and R₄ is 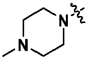 . The molecular weight is approximately 882 Da.

The preparation process is as follows:
Step a: Under nitrogen protection, 6-bromohexyl-4-nitrophenyl carbonate (**S6-1**, 10.35 g, 30.0 mmol, prepared via the condensation reaction of *p*-nitrophenyl chloroformate and 6-bromo-*n*-hexanol) was dissolved with DCM (350 mL), added dropwise by **S4-2** (27.36 g, 120.0 mmol) at room temperature while stirring, and then slowly added with pyridine (3.02 mL, 37.5 mmol) in over 10 min, and added with DMAP in one portion (0.73 g, 6.0 mmol). The reaction was continued for 16 h at room temperature while stirring. After the reaction was over, extraction was performed twice using DCM and water, respectively. The organic phases were combined and washed with brine, and then dried over anhydrous sodium sulfate, filtered, concentrated, and isolated and purified by silica gel column. The eluents were collected and concentrated to obtain **S6-2** (3.39 g).
Step b: **S6-2** (2.86 g, 6.6 mmol) was dissolved with 50 mL DMF, added with **S1-2** (0.86 g, 3.0 mmol) and K₂CO₃ (1.09 g, 7.9 mmol), and reacted overnight at room temperature while stirring. After the reaction was over, the mixture was concentrated under reduced pressure and then poured into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran, and then added with 25 mL solution of TBAF in tetrahydrofuran (1M). The deprotection reaction was continued overnight to have the TBS removed. After the reaction was over, the reaction solution was concentrated and extracted, and the organic phases were combined, dried over anhydrous sodium sulfate, filtered, and concentrated. The crude product was purified by silica gel column chromatography to obtain **E6-1** (2.20 g). The main data of the ¹H-NMR spectrum of **E6-1** are as follows: ¹H NMR (400 MHz, CDCl₃) δ: 4.15-4.04 (m, 6H), 3.38-2.92 (m, 12H), 2.88-2.37 (m, 8H), 1.75-1.20 (m, 64H), 0.88 (t, 12H). MS (ESI): m/z = 882.86 ([M+H]⁺).

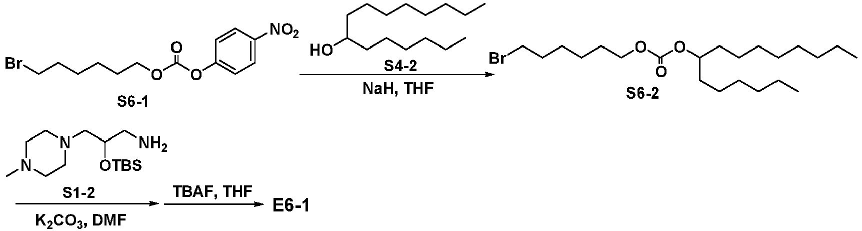

### Example 7: cationic lipid E7-1

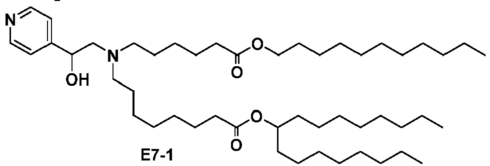

**E7-1** corresponds to the general formula (1); wherein, R₁ is an undecyl group, R₂ is 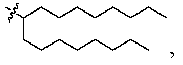 B₁ is a pentylene group, B₂ is a heptylene group, L₁ and L₂ are both ester groups (-C(=O)O-), -L₃(R₃)- is 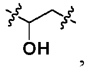 R₃ is -OH, and R₄ is 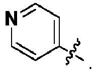 The molecular weight is approximately 787 Da.

The preparation process is as follows:
Step a: **S2-4** (4.42 g, 9.6 mmol) was dissolved with 60 mL DMF, added with the derivative of 2-amino-1-(4-pyridyl)ethanol containing a TBS-protected hydroxyl group (**S7-3**, 2.02 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column to obtain **S7-4** (4.15 g).
Step b: The above described **S7-4** (3.80 g, 6.0 mmol) was dissolved with dried THF (60 mL), followed by slowly adding NaH (60%, 2.40 g, 60.0 mmol) in an ice bath and reacting for 1 h in an ice bath. After the reaction was over, **S2-6** (2.51 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (60 mL) was added and mixed while stirring. Then, extraction was performed twice using dichloromethane (30 mL*2). The organic phases were collected and combined, and then washed with a saturated solution of sodium chloride (60 mL). The organic phase was concentrated under reduced pressure and added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E7-1** which was further purified by column chromatography, concentrated, and dried with an oil pump to obtain the cationic lipid **E7-1** (3.64 g). The main data of the ¹H-NMR spectrum of **E7-1** are as follows: ¹H NMR (400 MHz, CDCl₃) δ: 8.44 (d, 2H), 7.24 (d, 2H), 4.89-4.83 (m, 1H), 4.72-4.68 (m, 1H), 4.07 (t, 2H), 2.56-2.24 (m, 10H), 1.78-1.22 (m, 62H), 0.88 (t, 9H). MS (ESI): m/z = 787.70 ([M+H]⁺).

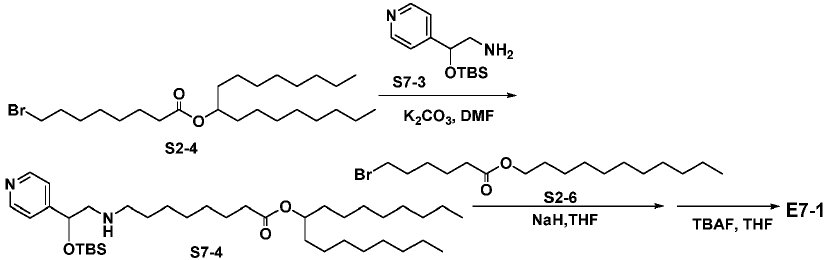

### Example 8: cationic lipid E8-1

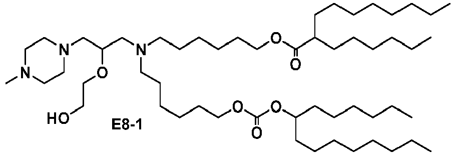

**E8-1** corresponds to the general formula (1); wherein, R₁ and R₂ are both 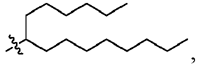 B₁ and B₂ are both hexylene groups, L₁ is an ester group (-OC(=O)-), L₂ is a carbonate group (-OC(=O)O-), -L₃(R₃)- is 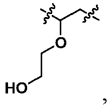 R₃ is -O(CH₂)₂OH, and R₄ is 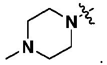 The molecular weight is approximately 910 Da.

The preparation process is as follows:
Step a: **S6-2** (3.12 g, 7.2 mmol) was dissolved with 50 mL DMF, added with **S8-1** (1.73 g, 6.0 mmol, prepared according to Example 42) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S8-2** (3.09 g).
Step b: The above described **S8-2** (2.58 g, 4.0 mmol) was dissolved with dried THF (50 mL), followed by slowly adding NaH (60%, 1.60 g, 40.0 mmol) in an ice bath and reacting for 1 h in an ice bath. After the reaction was over, **S3-3** (2.01 g, 4.8 mmol, prepared via the condensation reaction of 6-bromohexanol and 2-hexyldecanoic acid) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure, and then dissolved with methanol. The 1M hydrochloric acid was added to the solution until pH=3.5. After 4 h of reaction, the reaction solution was concentrated, precipitated, filtered, recrystallized, and dried to obtain the crude product of **E8-1** with a free hydroxyl group. The crude product was purified by column chromatography, concentrated, and dried with an oil pump to obtain the cationic lipid **E8-1** (3.02 g). The main data of the ¹H-NMR spectrum of **E8-1** are as follows: ¹H NMR (400 MHz, CDCl₃) δ: 4.24-4.03 (m, 5H), 3.91-3.82 (m, 1H), 3.70-3.52 (m, 4H), 2.68-2.20 (m, 20H), 1.75-1.22 (m, 64H), 0.89 (t, 12H). MS (ESI): m/z = 910.83 ([M+H]⁺).

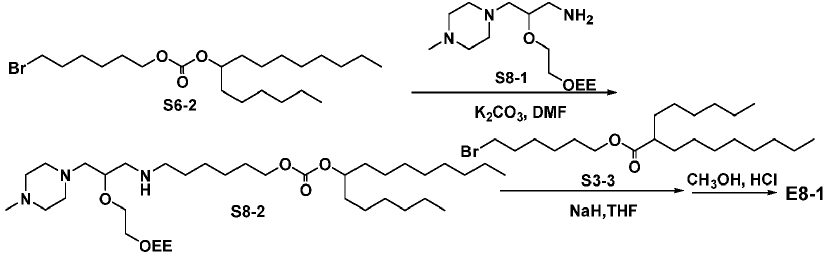

### Example 9: cationic lipid E9-1

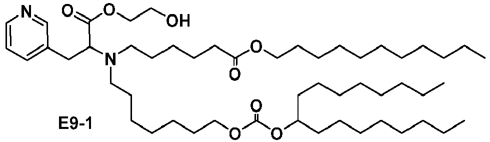

**E9-1** corresponds to the general formula (1); wherein, R₁ is an undecyl group, R₂ is 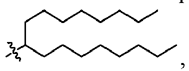 B₁ is a pentylene group, B₂ is a heptylene group, L₁ is an ester group (-C(=O)O-), L₂ is a carbonate group (-OC(=O)O-), -L₃(R₃)- is 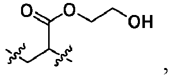 R₃ is -C(=O)O(CH₂)₂OH, and R₄ is 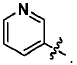 The molecular weight is approximately 875 Da.

The preparation process is as follows:
Step a: Under argon atmosphere, DCC (3.09 g, 15.0 mmol) was added into a round-bottom flask containing the derivative of 3-(3-pyridyl)-*L*-alanine containing a Boc-protected amino group (**S9-1**, 2.66 g, 10.0 mmol), the derivative of ethylene glycol containing an EE-protected hydroxyl group (**S9-2**, 1.47 g, 11.0 mmol, wherein EE is an α-ethoxyethyl ether group formed by the reaction of an ethyl vinyl ether and a hydroxyl group, -CH(CH₃)OEt) and DMAP (0.31 g, 2.5 mmol) which were dissolved in dichloromethane (50 mL), and reacted overnight at room temperature for 16 h. After the reaction was over, the precipitates were removed by filtration. The filtrate was concentrated, and then dissolved with dichloromethane. TFA was added until its concentration reached 0.1 M. After 4 h of reaction, the pH was adjusted to neutral. The reaction solution was concentrated, added with purified water, and extracted with dichloromethane. The extract was dried over anhydrous sodium sulfate, filtered, concentrated, and recrystallized to obtain the intermediate **S9-3** containing a free amino group (1.79 g).
Step b: **S9-4** (3.43 g, 7.2 mmol, prepared via the reaction of 7-bromoheptyl-4-nitrophenyl carbonate and 9-heptadecanol) was dissolved with 50 mL DMF, added with **S9-3** (1.69 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S9-5** (3.59 g).
Step c: The above described **S9-5** (2.72 g, 4.0 mmol) was dissolved with dried THF (50 mL), followed by slowly adding NaH (60%, 1.60 g, 40.0 mmol) in an ice bath and reacting for 1 h in an ice bath. After the reaction was over, **S2-6** (1.67 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure, and then dissolved with methanol. The 1M hydrochloric acid was added to the solution until pH=3.5. After 4 h of reaction, the reaction solution was concentrated, precipitated, filtered, recrystallized, and dried to obtain the crude product of **E9-1** with a free hydroxyl group. The crude product was purified by column chromatography, concentrated, and dried with an oil pump to obtain the cationic lipid **E9-1** (2.70 g). The main data of the ¹H-NMR spectrum of **E9-1** are as follows: ¹H NMR (400 MHz, CDCl₃) δ: 8.48-7.14 (m, 4H), 4.21-4.05 (m, 7H), 3.86-3.80 (m, 2H), 3.68-3.63 (m, 1H), 3.08-3.01 (m, 2H), 2.69-2.24 (m, 6H), 1.72-1.23 (m, 62H), 0.89 (t, 9H). MS (ESI): m/z = 875.74 ([M+H]⁺).

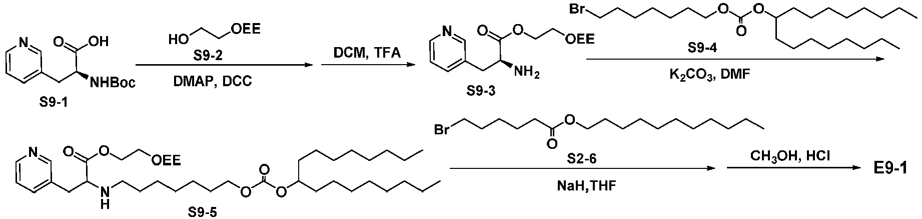

### Example 10: cationic lipids E10-1 and E10-2

### Example 10.1: preparation of the cationic lipid E10-1

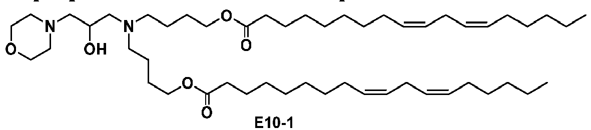

**E10-1** corresponds to the general formula (1); wherein, R₁ and R₂ are both 8,11-heptadecadienyl groups, B₁ and B₂ are both butylene groups, L₁ and L₂ are both ester groups (-OC(=O)-), -L₃(R₃)- is 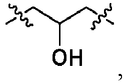 R₃ is -OH, and R₄ is 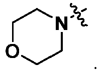 The molecular weight is approximately 829 Da.

The preparation process is as follows:
Step a: Under argon atmosphere, DCC (3.09 g, 15.0 mmol) was added into a round-bottom flask containing linoleic acid (**S10-1**, 2.80 g, 10.0 mmol), 4-bromobutanol (**S10-2**, 1.67 g, 11.0 mmol) and DMAP (0.31 g, 2.5 mmol) which were dissolved in dichloromethane (50 mL), and reacted overnight at room temperature for 16 h. After the reaction was over, the precipitates were removed by filtration. The filtrate was concentrated, and the residue was purified by silica gel column chromatography to obtain **S10-3** (3.45 g).
Step b: **S10-3** (2.73 g, 6.6 mmol) was dissolved with 50 mL DMF, added with the derivative of 1-amino-3-(4-morpholinyl)-2-propanol containing a TBS-protected hydroxyl group (**S10-4**, 0.82 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was further purified by silica gel column chromatography to obtain the cationic lipid **E10-1** (1.96 g). The main data of the ¹H-NMR spectrum of **E10-1** are as follows: ¹H NMR (400 MHz, CDCl₃) δ: 5.37-5.33 (m, 8H), 4.03 (t, 4H), 3.92-3.86 (m, 1H), 3.72-3.67 (m, 4H), 2.76 (t, 4H), 2.72-2.42 (m, 10H), 2.39-2.23 (m, 4H, 2H), 2.06-2.00 (m, 8H), 1.69-1.21 (m, 40H), 0.87 (t, 6H). MS (ESI): m/z = 829.76 ([M+H]⁺).

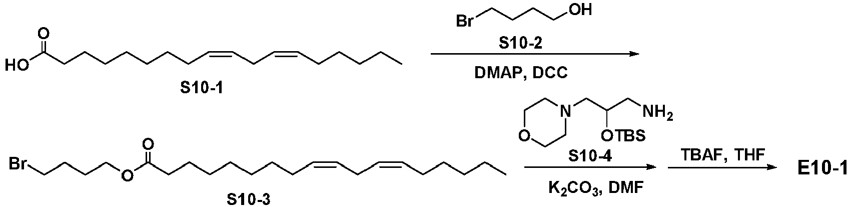

### Example 10.2: preparation of the cationic lipid E10-2

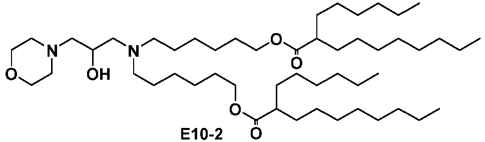

**E10-2** corresponds to the general formula (1); wherein, R₁ and R₂ are both 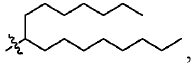 B₁ and B₂ are both hexylene groups, L₁ and L₂ are both ester groups (-OC(=O)-), -L₃(R₃)- is 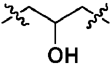 , R₃ is -OH, and R₄ is 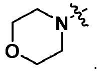 The molecular weight is approximately 837 Da.

Referring to the preparation of **E10-1**, **E10-2** was obtained via replacing **S10-3** with **S3-3** while the rest steps were kept the same. The main data of the ¹H-NMR spectrum are as follows: ¹H NMR (400 MHz, CDCl₃) δ: 4.03 (t, 4H), 3.94-3.87 (m, 1H), 3.71-3.66 (m, 4H), 2.72-2.42 (m, 10H), 2.37-2.24 (m, 2H, 2H), 1.62-1.50 (m, 10H), 1.43-1.32 (m, 10H), 1.26-1.19 (m, 44H), 0.85 (t, 12H). MS (ESI): m/z = 837.80 ([M+H]⁺).

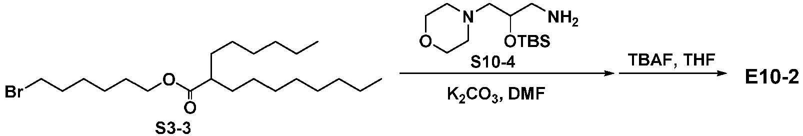

### Example 11: cationic lipid E11-1

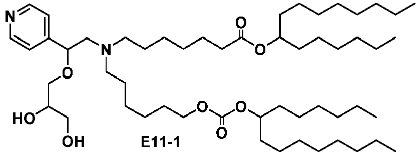

**E11-1** corresponds to the general formula (1); wherein, R₁ and R₂ are both 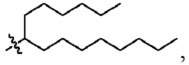 B₁ and B₂ are both hexylene groups, L₁ is an ester group (-C(=O)O-), L₂ is a carbonate group (-OC(=O)O-), -L₃(R₃)- is 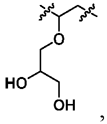 R₃ is 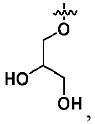 and R₄ is 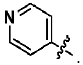 The molecular weight is approximately 905 Da.

The preparation process is as follows:
Step a: Into a sealed reactor under anhydrous and oxygen-free conditions, 200 mL tetrahydrofuran, excessive amounts of diphenylmethyl potassium (14.42 g, 70.0 mmol), the derivative of 2-amino-1-(4-pyridyl)ethanol containing a Boc-protected amino group (**S11-1**, 4.00 g, 16.8 mmol) and compound **S11-2** (6.55 g, 16.8 mmol) were added successively, wherein **S11-2** is the derivative of glycerin having one hydroxyl group substituted by a *p*-toluenesulfonate group (-OTs) and two hydroxyl groups protected by EE. After 12 h of reaction at 30°C, the reactor was opened. The reaction solution was concentrated and washed, and further dissolved with dichloromethane. TFA was added until its concentration reached 0.1 M. After 4 h of reaction, the pH was adjusted to neutral. The reaction solution was concentrated, added with purified water, and extracted with dichloromethane. The extract was concentrated, precipitated, filtered, and dried to obtain the intermediate **S11-3** with a free amino group (3.51 g).
Step b: **S6-2** (4.69 g, 10.8 mmol) was dissolved with 80 mL DMF, added with **S11-3** (3.20 g, 9.0 mmol, prepared according to Example 43) and K₂CO₃ (1.79 g, 13.0 mmol), and reacted overnight at room temperature while stirring. After the reaction was over, the mixture was concentrated under reduced pressure and then added into 80 mL dichloromethane, followed by washing with 10% citric acid (40 mL*2) and brine (40 mL*2) successively. The organic phase was dried over anhydrous sodium sulfate, filtered, concentrated under reduced pressure, and purified by silica gel column chromatography to obtain **S11-4** (5.31 g).
Step c: The above described **S11-4** (4.27 g, 6.0 mmol) was dissolved with dried THF (80 mL), followed by slowly adding NaH (60%, 2.40 g, 60.0 mmol) in an ice bath and reacting for 1 h in an ice bath. After the reaction was over, **S4-3** (3.01 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (80 mL) was added and mixed while stirring. Then, extraction was performed twice using dichloromethane (40 mL*2). The organic phases were collected and combined, and then washed with a saturated solution of sodium chloride (80 mL). The organic phase was concentrated under reduced pressure, and then dissolved with methanol. The 1M hydrochloric acid was added to the solution until pH=3.5. After 4 h of reaction, the reaction solution was concentrated, precipitated, filtered, recrystallized, and dried to obtain the crude product of **E11-1** with a free hydroxyl group. The crude product was purified by column chromatography, concentrated, and dried with an oil pump to obtain the cationic lipid **E11-1** (4.40 g). The main data of the ¹H-NMR spectrum of **E11-1** are as follows: ¹H NMR (400 MHz, CDCl₃) δ: 8.55 (d, 2H), 7.20 (d, 2H), 4.80-4.75 (m, 1H), 4.30-4.25 (m, 1H), 4.21-4.06 (m, 3H), 3.79-3.73 (m, 1H), 3.64-3.56 (m, 2H), 3.41-3.34 (m, 2H), 3.02-2.93 (m, 2H), 2.60-2.25 (m, 6H), 1.72-1.20 (m, 64H), 0.88 (t, 12H). MS (ESI): m/z = 905.78 ([M+H]⁺).

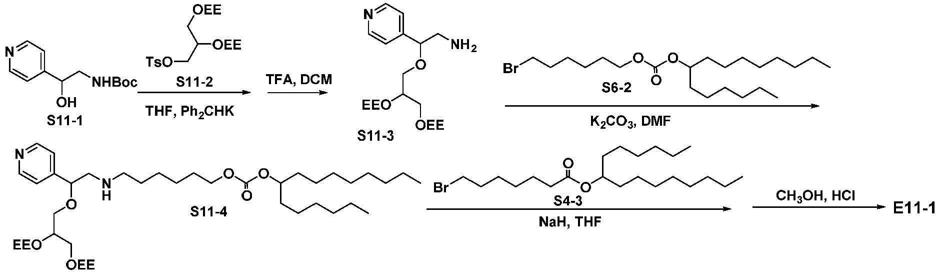

### Example 12: cationic lipid E12-1

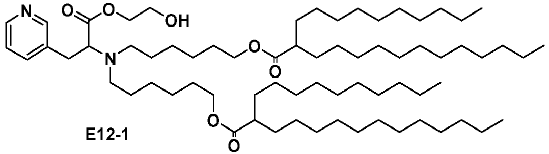

**E12-1** corresponds to the general formula (1); wherein, R₁ and R₂ are both 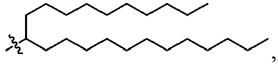 B₁ and B₂ are both hexylene groups, L₁ and L₂ are both ester groups (-OC(=O)-), -L₃(R₃)- is 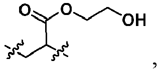 R₃ is -C(=O)O(CH₂)₂OH, and R₄ is 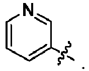 The molecular weight is approximately 1111 Da.

The preparation process is as follows:
Step a: Under argon atmosphere, DCC (3.09 g, 15.0 mmol) was added into a round-bottom flask containing the derivative of 3-(3-pyridyl)*-L*-alanine containing a Boc-protected amino group (**S12-1**, 2.66 g, 10.0 mmol), the derivative of ethylene glycol containing an EE-protected hydroxyl group (**S9-2**, 1.47 g, 11.0 mmol) and DMAP (0.31 g, 2.5 mmol) which were dissolved in dichloromethane (50 mL), and reacted overnight at room temperature for 16 h. After the reaction was over, the precipitates were removed by filtration. The filtrate was concentrated, and the residue was dissolved with dichloromethane. TFA was added until its concentration reached 0.1 M. After 4 h of reaction, the pH was adjusted to neutral. The reaction solution was concentrated, added with purified water, and extracted with dichloromethane. The extract was dried over anhydrous sodium sulfate, filtered, concentrated, and recrystallized to obtain the intermediate **S12-2** with a free amino group (1.79 g).
Step b: **S12-3** (3.50 g, 6.6 mmol, prepared via the condensation reaction of 6-bromo-*n*-hexanol and 2-decyltetradecanoic acid) was dissolved with 50 mL DMF, added with **S12-2** (0.85 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and dissolved with methanol. The 1M hydrochloric acid was added to the solution until pH=3.5. After 4 h of reaction, the reaction solution was concentrated, precipitated, filtered, recrystallized, and dried to obtain the crude product of **E12-1** with a free hydroxyl group. The crude product was purified by silica gel column chromatography to obtain **E12-1** (2.77 g). The main data of the ¹H-NMR spectrum of **E12-1** are as follows: ¹H NMR (400 MHz, CDCl₃) δ: 8.48-7.14 (m, 4H), 4.23-4.19 (m, 2H), 4.10-4.04 (m, 4H), 3.87-3.82 (m, 2H), 3.69-3.63 (m, 1H), 3.10-3.00 (m, 2H), 2.34-2.25 (m, 2H), 1.74-1.20 (m, 100H), 0.88 (t, 12H). MS (ESI): m/z = 1111.99 ([M+H]⁺).

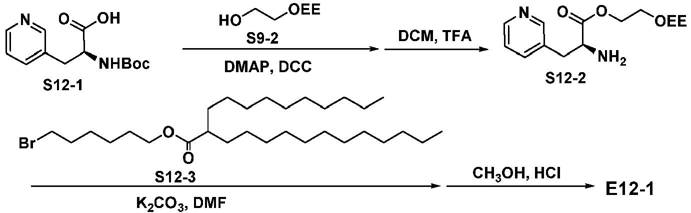

### Example 13: cationic lipid E13-1

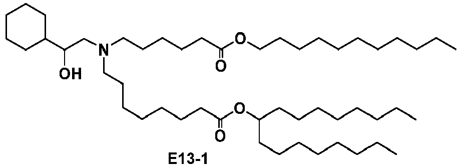

**E13-1** corresponds to the general formula (1); wherein, R₁ is an undecyl group, R₂ is 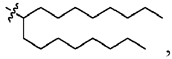 B₁ is a pentylene group, B₂ is a heptylene group, L₁ and L₂ are both ester groups (-C(=O)O-), -L₃(R₃)- is 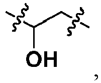 R₃ is -OH, and R₄ is 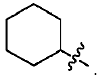 The molecular weight is approximately 792 Da.

The preparation process is as follows:
Step a: **S2-4** (4.42 g, 9.6 mmol) was dissolved with 60 mL DMF, added with **S4-4** (2.06 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S13-1** (4.44 g).
Step b: The above described compound **S13-1** (3.83 g, 6.0 mmol) was dissolved with dried THF (60 mL), followed by slowly adding NaH (60%, 2.40 g, 60.0 mmol) in an ice bath and reacting for 1 h in an ice bath. After the reaction was over, **S2-6** (2.51 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (60 mL) was added and mixed while stirring. Then, extraction was performed twice using dichloromethane (30 mL*2). The organic phases were collected and combined, and then washed with a saturated solution of sodium chloride (60 mL). The organic phase was concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E13-1.** The crude product was purified by column chromatography, concentrated, and dried with an oil pump to obtain the cationic lipid **E13-1** (3.37 g). The main data of the ¹H-NMR spectrum of **E13-1** are as follows: ¹H NMR (400 MHz, CDCl₃) δ: 4.87-4.81 (m, 1H), 4.09 (t, 2H), 3.35-3.27 (m, 1H), 2.63-2.24 (m, 10H), 1.90 (d, 1H), 1.78-0.97 (m, 72H), 0.88 (t, 9H). MS (ESI): m/z = 792.79 ([M+H]⁺).

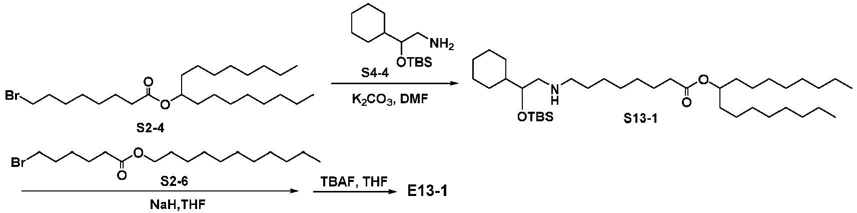

### Example 14: cationic lipid E14-1

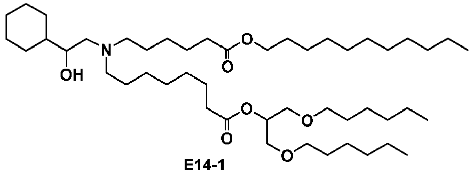

**E14-1** corresponds to the general formula (1); wherein, R₁ is an undecyl group, R₂ is 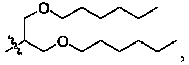 B₁ is a pentylene group, B₂ is a heptylene group, L₁ and L₂ are both ester groups (-C(=O)O-), -L₃(R₃)- is 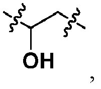 R₃ is -OH, and R₄ is 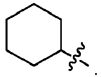 The molecular weight is approximately 796 Da.

The preparation process is as follows:
Step a: **S5-5** (4.45 g, 9.6 mmol) was dissolved with 60 mL DMF, added with **S4-4** (2.06 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S14-1** (4.37 g).
Step b: The above described compound **S14-1** (3.85 g, 6.0 mmol) was dissolved with dried THF (60 mL), followed by slowly adding NaH (60%, 2.40 g, 60.0 mmol) in an ice bath and reacting for 1 h in an ice bath. After the reaction was over, **S2-6** (2.51 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (60 mL) was added and mixed while stirring. Then, extraction was performed twice using dichloromethane (30 mL*2). The organic phases were collected and combined, and then washed with a saturated solution of sodium chloride (60 mL). The organic phase was concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E14-1.** The crude product was purified by column chromatography, concentrated, and dried with an oil pump to obtain the cationic lipid **E14-1** (3.63 g). The main data of the ¹H-NMR spectrum of **E14-1** are as follows: ¹H NMR (400 MHz, CDCl₃) δ: 4.94-4.89 (m, 1H), 4.11 (t, 2H), 3.58-3.29 (m, 9H), 2.65-2.23 (m, 10H), 1.92 (d, 1H), 1.79-0.99 (m, 50H), 0.87 (t, 9H). MS (ESI): m/z = 796.77 ([M+H]⁺).

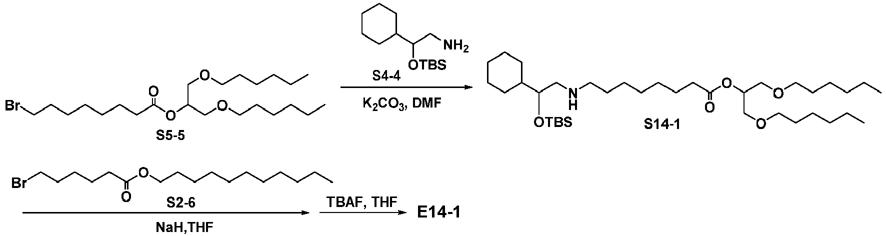

### Example 15: cationic lipid E15-1

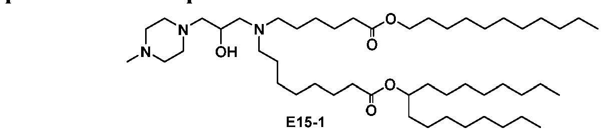

**E15-1** corresponds to the general formula (1); wherein, R₁ is an undecyl group, R₂ is 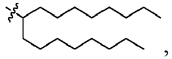 B₁ is a pentylene group, B₂ is a heptylene group, L₁ and L₂ are both ester groups (-C(=O)O-), -L₃(R₃)- is 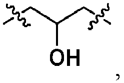 R₃ is -OH, and R₄ is 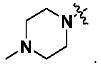 The molecular weight is approximately 822 Da.

The preparation process is as follows:
Step a: **S2-4** (4.42 g, 9.6 mmol) was dissolved with 60 mL DMF, added with **S1-2** (2.30 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S15-1** (4.36 g).
Step b: The above described compound **S15-1** (4.01 g, 6.0 mmol) was dissolved with dried THF (60 mL), followed by slowly adding NaH (60%, 2.40 g, 60.0 mmol) in an ice bath and reacting for 1 h in an ice bath. After the reaction was over, **S2-6** (2.51 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (60 mL) was added and mixed while stirring. Then, extraction was performed twice using dichloromethane (30 mL*2). The organic phases were collected and combined, and then washed with a saturated solution of sodium chloride (60 mL). The organic phase was concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E15-1.** The crude product was purified by column chromatography, concentrated, and dried with an oil pump to obtain the cationic lipid **E15-1** (3.92 g). The main data of the ¹H-NMR spectrum of **E15-1** are as follows: ¹H NMR (400 MHz, CDCl₃) δ: 4.88-4.80 (m, 1H), 4.08 (t, 2H), 3.84-3.73 (m, 1H), 2.77-2.19 (m, 23H), 1.72-1.18 (m, 62H), 0.87 (t, 9H). MS (ESI): m/z = 822.81 ([M+H]⁺).

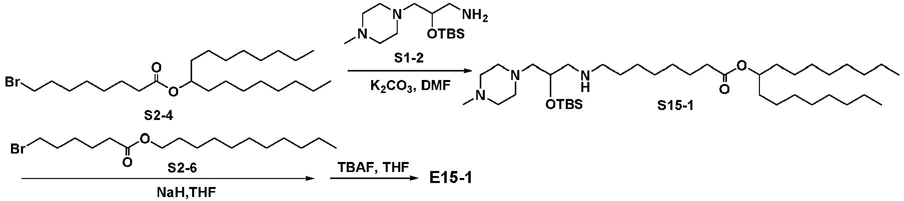

### Example 16: cationic lipid E16-1

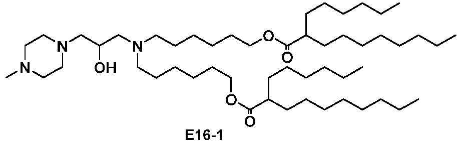

**E16-1** corresponds to the general formula (1); wherein, R₁ and R₂ are both 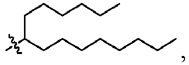 B₁ and B₂ are both hexylene groups, L₁ and L₂ are both ester groups (-OC(=O)-), -L₃(R₃)- is 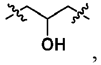 R₃ is -OH, and R₄ is 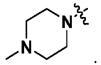 The molecular weight is approximately 850 Da.

The preparation process is as follows:
**S3-3** (2.76 g, 6.6 mmol) was dissolved with 50 mL DMF, added with **S1-2** (0.86 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain **E16-1** (2.07 g). The main data of the ¹H-NMR spectrum of **E16-1** are as follows: ¹H NMR (400 MHz, CDCl₃) δ: 4.04 (t, 4H), 3.85-3.77 (m, 1H), 2.80-2.20 (m, 21H), 1.66-1.39 (m, 16H), 1.35-1.20 (m, 48H), 0.86 (t, 12H). MS (ESI): m/z = 850.83 ([M+H]⁺).

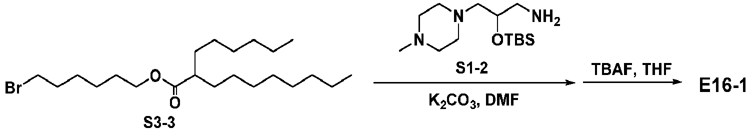

### Example 17: cationic lipid E17-1

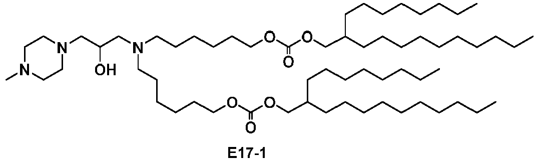

**E17-1** corresponds to the general formula (1); wherein, R₁ and R₂ are both 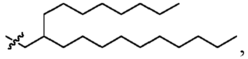 B₁ and B₂ are both hexylene groups, L₁ and L₂ are both carbonate groups (-OC(=O)O-), -L₃(R₃)- is 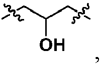 R₃ is -OH, and R₄ is 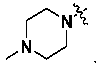 The molecular weight is approximately 1022 Da.

The preparation process is as follows:
Step a: Under nitrogen protection, 6-bromohexyl-4-nitrophenyl carbonate (**S6-1**, 10.35 g, 30.0 mmol) was dissolved with DCM (400 mL), added dropwise by 2-octyldodecanol (**S17-1**, 35.76 g, 120.0 mmol) at room temperature while stirring, and then slowly added with pyridine (3.02 mL, 37.5 mmol) in over 10 min, and added with DMAP in one portion (0.73 g, 6.0 mmol). The reaction was continued for 16 h at room temperature while stirring. After the reaction was over, extraction was performed twice using DCM and water, respectively. The organic phases were combined and washed with brine, and then dried over anhydrous sodium sulfate, filtered, concentrated, and isolated and purified by silica gel column. The eluents were collected and concentrated to obtain **S17-2** (3.63 g).
Step b: **S17-2** (3.33 g, 6.6 mmol) was dissolved with 50 mL DMF, added with **S1-2** (0.86 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain **E17-1** (2.42 g). The main data of the ¹H-NMR spectrum of **E17-1** are as follows: ¹H NMR (400 MHz, CDCl₃) δ: 4.13-3.99 (m, 8H), 3.33-2.94 (m, 12H), 2.92-2.57 (m, 6H), 2.55-2.41 (m, 2H), 1.76-1.38 (m, 16H), 1.34-1.22 (m, 66H), 0.88 (t, 12H). MS (ESI): m/z = 1022.95 ([M+H]⁺).

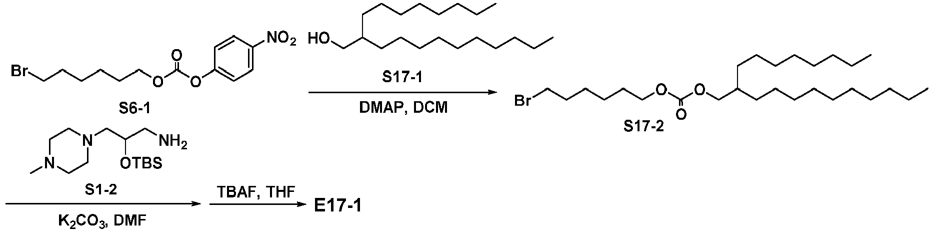

### Example 18: cationic lipid E18-1

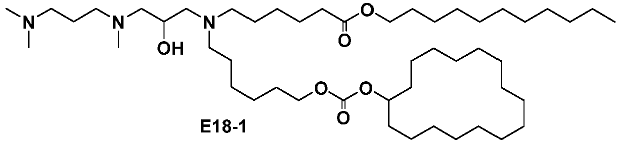

**E18-1** corresponds to the general formula (1); wherein, R₁ is an undecyl group, R₂ is a cyclohexadecyl group, B₁ is a pentylene group, B₂ is a hexylene group, L₁ is an ester group (-C(=O)O-), L₂ is a carbonate group (-OC(=O)O-), -L₃(R₃)- is 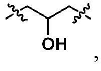 R₃ is -OH, and R₄ is 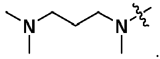 The molecular weight is approximately 824 Da.

The preparation process is as follows:
Step a: Under nitrogen protection, **S6-1** (10.35 g, 30.0 mmol) was dissolved with DCM (400 mL), added dropwise with cyclohexadecyl-1-ol (**S18-1**, 28.80 g, 120.0 mmol) at room temperature while stirring, and then slowly added with pyridine (3.02 mL, 37.5 mmol) in over 10 min, and added with DMAP in one portion (0.73 g, 6.0 mmol). The reaction was continued for 16 h at room temperature while stirring. After the reaction was over, extraction was performed twice using DCM and water, respectively. The organic phases were combined and washed with brine, and then dried over anhydrous sodium sulfate, filtered, concentrated, and isolated and purified by silica gel column. The eluents were collected and concentrated to obtain **S18-2** (3.75 g).
Step b: **S18-2** (3.21 g, 7.2 mmol) was dissolved with 50 mL DMF, added with **S2-3** containing a TBS-protected hydroxyl group (1.82 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S18-3** (3.34 g).
Step c: The above described compound **S18-3** (2.68 g, 4.0 mmol) was dissolved with dried THF (40 mL), followed by slowly adding NaH (60%, 1.59 g, 40.0 mmol) in an ice bath and reacting for 1 h in an ice bath. After the reaction was over, **S2-6** (1.67 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (40 mL) was added and mixed while stirring. Then, extraction was performed twice using dichloromethane (20 mL*2). The organic phases were collected and combined, and then washed with a saturated solution of sodium chloride (40 mL). The organic phase was concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E18-1.** The crude product was purified by column chromatography, concentrated, and dried with an oil pump to obtain the cationic lipid **E18-1** (2.49 g). The main data of the ¹H-NMR spectrum of **E18-1** are as follows: ¹H NMR (400 MHz, CDCl₃) δ: 4.20-4.07 (m, 2H), 4.05 (t, 2H), 3.82-3.77 (m, 1H), 2.65-2.31 (m, 14H), 2.26-2.20 (m, 9H), 1.74-1.25 (m, 64H), 0.89 (t, 3H). MS (ESI): m/z = 824.78 ([M+H]⁺).

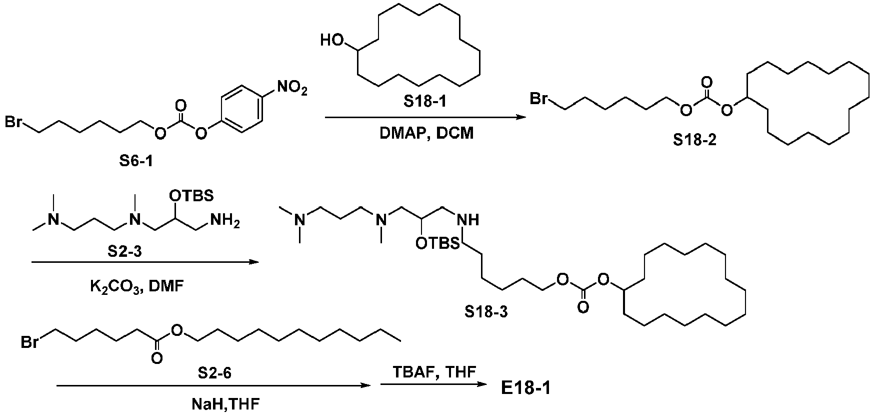

### Example 19: cationic lipid E19-1

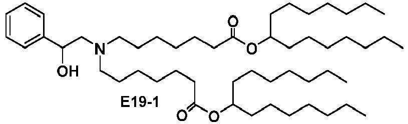

**E19-1** corresponds to the general formula (1); wherein, R₁ and R₂ are both 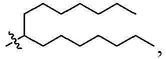 B₁ and B₂ are both hexylene groups, L₁ and L₂ are both ester groups (-C(=O)O-), -L₃(R₃)- is 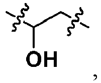 R₃ is -OH, and R₄ is 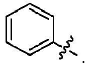 The molecular weight is approximately 814 Da.

The preparation process is as follows:
Step a: Under argon atmosphere, DCC (3.09 g, 15.0 mmol) was added into a round-bottom flask containing 7-bromoheptanoic acid (**S4-1**, 2.08 g, 10.0 mmol), 8-pentadecanol (**S19-1**, 2.51 g, 11.0 mmol) and DMAP (0.31 g, 2.5 mmol) which were dissolved in dichloromethane (50 mL), and reacted overnight at room temperature for 16 h. After the reaction was over, the precipitates were removed by filtration. The filtrate was concentrated, and the residue was purified by silica gel column chromatography to obtain **S19-2** (3.68 g).
Step b: **S19-2** (2.76 g, 6.6 mmol) was dissolved with 50 mL DMF, added with the derivative of 2-amino-1-phenylethanol (**S19-3**, 0.75 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain **E19-1** (1.95 g). The main data of the ¹H-NMR spectrum of **E19-1** are as follows: ¹H NMR (400 MHz, CDCl₃) δ: 7.40-7.22 (m, 5H), 4.86-4.77 (m, 2H), 4.72-4.65 (m, 1H), 2.53-2.24 (m, 10H), 1.69-1.21 (m, 64H), 0.88 (t, 12H). MS (ESI) : m/z = 814.77 ([M+H]⁺).

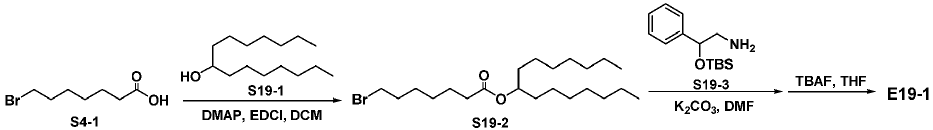

### Example 20: cationic lipid E20-1

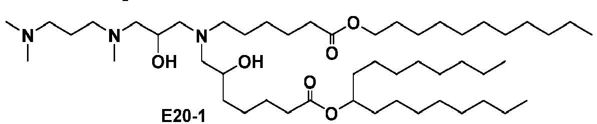

**E20-1** corresponds to the general formula (1); wherein, R₁ is an undecyl group, R₂ is 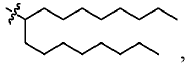 B₁ is a pentylene group, B₂ is a hexylene group substituted by -OH, L₁ and L₂ are both ester groups (-C(=O)O-), -L₃(R₃)- is 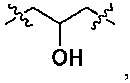 R₃ is -OH, and R₄ is 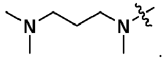 The molecular weight is approximately 840 Da.

The preparation process is as follows:
Step a: Under nitrogen protection, 6-heptenoic acid (**S20-2**, 2.56 g, 20.0 mmol) was dissolved with DCM (100 mL), added with *N*,*N*-Diisopropylethylamine (DIEA, 7.74 g, 60.0 mmol), **S20-1** (7.68 g, 30.0 mmol), EDCI (7.68 g, 40.0 mmol) and DMAP (0.73 g, 6.0 mmol). The mixture was stirred at 50°C, and the reaction was continued for 10 h. After the reaction was over, extraction was performed twice using DCM and water, respectively. The organic phases were combined and washed with brine, and then dried over anhydrous sodium sulfate, filtered, concentrated, and isolated and purified by silica gel column. The eluents were collected and concentrated to obtain **S20-3** (6.15 g).
Step b: The above described compound **S20-3** (5.12 g, 14.0 mmol) was dissolved with 70 mL DCM, added with *m*-chloroperoxybenzoic acid (*m*-CPBA, 4.84 g, 28.0 mmol), and reacted at room temperature for 10 h while stirring. After the reaction was over, the mixture was poured into 70 mL saturated solution of sodium bicarbonate and mixed sufficiently. The aqueous phase was isolated and extracted with DCM (35 mL*2). The organic phases were combined and washed with saturated brine, and then dried over anhydrous sodium sulfate, filtered, concentrated, and isolated and isolated and purified by silica gel column. The eluents were collected and concentrated to obtain **S20-4** (4.44 g).
Step c: The above described **S20-4** (3.67 g, 9.6 mmol) was dissolved with 50 mL ethanol, added with **S2-3** containing a TBS-protected hydroxyl group (2.42 g, 8.0 mmol), and reacted at 50°C for 4 h while stirring. After the reaction was over, the mixture was diluted with 50 mL dichloromethane and then washed with 100 mL saturated solution of sodium chloride. The organic phase was isolated, dried over anhydrous sodium sulfate, filtered, concentrated, and purified by silica gel column chromatography to obtain **S20-5** (3.62 g).
Step d: The above descried compound **S20-5** (3.43 g, 5.0 mmol) and imidazole (0.54 g, 8.0 mmol) were dissolved with 50 mL dichloromethane, and added with tert-butyldimethylchlorosilane (1.05 g, 7.0 mmol) in an ice bath. After 2 h of reaction, the mixture was filtered and washed with 50 mL dichloromethane. The filtrates were combined, dried over anhydrous sodium sulfate, and concentrated to obtain the intermediate **S20-6** containing two TBS-protected hydroxyl groups (3.92 g).
Step e: **S20-6** (3.20 g, 4.0 mmol) was dissolved with dried THF (40 mL), followed by slowly adding NaH (60%, 1.59 g, 40.0 mmol) in an ice bath and reacting for 1 h in an ice bath. After the reaction was over, **S2-6** (1.67 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (40 mL) was added and mixed while stirring. Then, extraction was performed twice using dichloromethane (20 mL*2). The organic phases were collected and combined, and then washed with a saturated solution of sodium chloride (40 mL). The organic phase was concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E20-1.** The crude product was purified by column chromatography, concentrated, and dried with an oil pump to obtain the cationic lipid **E20-1** (2.72 g). The main data of the ¹H-NMR spectrum of **E20-1** are as follows: ¹H NMR (400 MHz, CDCl₃) δ: 4.85-4.80 (m, 1H), 4.07 (t, 2H), 3.83-3.78 (m, 1H), 3.63-3.59 (m, 1H), 2.67-2.21 (m, 25H), 1.73-1.23 (m, 60H), 0.87 (t, 9H). MS (ESI): m/z = 840.82 ([M+H]⁺).

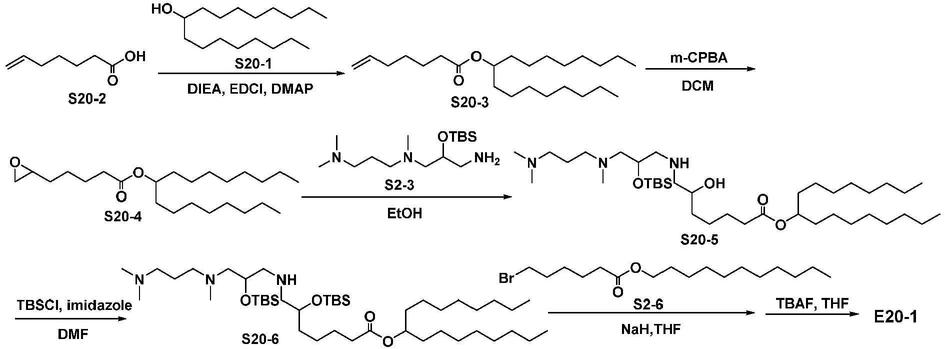

### Example 21: cationic lipid E21-1

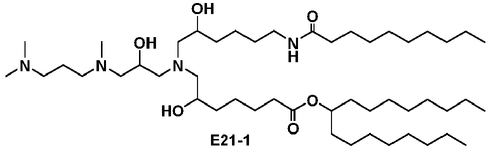

**E21-1** corresponds to the general formula (1); wherein, R₁ is an undecyl group, R₂ is 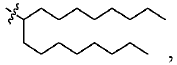 B₁ and B₂ are both hexylene groups substituted by -OH, L₁ is an amide group (-NHC(=O)-), L₂ is an ester group (-C(=O)O-), -L₃(R₃)- is 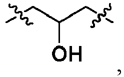 R₃ is -OH, and R₄ is 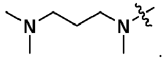 The molecular weight is approximately 841 Da.

The preparation process is as follows:
Step a: Under nitrogen protection, 1-amino-5-hexene (**S21-1**, 1.98 g, 20.0 mmol) was dissolved with 50 mL dichloromethane. The solution was placed in an ice bath, and then triethylamine (2.77 mL, 20.0 mmol) was added. Decanoyl chloride (**S21-2**, 3.80 g, 20.0 mmol) was added in 1 h while stirring, after which the ice bath was removed and the temperature of the reaction solution was elevated to room temperature. 1 h later, 50 mL water was added and mixed by stirring. The organic phase was isolated, and the aqueous phase was extracted with DCM (25 mL*2). The organic phases were collected and combined, washed with 100 mL saturated solution of sodium chloride, and dried over anhydrous sodium sulfate. The solvent and the remaining triethylamine were removed under reduced pressure. Purification was performed with silica gel column chromatography to obtain **S21-3** (4.45 g).
Step b: The above described compound **S21-3** (3.54 g, 14.0 mmol) was dissolved with 60 mL DCM, added with *m*-chloroperoxybenzoic acid (*m*-CPBA, 4.84 g, 28.0 mmol), and reacted at room temperature for 10 h while stirring. After the reaction was over, the mixture was poured into 60 mL saturated solution of sodium bicarbonate and mixed sufficiently. The aqueous phase was isolated and extracted with DCM (20 mL*3). The organic phases were combined and washed with saturated brine, and then dried over anhydrous sodium sulfate, filtered, concentrated, and purified by silica gel column chromatography to obtain **S21-4** (3.13 g).
Step c: The above described compound **S21-4** (2.58 g, 9.6 mmol) was dissolved with 50 mL ethanol, added with **S2-3** containing a TBS-protected hydroxyl group (2.42 g, 8.0 mmol), and reacted at 50°C for 4 h while stirring. After the reaction was over, the mixture was diluted with 50 mL dichloromethane and then washed with 100 mL saturated solution of sodium chloride. The organic phase was isolated, dried over anhydrous sodium sulfate, filtered, concentrated, and purified by silica gel column chromatography to obtain **S21-5** (3.12 g).
Step d: The above descried compound **S21-5** (2.87 g, 5.0 mmol) and imidazole (0.54 g, 8.0 mmol) were dissolved with 50 mL dichloromethane, and added with tert-butyldimethylchlorosilane (1.05 g, 7.0 mmol) in an ice bath. After 2 h of reaction, the mixture was filtered and washed with 50 mL dichloromethane. The filtrates were combined, dried over anhydrous sodium sulfate, and concentrated to obtain the intermediate **S21-6** containing two TBS-protected hydroxyl groups (3.37 g).
Step e: The above described compound **S21-6** (2.75 g, 4.0 mmol) was dissolved with 50 mL ethanol, added with **S20-4** (1.67 g, 4.8 mmol), and reacted at 50°C for 8 h while stirring. After the reaction was over, the mixture was diluted with 50 mL dichloromethane and then washed with 100 mL saturated solution of sodium chloride. The organic phase was isolated, concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was further purified by column chromatography, concentrated, and dried with an oil pump to obtain the cationic lipid **E21-1** (2.39 g). The main data of the ¹H-NMR spectrum of **E21-1** are as follows: ¹H NMR (400 MHz, CDCl₃) δ: 4.86-4.81 (m, 1H), 3.84-3.78 (m, 1H), 3.66-3.60 (m, 2H), 3.20-3.15 (m, 2H), 2.67-2.19 (m, 23H), 2.13-2.08 (m, 2H), 1.71-1.22 (m, 56H), 0.86 (t, 9H). MS (ESI): m/z = 841.81 ([M+H]⁺).

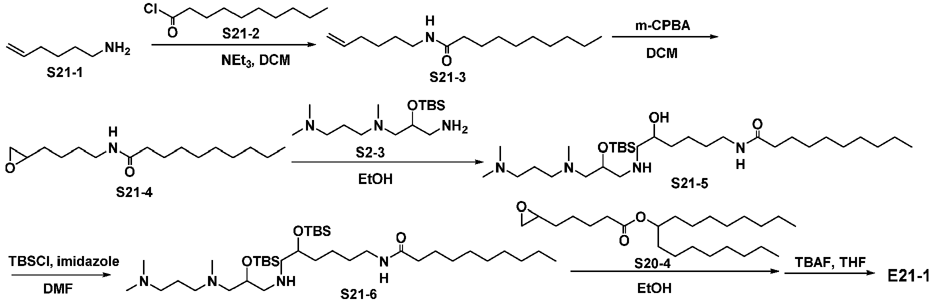

### Example 22: cationic lipid E22-1

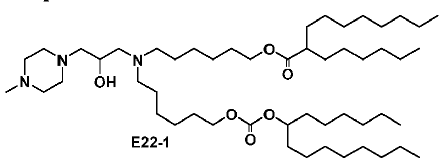

**E22-1** corresponds to the general formula (1); wherein, R₁ and R₂ are both 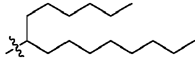 , B₁ and B₂ are both hexylene groups, L₁ is an ester group (-OC(=O)-), L₂ is a carbonate group (-OC(=O)O-), -L₃(R₃)- is 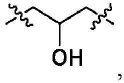 R₃ is -OH, and R₄ is 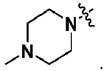 The molecular weight is approximately 866 Da.

The preparation process is as follows:
Step a: **S6-2** (3.12 g, 7.2 mmol) was dissolved with 50 mL DMF, added with **S1-2** (1.72 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S22-1** (3.02 g).
Step b: The above described compound **S22-1** (2.57 g, 4.0 mmol) was dissolved with dried THF (40 mL), followed by slowly adding NaH (60%, 1.60 g, 40.0 mmol) in an ice bath and reacting for 1 h in an ice bath. After the reaction was over, **S3-3** (2.01 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (40 mL) was added and mixed while stirring. Then, extraction was performed twice using dichloromethane (20 mL*2). The organic phases were collected and combined, and then washed with a saturated solution of sodium chloride (40 mL). The organic phase was concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E22-1.** The crude product was purified by column chromatography, concentrated, and dried with an oil pump to obtain the cationic lipid **E22-1** (2.60 g). The main data of the ¹H-NMR spectrum of **E22-1** are as follows: ¹H NMR (400 MHz, CDCl₃) δ: 4.18-4.06 (m, 5H), 3.87-3.78 (m, 1H), 2.76-2.20 (m, 20H), 1.68-1.21 (m, 64H), 0.88 (t, 12H). MS (ESI): m/z = 866.84 ([M+H]⁺).

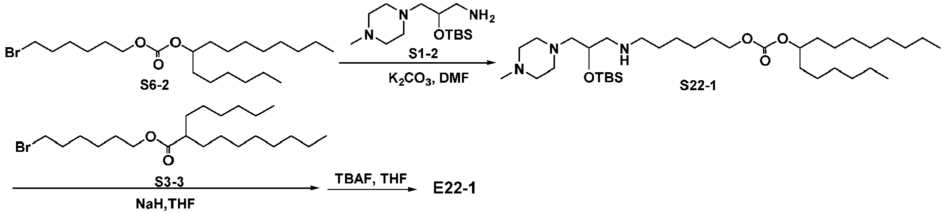

### Example 23: cationic lipid E23-1

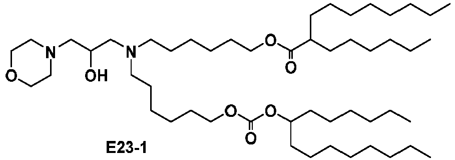

**E23-1** corresponds to the general formula (1); wherein, R₁ and R₂ are both 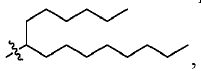 B₁ and B₂ are both hexylene groups, L₁ is an ester group (-OC(=O)-), L₂ is a carbonate group -OC=OO-, -L₃(R₃)- is 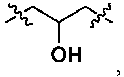 R₃ is -OH, and R₄ is 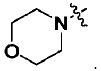 The molecular weight is approximately 853 Da.

The preparation process is as follows:
Step a: **S6-2** (3.12 g, 7.2 mmol) was dissolved with 50 mL DMF, added with the derivative of 1-amino-3-(4-morpholinyl)-2-propanol containing a TBS-protected hydroxyl group (**S10-4**, 1.64 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S23-1** (2.94 g).
Step b: The above described compound **S23-1** (2.52 g, 4.0 mmol) was dissolved with dried THF (40 mL), followed by slowly adding NaH (60%, 1.60 g, 40.0 mmol) in an ice bath and reacting for 1 h in an ice bath. After the reaction was over, **S3-3** (2.01 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (40 mL) was added and mixed while stirring. Then, extraction was performed twice using dichloromethane (20 mL*2). The organic phases were collected and combined, and then washed with a saturated solution of sodium chloride (40 mL). The organic phase was concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E23-1.** The crude product was purified by column chromatography, concentrated, and dried with an oil pump to obtain the cationic lipid **E23-1** (2.63 g). The main data of the ¹H-NMR spectrum of **E23-1** are as follows: ¹H NMR (400 MHz, CDCl₃) δ: 4.21-4.02 (m, 5H), 3.92-3.84 (m, 1H), 3.72-3.64 (m, 4H), 2.71-2.39 (m, 10H), 2.37-2.27 (m, 3H), 1.67-1.21 (m, 64H), 0.86 (t, 12H). MS (ESI): m/z = 853.81 ([M+H]⁺).

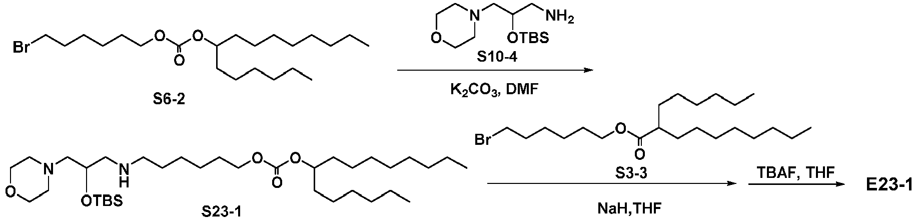

### Example 24: cationic lipid E24-1

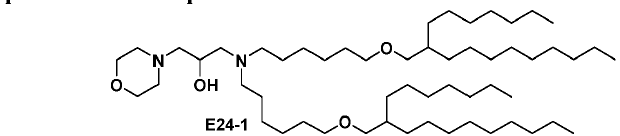

**E24-1** corresponds to the general formula (1); wherein, R₁ and R₂ are both 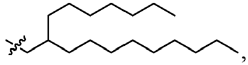 B₁ and B₂ are both hexylene groups, L₁ and L₂ are both -O-, -L₃(R₃)-is 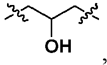 R₃ is -OH, and R₄ is 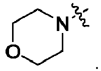 The molecular weight is approximately 865 Da.

The preparation process is as follows:
Step a: 2-Heptyl undecanol (**S24-1**, 4.05 g, 15.0 mmol) was dissolved with dried tetrahydrofuran (60 mL). Under nitrogen protection, NaH (60%, 0.60 g, 15.0 mmol) was added slowly in an ice bath while stirring. The reaction was continued for 1 h in the ice bath and then 1,6-dibromohexane (**S24-2**, 4.36 g, 18.0 mmol) was added to react overnight at room temperature, after which 3 mL water was added and mixed for 10 min. The reaction solution was poured into 60 mL water, and then extracted twice with dichloromethane (30 mL*2). The organic phases were combined, washed with a saturated solution of NaCl, dried over anhydrous sodium sulfate, filtered, concentrated, and purified by column chromatography to obtain the small molecule intermediate **S24-3** (3.50 g).
Step b: **S24-3** (2.85 g, 6.6 mmol) was dissolved with 50 mL DMF, added with **S10-4** which contains a TBS-protected hydroxyl group (0.82 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E24-1.** The crude product was purified by silica gel column chromatography to obtain the cationic lipid **E24-1** (2.26 g). The main data of the ¹H-NMR spectrum **of E24-1** are as follows: ¹H NMR (400 MHz, CDCl₃) δ: 3.80-3.41 (m, 13H), 2.72-2.30 (m, 12H), 1.82-1.22 (m, 74H), 0.86 (t, 12H). MS (ESI): m/z = 865.91 ([M+H]⁺).

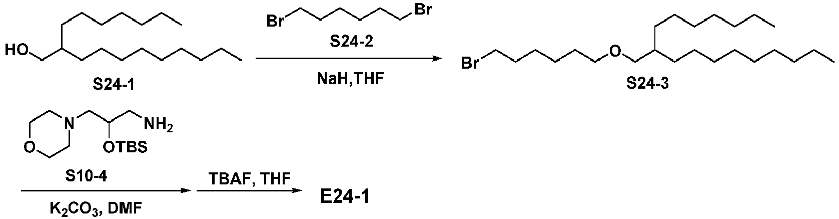

### Example 25: cationic lipid E25-1

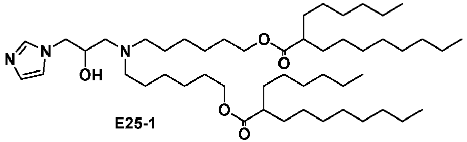

**E25-1** corresponds to the general formula (1); wherein R₁ and R₂ are both 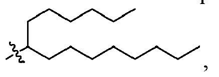 B₁ and B₂ are both hexylene groups, L₁ and L₂ are both ester groups (-OC(=O)-), -L₃(R₃)- is 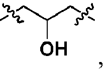 R₃ is -OH, and R₄ is 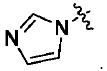 The molecular weight is approximately 818 Da.

The preparation process is as follows:
**S3-3** (2.76 g, 6.6 mmol) was dissolved with 50 mL DMF, added with 1-amino-3-(1-imidazolyl)-2-propanol (**S25-1**, 0.77 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain **E25-1** (1.99 g). The main data of the ¹H-NMR spectrum of **E25-1** are as follows: ¹H NMR (400 MHz, CDCl₃) δ: 7.61-6.88 (m, 3H), 4.20-4.14 (m, 2H), 4.09-4.03 (m, 5H), 2.68-2.63 (m, 2H), 2.49-2.42 (m, 4H), 2.36-2.25 (m, 2H), 1.70-1.21 (m, 64H), 0.87 (t, 12H). MS (ESI): m/z = 818.74 ([M+H]⁺).

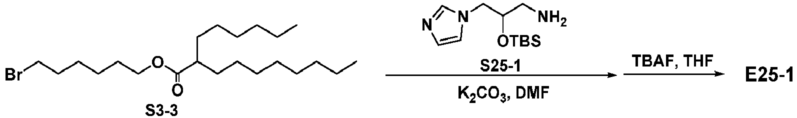

### Example 26: cationic lipid E26-1

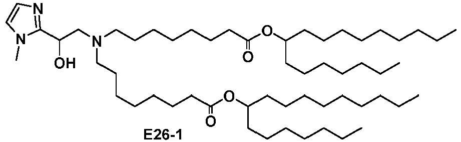

**E26-1** corresponds to the general formula (1); wherein, R₁ and R₂ are both 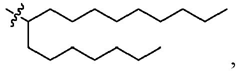 B₁ and B₂ are both heptylene groups, L₁ and L₂ are both ester groups (-OC(=O)-), -L₃(R₃)- is 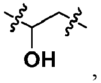 R₃ is -OH, and R₄ is 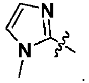 The molecular weight is approximately 902 Da.

The preparation process is as follows:
Step a: Under argon atmosphere, DCC (3.09 g, 15.0 mmol) was added into a round-bottom flask containing 8-bromooctanoic acid (**S5-4**, 2.22 g, 10.0 mmol), 8-heptadecanol (**S26-1**, 2.82 g, 11.0 mmol) and DMAP (0.31 g, 2.5 mmol) which were dissolved in dichloromethane (50 mL), and reacted overnight at room temperature for 16 h. After the reaction was over, the precipitates were removed by filtration. The filtrate was concentrated, and the residue was purified by silica gel column chromatography to obtain 1-heptyldecyl 8-bromooctanoate (**S26-2**, 3.80 g).
Step b: **S26-2** (3.04 g, 6.6 mmol) was dissolved with 50 mL DMF, added with 2-amino-1-[2-(1-methylimidazolyl)]ethanol (**S26-3**, 0.77 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain **E26-1** (2.19 g). The main data of the ¹H-NMR spectrum of **E26-1** are as follows: ¹H NMR (400 MHz, CDCl₃) δ: 6.94-6.91 (m, 1H), 6.88-6.84 (m, 1H), 4.90-4.84 (m, 2H), 4.82-4.78 (m, 1H), 3.77 (s, 3H), 2.90-2.75 (m, 2H), 2.53-2.22 (m, 8H), 1.75-1.22 (m, 76H), 0.87 (t, 12H). MS (ESI): m/z = 902.82 ([M+H]⁺).

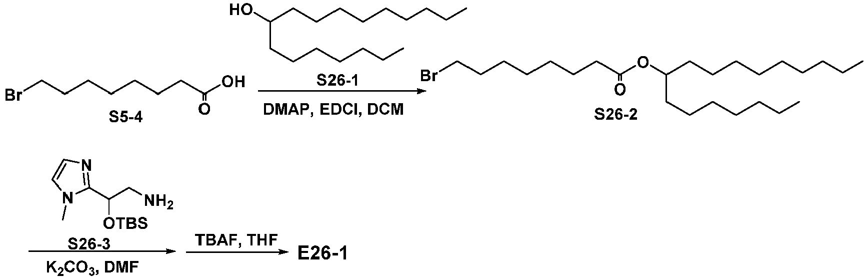

### Example 27: cationic lipid E27-1

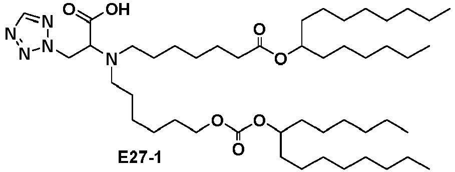

**E27-1** corresponds to the general formula (1); wherein, R₁ and R₂ are both 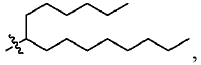 B₁ and B₂ are both hexylene groups, L₁ is an ester group (-C(=O)O-), L₂ is a carbonate group (-OC(=O)O-), -L₃(R₃)- is 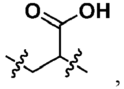 R₃ is -C(=O)OH, and R₄ is 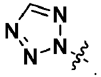 The molecular weight is approximately 850 Da.

The preparation process is as follows:
Step a: **S6-2** (4.69 g, 10.8 mmol) was dissolved with 60 mL DMF, added with 3-(2-tetrazolyl)-*L*-alanine (**S27-1**, 1.41 g, 9.0 mmol) and K₂CO₃ (1.79 g, 13.0 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S27-2** (3.59 g).
Step b: The above described compound **S27-2** (3.07 g, 6.0 mmol) was dissolved with dried THF (50 mL), followed by slowly adding NaH (60%, 2.40 g, 60.0 mmol) in an ice bath and reacting for 1 h in an ice bath. After the reaction was over, **S4-3** (3.01 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was dried over anhydrous sodium sulfate, filtered, and concentrated to obtain the crude product of **E27-1.** The crude product was purified by column chromatography, concentrated, and dried with an oil pump to obtain the cationic lipid **E27-1** (4.13 g). The main data of the ¹H-NMR spectrum of **E27-1** are as follows: ¹H NMR (400 MHz, CDCl₃) δ: 8.40 (s, 1H), 4.98-4.75 (m, 3H), 4.21-4.09 (m, 3H), 3.50-3.45 (m, 1H), 2.51-2.42 (m, 4H), 2.38-2.32 (t, 2H), 1.72-1.24 (m, 64H), 0.89 (t, 12H). MS (ESI): m/z = 850.84 ([M+H]⁺).

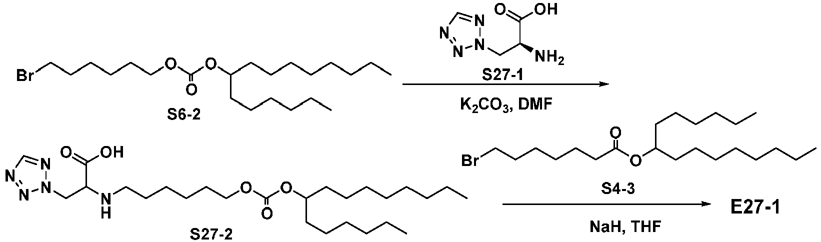

### Example 28: cationic lipid E28-1

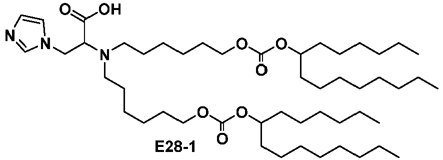

**E28-1** corresponds to the general formula (1); wherein, R₁ and R₂ are both 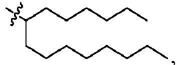 B₁ and B₂ are both hexylene groups, L₁ and L₂ are both carbonate groups (-OC(=O)O-), -L₃(R₃)- is 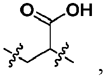 R₃ is -C(=O)OH, and R₄ is 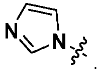 The molecular weight is approximately 864 Da.

The preparation process is as follows:
**S6-2** (2.86 g, 6.6 mmol) was dissolved with 50 mL DMF, added with 3-imidazolyl-*L*-alanine (**S28-1**, 0.47 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **E28-1** (2.10 g). The main data of the ¹H-NMR spectrum of **E28-1** are as follows: ¹H NMR (400 MHz, CDCl₃) δ: 7.63-6.89 (m, 3H), 4.61-4.40 (m, 2H), 4.18-4.06 (m, 6H), 3.49-3.44 (m, 1H), 2.56-2.47 (m, 4H), 1.72-1.22 (m, 64H), 0.88 (t, 12H). MS (ESI): m/z = 864.80 ([M+H]⁺).

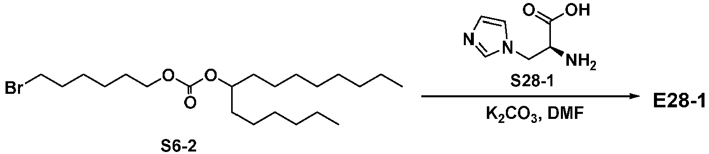

### Example 29: cationic lipid E29-1

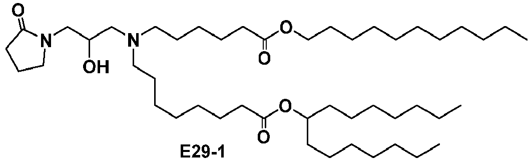

**E29-1** corresponds to the general formula (1); wherein, R₁ is an undecyl group, R₂ is 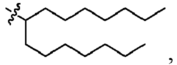 B₁ is a pentylene group, B₂ is a heptylene group, L₁ and L₂ are both ester groups (-C(=O)O-), -L₃(R₃)- is 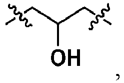 R₃ is -OH, and R₄ is 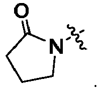 The molecular weight is approximately 779 Da.

The preparation process is as follows:
Step a: Under argon atmosphere, DCC (3.71 g, 18.0 mmol) was added into a round-bottom flask containing **S5-4** (2.66 g, 12.0 mmol), **S19-1** (3.01 g, 13.2 mmol) and DMAP (0.37 g, 3.0 mmol) which were dissolved in dichloromethane (50 mL), and reacted overnight at room temperature for 16 h. After the reaction was over, the precipitates were removed by filtration. The filtrate was concentrated, and the residue was purified by silica gel column chromatography to obtain **S29-1** (4.28 g).
Step b: **S29-1** (4.15 g, 9.6 mmol) was dissolved with 60 mL DMF, added with the derivative of 1-(3-amino-2-hydroxypropyl)pyrrolidin-2-one containing a TBS-protected hydroxyl group (**S29-2**, 2.18 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S29-3** (4.15 g).
Step c: The above described compound **S29-3** (3.75 g, 6.0 mmol) was dissolved with dried THF (50 mL), followed by slowly adding NaH (60%, 2.40 g, 60.0 mmol) in an ice bath and reacting for 1 h in an ice bath. After the reaction was over, **S2-6** (2.51 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E29-1.** The crude product was purified by column chromatography, concentrated, and dried with an oil pump to obtain the cationic lipid **E29-1** (3.65 g). The main data of the ¹H-NMR spectrum of **E29-1** are as follows: ¹H NMR (400 MHz, CDCl₃) δ: 4.90-4.85 (m, 1H), 4.06 (t, 2H), 3.96-3.87 (m, 1H), 3.67-3.52 (m, 4H), 2.64-2.58 (m, 2H), 2.52-2.26 (m, 10H), 2.09-1.94 (m, 2H), 1.74-1.23 (m, 58H), 0.89 (t, 9H). MS (ESI): m/z = 779.73 ([M+H]⁺).

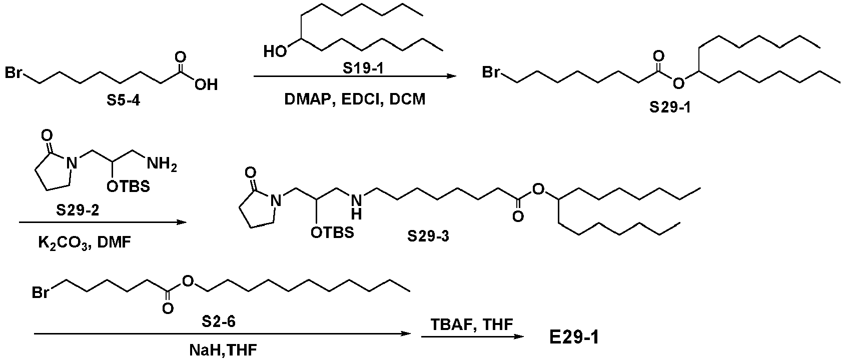

### Example 30: cationic lipid E30-1

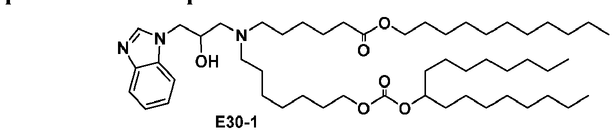

**E30-1** corresponds to the general formula (1); wherein, R₁ is an undecyl group, R₂ is 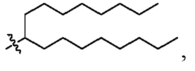 B₁ is a pentylene group, B₂ is a heptylene group, L₁ is an ester group (-C(=O)O-), L₂ is a carbonate group (-OC(=O)O-), -L₃(R₃)- is 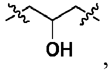 R₃ is -OH, and R₄ is 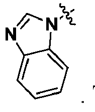 The molecular weight is approximately 856 Da.

The preparation process is as follows:
Step a: Under nitrogen protection, 7-bromoheptyl-4-nitrophenyl carbonate (**S30-1**, 10.77 g, 30.0 mmol, prepared via the reaction of *p*-nitrophenyl chloroformate and 7-bromo-*n*-heptanol) was dissolved with DCM (400 mL), added dropwise by **S20-1** (30.72 g, 120.0 mmol) at room temperature while stirring, and then slowly added with pyridine (3.02 mL, 37.5 mmol) in over 10 min, and added with DMAP in one portion (0.73 g, 6.0 mmol). The reaction was continued for 16 h at room temperature while stirring. After the reaction was over, extraction was performed twice using DCM and water, respectively. The organic phases were combined and washed with brine, and then dried over anhydrous sodium sulfate, filtered, concentrated, and isolated and purified by silica gel column. The eluents were collected and concentrated to obtain **S30-2** (3.71 g).
Step b: **S30-2** (3.43 g, 7.2 mmol) was dissolved with 50 mL DMF, added with the derivative of 1-amino-3-(1-benzoimidazolyl)-2-propanol containing a TBS-protected hydroxyl group (**S30-3**, 1.83 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S30-4** (3.30 g).
Step c: The above described compound **S30-4** (2.81 g, 4.0 mmol) was dissolved with dried THF (50 mL), followed by slowly adding NaH (60%, 1.60 g, 40.0 mmol) in an ice bath and reacting for 1 h in an ice bath. After the reaction was over, **S2-6** (1.67 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E30-1.** The crude product was purified by column chromatography, concentrated, and dried with an oil pump to obtain the cationic lipid **E30-1** (2.57 g). The main data of the ¹H-NMR spectrum of **E30-1** are as follows: ¹H NMR (400 MHz, CDCl₃) δ: 8.20 (s, 1H), 7.65 (d, 1H), 7.60 (d, 1H), 7.23-7.20 (m, 2H), 4.45-4.39 (m, 1H), 4.36-3.32 (m, 2H), 4.21-4.08 (m, 3H), 4.07 (t, 2H), 2.63-2.23 (m, 8H), 1.70-1.21 (m, 62H), 0.87 (t, 9H). MS (ESI): m/z = 856.83 ([M+H]⁺).

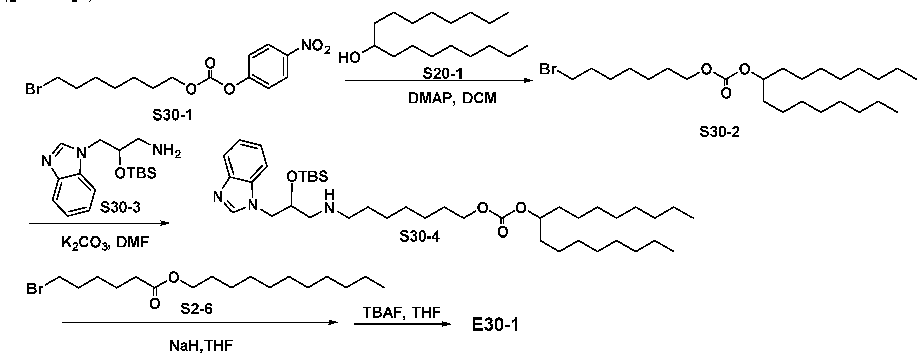

### Example 31: cationic lipid E31-1

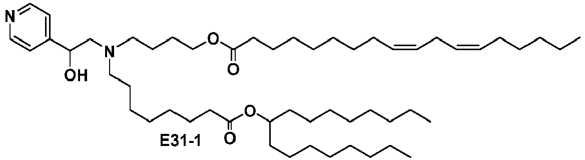

**E31-1** corresponds to the general formula (1); wherein, R₁ is an 8,11-heptadecadienyl, R₂ is 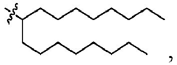 B₁ is a butylene group, B₂ is a heptylene group, L₁ is an ester group (-OC(=O)-), L₂ is an ester group (-C(=O)O-), -L₃(R₃)- is 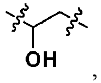 R₃ is -OH, and R₄ is 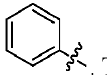 The molecular weight is approximately 853 Da.

The preparation process is as follows:
Step a: **S2-4** (4.42 g, 9.6 mmol) was dissolved with 60 mL DMF, added with **S19-3** which contains a TBS-protected hydroxyl group (2.01 g, 8.0 mmol) and K₂CO₃ (1.59 g, 11.5 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S31-1** (4.41 g).
Step b: The above described compound **S31-1** (3.80 g, 6.0 mmol) was dissolved with dried THF (60 mL), followed by slowly adding NaH (60%, 2.40 g, 60.0 mmol) in an ice bath and reacting for 1 h in an ice bath. After the reaction was over, **S10-3** (2.98 g, 7.2 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (60 mL) was added and mixed while stirring. Then, extraction was performed twice using dichloromethane (30 mL*2). The organic phases were collected and combined, and then washed with a saturated solution of sodium chloride (60 mL). The organic phase was concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E31-1.** The crude product was purified by column chromatography, concentrated, and dried with an oil pump to obtain the cationic lipid **E31-1** (4.04 g). The main data of the ¹H-NMR spectrum of **E31-1** are as follows: ¹H NMR (400 MHz, CDCl₃) δ: 8.42 (d, 2H), 7.21 (d, 2H), 5.39-5.34 (m, 4H), 4.86-4.81 (m, 1H), 4.73-4.66 (m, 1H), 4.05 (t, 2H), 2.73 (t, 2H), 2.57-2.23 (m, 10H), 2.04-1.96 (m, 4H), 1.72-1.23 (m, 58H), 0.87 (t, 9H). MS (ESI): m/z = 853.80 ([M+H]⁺).

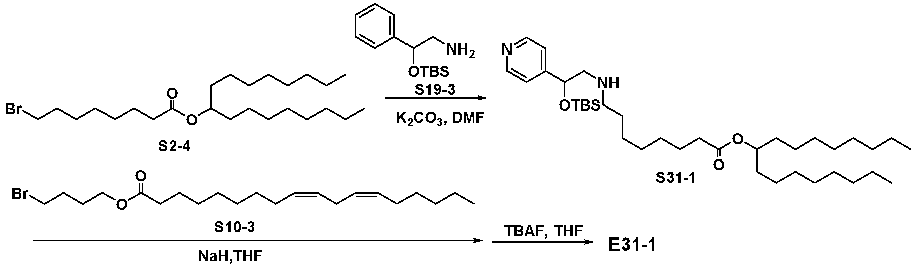

### Example 32: cationic lipid E32-1

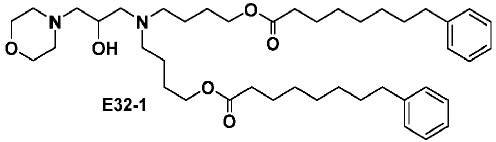

**E32-1** corresponds to the general formula (1); wherein, R₁ and R₂ are both 7-phenylheptyl groups, B₁ and B₂ are both butylene groups, L₁ and L₂ are both ester groups (-OC(=O)-), -L₃(R₃)- is 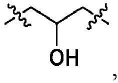 R₃ is -OH, and R₄ is 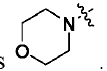 The molecular weight is approximately 709 Da.

The preparation process is as follows:
Step a: Under argon atmosphere, DCC (3.09 g, 15.0 mmol) was added into a round-bottom flask containing 8-phenyloctanoic acid (**S32-1**, 2.20 g, 10.0 mmol), 4-bromobutanol **(S10-2,** 1.67 g, 11.0 mmol) and DMAP (0.31 g, 2.5 mmol) which were dissolved in dichloromethane (40 mL), and reacted overnight at room temperature for 16 h. After the reaction was over, the precipitates were removed by filtration. The filtrate was concentrated, and the residue was purified by silica gel column chromatography to obtain **S32-2** (3.08 g).
Step b: **S32-2** (2.34 g, 6.6 mmol) was dissolved with 40 mL DMF, added with **S10-4** which contains a TBS-protected hydroxyl group (0.82 g, 3.0 mmol) and K₂CO₃ (1.09 g, 7.9 mmol), and reacted overnight at room temperature while stirring. After the reaction was over, the mixture was concentrated under reduced pressure and then added into 40 mL dichloromethane, followed by washing with 10% citric acid (20 mL*2) and brine (20 mL*2) successively. The organic phase was dried over anhydrous sodium sulfate, filtered, concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by silica gel column chromatography to obtain the cationic lipid **E32-1** (1.75 g). The main data of the ¹H-NMR spectrum of **E32-1** are as follows: ¹H NMR (400 MHz, CDCl₃) δ: 7.24-7.19 (m, 10H), 3.90-3.85 (m, 1H), 3.74-3.68 (m, 4H), 4.05 (t, 4H), 2.68-2.23 (m, 20H), 1.69-1.24 (m, 28H). MS (ESI): m/z = 709.66 Da ([M+H]⁺).

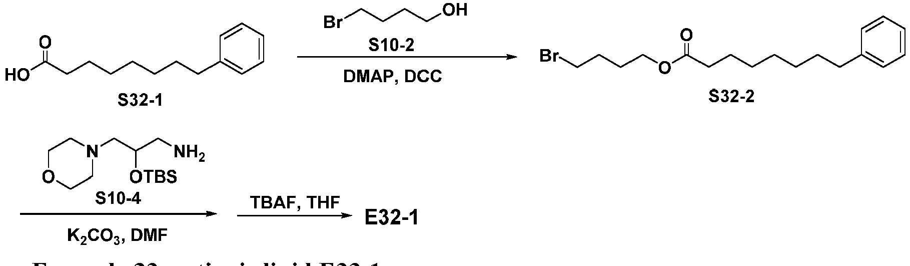

### Example 33: cationic lipid -

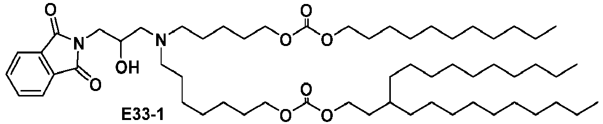

**E33-1** corresponds to the general formula (1); wherein, R₁ is a undecyl group, R₂ is 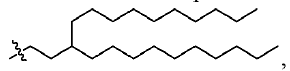 B₁ is a pentylene group, B₂ is a heptylene group, L₁ and L₂ are both carbonate groups (-OC(=O)O-), -L₃(R₃)- is 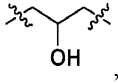 R₃ is -OH, and R₄ is 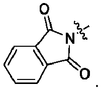 The molecular weight is approximately 985 Da.

The preparation process is as follows:
Step a: **S8-3** (2.62 g, 7.2 mmol, prepared via the reaction of 5-bromopentyl-4-nitrophenyl carbonate and 1-undecanol) was dissolved with 50 mL DMF, added with the derivative of 2-(3-amino-2-hydroxypropyl)-2,3-dihydro-1H-isoindole-1,3-dione containing a TBS-protected hydroxyl group (**S33-1**, 2.00 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S33-2** (2.89 g).
Step b: The above described compound **S33-2** (2.47 g, 4.0 mmol) was dissolved with dried THF (40 mL), followed by slowly adding NaH (60%, 1.60 g, 40.0 mmol) in an ice bath and reacting for 1 h in an ice bath. After the reaction was over, **S33-3** (2.69 g, 4.8 mmol, prepared via the reaction of 7-bromoheptyl-4-nitrophenyl carbonate and 3-decyltridecyl alcohol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (40 mL) was added and mixed while stirring. Then, extraction was performed twice using dichloromethane (20 mL*2). The organic phases were collected and combined, and then washed with a saturated solution of sodium chloride (40 mL). The organic phase was concentrated under reduced pressure, added with 20 mL tetrahydrofuran and further with 20 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E33-1.** The crude product was purified by column chromatography, concentrated, and dried with an oil pump to obtain the cationic lipid **E33-1** (2.99 g). The main data of the ¹H-NMR spectrum of **E33-1** are as follows: ¹H NMR (400 MHz, CDCl₃) δ: 7.90-7.65 (m, 4H), 4.20-3.98 (m, 9H), 3.66-3.59 (m, 2H), 2.61-2.44 (m, 6H), 1.78-1.24 (m, 73H), 0.90 (t, 9H). MS (ESI): m/z = 985.79 ([M+H]⁺).

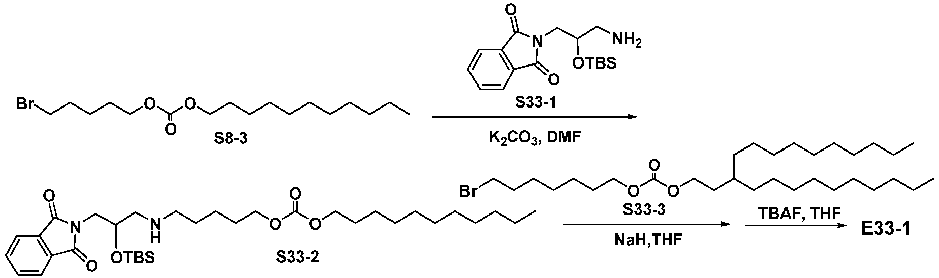

### Example 34: cationic lipid E34-1

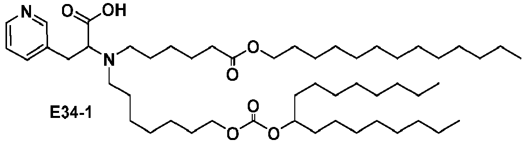

**E34-1** corresponds to the general formula (1); wherein, R₁ is a tridecyl group, R₂ is 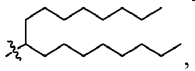 B₁ is a pentylene group, B₂ is a heptylene group, L₁ is an ester group (-C(=O)O-), L₂ is a carbonate group (-OC(=O)O-), -L₃(R₃)- is 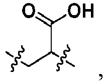 R₃ is -C(=O)OH, and R₄ is 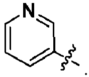 The molecular weight is approximately 859 Da.

The preparation process is as follows:
Step a: **S30-2** (3.43 g, 7.2 mmol) was dissolved with 50 mL DMF, added with 3-(3-pyridyl)-*L*-alanine (**S34-1**, 1.00 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S34-2** (2.66 g).
Step b: The above described compound **S34-2** (2.25 g, 4.0 mmol) was dissolved with dried THF (40 mL), followed by slowly adding NaH (60%, 1.60 g, 40.0 mmol) in an ice bath and reacting for 1 h in an ice bath. After the reaction was over, tridecyl 6-bromohexanoate **(S34-3,** 1.80 g, 7.2 mmol, prepared via the reaction of 6-bromohexanoic acid and 1-tridecyl alcohol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (400 mL) was added and mixed while stirring. Then, extraction was performed twice using dichloromethane (20 mL*2). The organic phases were collected and combined, and then washed with a saturated solution of sodium chloride (40 mL). The organic phase was dried over anhydrous sodium sulfate, filtered, and concentrated to obtain the crude product of **E34-1.** The crude product was purified by column chromatography, concentrated, and dried with an oil pump to obtain the cationic lipid **E34-1** (2.56 g). The main data of the ¹H-NMR spectrum of **E34-1** are as follows: ¹H NMR (400 MHz, CDCl₃) δ: 8.48-7.14 (m, 4H), 4.22-4.06 (m, 3H), 4.09 (t, 2H), 3.73-3.69 (m, 1H), 3.08-3.03 (m, 2H), 2.54-2.23 (m, 6H), 1.70-1.20 (m, 66H), 0.87 (t, 9H). MS (ESI): m/z = 859.75 ([M+H]⁺).

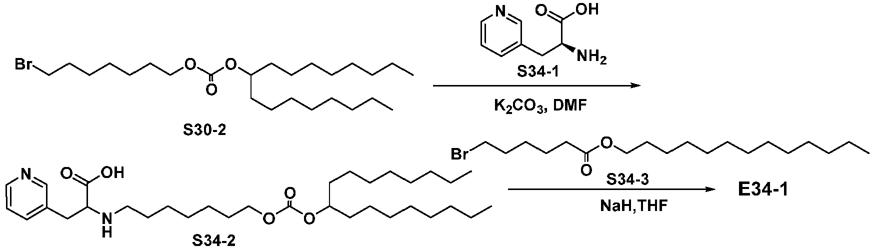

### Example 35: cationic lipid E35-1

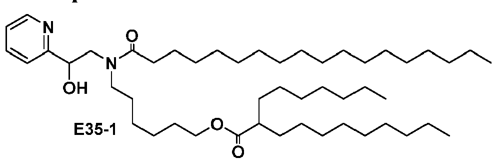

**E35-1** corresponds to the general formula (1); wherein, R₁ is a heptadecyl group, R₂ is 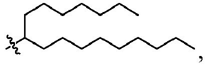 B₁ is a linking bond, B₂ is a hexylene group, L₁ is a carbonyl group (-C(=O)-), L₂ is an ester group (-OC(=O)-), -L₃(R₃)- is 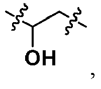 R₃ is -OH, and R₄ is 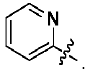 The molecular weight is approximately 771 Da.

The preparation process is as follows:
Step a: Under argon atmosphere, DCC (6.18 g, 30.0 mmol) was added into a round-bottom flask containing isostearic acid (**S35-1**, 5.68 g, 20.0 mmol), 6-bromohexanol (**S35-6**, 3.96 g, 22.0 mmol) and DMAP (0.61 g, 5.0 mmol) which were dissolved in dichloromethane (70 mL), and reacted overnight at room temperature for 16 h. After the reaction was over, the precipitates were removed by filtration. The filtrate was concentrated, and the residue was purified by silica gel column chromatography to obtain **S35-2** (5.98 g).
Step b: **S35-2** (5.35 g, 12.0 mmol) was dissolved with 60 mL DMF, added with the derivative of 2-amino-1-(2-pyridyl)ethanol containing a TBS-protected hydroxyl group (**S35-3**, 2.52 g, 10.0 mmol) and K₂CO₃ (1.99 g, 14.4 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S35-4** (4.85 g).
Step c: Compound **S35-4** (4.64 g, 7.5 mmol), stearic acid (**S35-5**, 1.42 g, 5.0 mmol), 1-ethyl-3-(3-dimethylaminopropyl)carbodiimide hydrochloride (EDCI, 1.44 g, 7.5 mmol), 1-hydroxybenzotriazole (HOBt, 0.88 g, 6.5 mmol), and triethanol (TEA, 1.01 g, 10.0 mmol) were successively dissolved in dichloromethane (60 mL), and reacted overnight at room temperature while stirring. After the reaction was over, the reaction solution was washed twice with an aqueous solution of 0.1 mol/L HCl (10% NaCl) (60 mL*2), and further washed with a saturated solution of NaCl (60 mL). The organic phase was concentrated under reduced pressure, added with 30 mL tetrahydrofuran and further with 30 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by column chromatography to obtain the target compound **E35-1** (2.97 g). The main data of the ¹H-NMR spectrum of **E35-1** are as follows: ¹H NMR (400 MHz, CDCl₃) δ: 8.56-8.52 (m, 1H), 7.73-7.65 (m, 1H), 7.55-7.51 (m, 1H), 7.20-7.15 (m, 1H), 4.79-7.76 (m, 1H), 4.10-4.05 (t, 2H), 3.71-3.59 (m, 2H), 3.16 (t, 2H), 2.35-2.28 (m, 1H), 2.23 (t, 2H), 1.70-1.19 (m, 66H), 0.88 (t, 9H). MS (ESI): m/z = 771.74 [M+H]⁺.

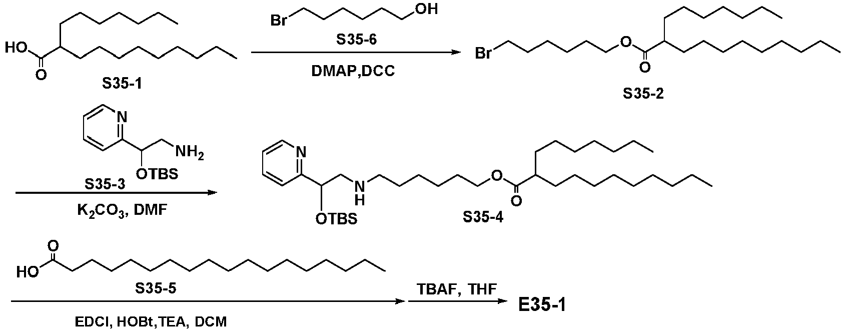

### Example 36: cationic lipid E36-1

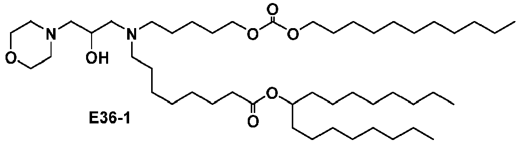

**E36-1** corresponds to the general formula (1); wherein, R₁ is an undecyl group, R₂ is 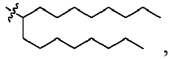 B₁ is a pentylene group, B₂ is a heptylene group, L₁ is a carbonate group (-OC(=O)O-), L₂ is an ester group (-C(=O)O-), -L₃(R₃)- is 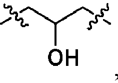 R₃ is -OH, and R₄ is 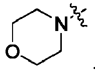 . The molecular weight is approximately 825 Da.

The preparation process is as follows:
Step a: **S8-3** (2.62 g, 7.2 mmol) was dissolved with 50 mL DMF, added with **S10-4** which contains a TBS-protected hydroxyl group (1.64 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S36-1** (2.73 g).
Step b: The above described compound **S36-1** (2.23 g, 4.0 mmol) was dissolved with dried THF (30 mL), followed by slowly adding NaH (60%, 1.60 g, 40.0 mmol) in an ice bath and reacting for 1 h in an ice bath. After the reaction was over, compound **S2-4** (2.21 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (30 mL) was added and mixed while stirring. Then, extraction was performed twice using dichloromethane (15 mL*2). The organic phases were collected and combined, and then washed with a saturated solution of sodium chloride (30 mL). The organic phase was concentrated under reduced pressure, added with 15 mL tetrahydrofuran and further with 15 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E36-1.** The crude product was purified by column chromatography, concentrated, and dried with an oil pump to obtain the cationic lipid **E36-1** (2.61 g). The main data of the ¹H-NMR spectrum of **E36-1** are as follows: ¹H NMR (400 MHz, CDCl₃) δ: 4.89-4.85 (m, 1H), 4.22-4.03 (m, 4H), 3.93-3.85 (m, 1H), 3.73-3.67 (m, 4H), 2.69-2.22 (m, 14H), 1.76-1.22 (m, 62H), 0.87 (t, 9H). MS (ESI): m/z = 825.78 ([M+H]⁺).

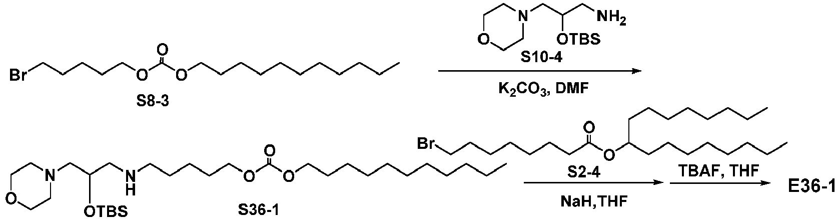

### Example 37: cationic lipid E37-1

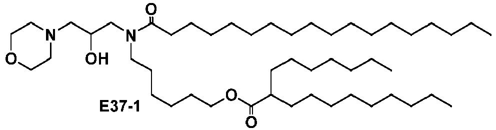

**E37-1** corresponds to the general formula (1); wherein, R₁ is a heptadecyl group, R₂ is 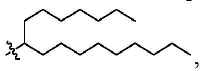 B₁ is a linking bond, B₂ is a hexylene group, L₁ is a carbonyl group (-C(=O)-), L₂ is an ester group (-OC(=O)-), -L₃(R₃)- is 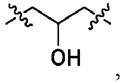 R₃ is -OH, and R₄ is 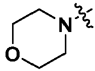 . The molecular weight is approximately 793 Da.

The preparation process is as follows:
Step a: **S35-2** (5.35 g, 12.0 mmol) was dissolved with 60 mL DMF, added with **S10-4** which contains a TBS-protected hydroxyl group (2.74 g, 10.0 mmol) and K₂CO₃ (1.99 g, 14.4 mmol), and reacted overnight at room temperature while stirring. After the reaction was over, the mixture was concentrated under reduced pressure and then added into 60 mL dichloromethane, followed by washing with 10% citric acid (30 mL*2) and brine (30 mL*2) successively. The organic phase was dried over anhydrous sodium sulfate, filtered, concentrated under reduced pressure, and purified by silica gel column chromatography to obtain **S37-1** (5.06 g).
Step b: Compound **S37-1** (4.81 g, 7.5 mmol), stearic acid (**S35-5**, 1.42 g, 5.0 mmol), 1-ethyl-3-(3-dimethylaminopropyl)carbodiimide hydrochloride (EDCI, 1.44 g, 7.5 mmol), 1-hydroxybenzotriazole (HOBt, 0.88 g, 6.5 mmol), and TEA (1.01 g, 10.0 mmol) were successively dissolved in dichloromethane (80 mL), and reacted overnight at room temperature while stirring. After the reaction was over, the reaction solution was washed twice with an aqueous solution of 0.1 mol/L HCl (10% NaCl) (80 mL*2), and further washed with a saturated solution of NaCl (80 mL). The organic phase was concentrated under reduced pressure, added with 40 mL tetrahydrofuran and further with 40 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by column chromatography, concentrated, and dried with an oil pump to obtain the target compound **E37-1** (4.16 g). The main data of the ¹H-NMR spectrum of **E37-1** are as follows: ¹H NMR (400 MHz, CDCl₃) δ: 4.05 (t, 2H), 3.86-3.80 (m, 1H), 3.69-3.65 (m, 4H), 3.63-3.20 (m, 2H), 3.14 (t, 2H), 2.66-2.19 (m, 9H), 1.68-1.18 (m, 66H), 0.87 (t, 9H). MS (ESI): m/z = 793.75 ([M+H]⁺).

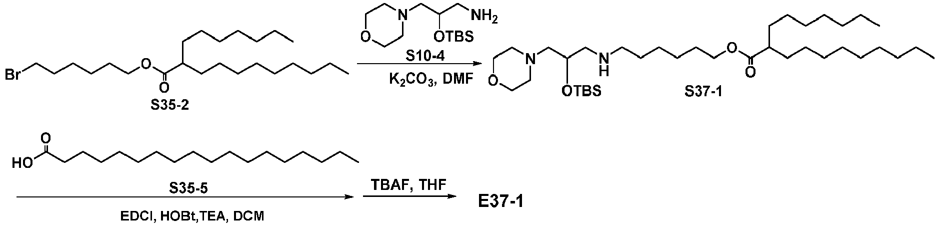

### Example 38: cationic lipid E38-1

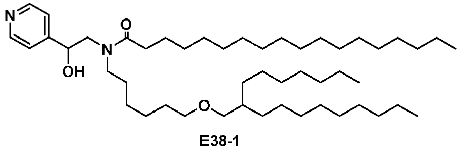

**E38-1** corresponds to the general formula (1); wherein, R₁ is a heptadecyl group, R₂ is 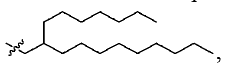 B₁ is a linking bond, B₂ is a hexylene group, L₁ is a carbonyl group (-C(=O)-), L₂ is -O-, -L₃(R₃)- is 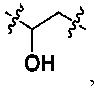 R₃ is -OH, and R₄ is 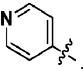 The molecular weight is approximately 757 Da.

The preparation process is as follows:
Step a: **S24-3** (3.11 g, 7.2 mmol) was dissolved with 50 mL DMF, added with **S7-3** which contains a TBS-protected hydroxyl group (1.51 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S38-1** (2.90 g).
Step b: Compound **S38-1** (2.72 g, 4.5 mmol), **S35-5** (0.85 g, 3.0 mmol), EDCI (0.86 g, 4.5 mmol), HOBt (0.53 g, 3.9 mmol), and TEA (0.61 g, 6.0 mmol) were successively dissolved in dichloromethane (50 mL), and reacted overnight at room temperature while stirring. After the reaction was over, the reaction solution was washed twice with an aqueous solution of 0.1 mol/L HCl (10% NaCl) (50 mL*2), and further washed with a saturated solution of NaCl (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by column chromatography, concentrated, and dried with an oil pump to obtain the target compound **E38-1** (1.75 g). The main data of the ¹H-NMR spectrum of **E38-1** are as follows: ¹H NMR (400 MHz, CDCl₃) δ: 8.44 (d, 2H), 7.24 (d, 2H), 4.74-4.69 (m, 1H), 3.71-3.41 (m, 6H), 3.18 (t, 2H), 2.24 (t, 2H), 1.55-1.21 (m, 67H), 0.88 (t, 9H). MS (ESI): m/z = 757.77 ([M+H]⁺).

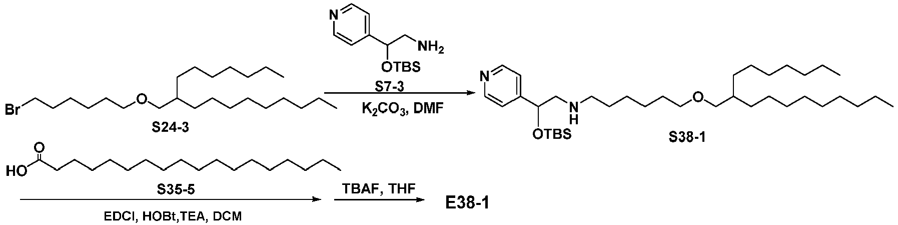

### Example 39: cationic lipid E39-1

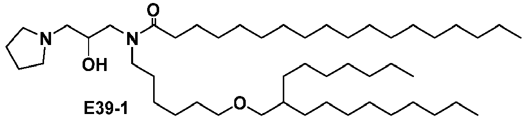

**E39-1** corresponds to the general formula (1); wherein, R₁ is a heptadecyl group, R₂ is 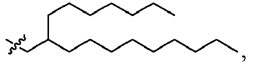 B₁ is a linking bond, B₂ is a hexylene group, L₁ is a carbonyl group (-C(=O)-), L₂ is -O-, -L₃(R₃)- is 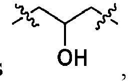 R₃ is -OH, and R₄ is 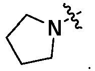 The molecular weight is approximately 763 Da.

The preparation process is as follows:
Step a: **S24-3** (3.11 g, 7.2 mmol) was dissolved with 50 mL DMF, added with the derivative of 1-amino-3-(1-pyrrolidinyl)-2-propanol containing a TBS-protected hydroxyl group (**S39-1**, 1.55 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S39-2** (3.01 g).
Step b: Compound **S39-2** (2.75 g, 4.5 mmol), **S35-5** (0.85 g, 3.0 mmol), EDCI (0.86 g, 4.5 mmol), HOBt (0.53 g, 3.9 mmol), and TEA (0.61 g, 6.0 mmol) were successively dissolved in dichloromethane (50 mL), and reacted overnight at room temperature while stirring. After the reaction was over, the reaction solution was washed twice with an aqueous solution of 0.1 mol/L HCl (10% NaCl) (50 mL*2), and further washed with a saturated solution of NaCl (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E39-1.** The crude product was purified by column chromatography, concentrated, and dried with an oil pump to obtain the target compound **E39-1** (1.79 g). The main data of the ¹H-NMR spectrum of **E39-1** are as follows: ¹H NMR (400 MHz, CDCl₃) δ: 3.82-3.21 (m, 7H), 3.19 (t, 2H), 2.45-2.38 (m, 2H), 2.25-2.19 (m, 6H), 1.63-1.20 (m, 71H), 0.87 (t, 9H). MS (ESI): m/z = 763.81 ([M+H]⁺).

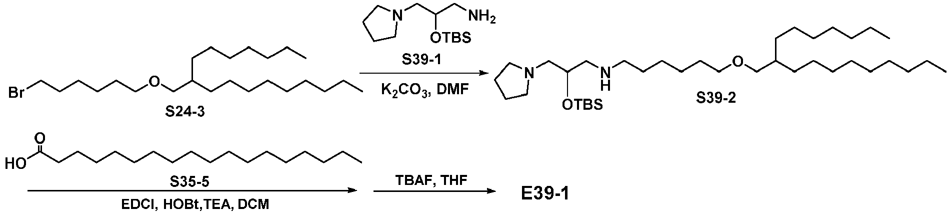

### Example 40: cationic lipid E40-1

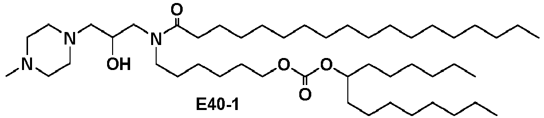

**E40-1** corresponds to the general formula (1); wherein, R₁ is a heptadecyl group, R₂ is 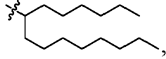 B₁ is a linking bond, B₂ is a hexylene group, L₁ is a carbonyl group (-C(=O)-), L₂ is a carbonate group (-OC(=O)O-), -L₃(R₃)- is 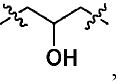 , R₃ is -OH, and R₄ is 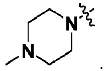 The molecular weight is approximately 794 Da.

The preparation process is as follows:
Compound **S22-1** which contains a TBS-protected hydroxyl group (2.89 g, 4.5 mmol), **S35-5** (0.85 g, 3.0 mmol), EDCI (0.86 g, 4.5 mmol), HOBt (0.53 g, 3.9 mmol), and TEA (0.61 g, 6.0 mmol) were successively dissolved in dichloromethane (50 mL), and reacted overnight at room temperature while stirring. After the reaction was over, the reaction solution was washed twice with an aqueous solution of 0.1 mol/L HCl (10% NaCl) (50 mL*2), and further washed with a saturated solution of NaCl (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product which was purified by column chromatography, concentrated, and dried with an oil pump to obtain the target compound **E40-1** (2.05 g). The main data of the ¹H-NMR spectrum of **E40-1** are as follows: ¹H NMR (400 MHz, CDCl₃) δ: 4.24-4.09 (m, 3H), 3.86-3.79 (m, 1H), 3.64-3.22 (m, 2H), 3.13 (t, 2H), 2.68-2.18 (m, 15H), 1.70-1.19 (m, 62H), 0.86 (t, 9H). MS (ESI): m/z = 794.77 ([M+H]⁺).

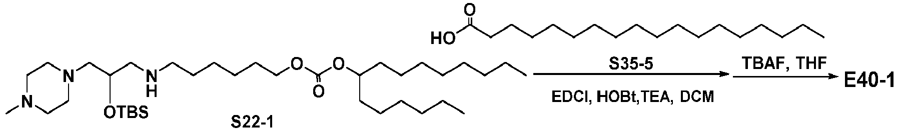

### Example 41: cationic lipid E41-1

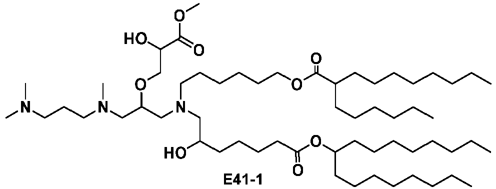

**E41-1** corresponds to the general formula (1); wherein, R₁ is 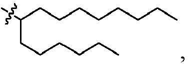 R₂ is 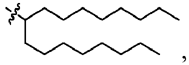 B₁ is a hexylene group, B₂ is a hexylene group substituted by -OH, L₁ is an ester group (-OC(=O)-), L₂ is an ester group (-C(=O)O-), -L₃(R₃)- is 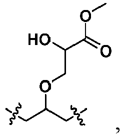 R₃ is 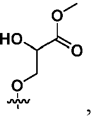 G₁ is -OCH₂CH<, k is 2, two F₁ are respectively -OH and -C(=O)OCH₃, and R₄ is 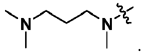 The molecular weight is approximately 1012 Da.

The preparation process is as follows:
Step a: Into a dried 500 mL round-bottom flask, **S2-3** (3.40 g, 18.0 mmol), 60 mL dichloromethane solution, and di-tert-butyl dicarbonate (4.71 g, 21.6 mmol) were added successively and then reacted overnight at room temperature, followed by adding a saturated solution of sodium bicarbonate and then extracting with dichloromethane (20 mL*3). The organic phases were combined, washed with saturated brine, dried, filtered, concentrated, and recrystallized to obtain the amine derivative **S41-1** (4.42 g) protected by a tert-butoxycarbonyl group (Boc).
Step b: Into a sealed reactor under anhydrous and oxygen-free conditions, 150 mL tetrahydrofuran, excessive amounts of diphenylmethyl potassium (10.30 g, 50.0 mmol), compound **S41-1** (3.76 g, 13.0 mmol) and compound **S41-2** (5.04 g, 13.0 mmol) were added successively, wherein **S41-2** is the derivative of methyl 2,3-dihydroxypropionate having one hydroxyl group substituted with a *p*-toluenesulfonate group and another hydroxyl group protected by TBS. After 12 h of reaction at 30°C, the reactor was opened. The reaction solution was concentrated and washed, and further dissolved with dichloromethane. TFA was added until its concentration reached 0.1 M. After 4 h of reaction, the pH was adjusted to neutral. The reaction solution was concentrated, added with purified water, and extracted with dichloromethane. The extract was dried over anhydrous sodium sulfate and filtered, and the filtrate was concentrated and recrystallized to obtain the intermediate **S41-3** with a free amino group (3.56 g).
Step c: **S20-4** (3.21 g, 8.4 mmol) was dissolved with 50 mL ethanol, added with **S41-3** (2.84 g, 7.0 mmol), and reacted at 50°C for 4 h while stirring. After the reaction was over, the mixture was diluted with 50 mL dichloromethane and then washed with 100 mL saturated solution of sodium chloride. The organic phase was isolated, dried over anhydrous sodium sulfate, filtered, concentrated, and purified by silica gel column chromatography to obtain **S41-4** (3.64 g).
Step d: The above descried compound **S41-4** (3.15 g, 4.0 mmol) and imidazole (0.45 g, 6.6 mmol) were dissolved with 50 mL dichloromethane, and added with tert-butyldimethylchlorosilane (0.84 g, 5.6 mmol) in an ice bath. After 2 h of reaction, the mixture was filtered and washed with 50 mL dichloromethane. The filtrates were combined, dried over anhydrous sodium sulfate, and concentrated to obtain the intermediate **S41-5** containing two TBS-protected hydroxyl groups (3.54 g).
Step e: The above described compound **S41-5** (2.71 g, 3.0 mmol) was dissolved with dried THF (50 mL), followed by slowly adding NaH (60%, 1.20 g, 30.0 mmol) in an ice bath and reacting for 1 h in an ice bath. After the reaction was over, **S3-3** (1.50 g, 3.6 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure, added with 25 mL tetrahydrofuran and further with 25 mL solution of TBAF in tetrahydrofuran (1M). The deprotection reaction was continued overnight and the TBS was removed. After the reaction was over, the reaction solution was concentrated and extracted. The organic phases were combined, dried over anhydrous sodium sulfate, filtered, and concentrated to obtain the crude product of **E41-1.** The crude product was purified by column chromatography, concentrated, and dried with an oil pump to obtain the cationic lipid **E41-1** (2.46 g). The main data of the ¹H-NMR spectrum of **E41-1** are as follows: ¹H NMR (400 MHz, CDCl₃) δ: 4.82-4.75 (m, 1H), 4.15-4.10 (m, 1H), 4.08 (t, 2H), 3.86-3.80 (m, 1H), 3.68 (s, 3H), 3.65-3.47 (m, 3H), 2.70-2.19 (m, 24H), 1.73-1.23 (m, 68H), 0.87 (t, 12H). MS (ESI): m/z = 1012.93 ([M+H]⁺).

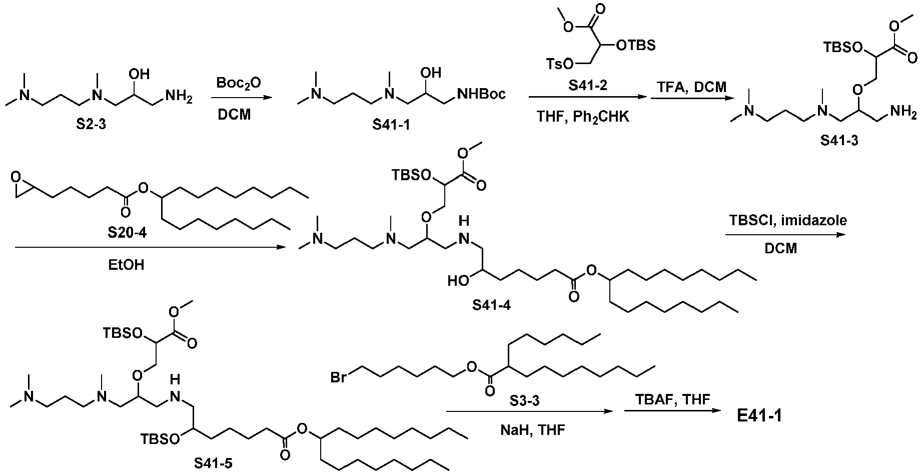

### Example 42: cationic lipid E42-1

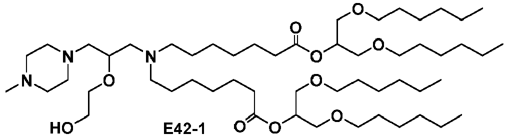

**E42-1** corresponds to the general formula (1); wherein, R₁ and R₂ are both 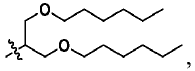 B₁ and B₂ are both hexylene groups, L₁ and L₂ are both ester groups (-OC(=O)-), -L₃(R₃)- is 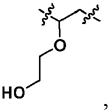 R₃ is -O(CH₂)₂OH, and R₄ is 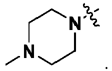 The molecular weight is approximately 958 Da.

The preparation process is as follows:
Step a: Into a sealed reactor under anhydrous and oxygen-free conditions, 150 mL tetrahydrofuran, excessive amounts of diphenylmethyl potassium (10.30 g, 50.0 mmol), compound **S42-1** (2.73 g, 10.0 mmol) and compound **S42-2** (2.88 g, 10.0 mmol) were added successively, wherein **S42-2** is the derivative of ethylene glycol having one hydroxyl group substituted with a *p*-toluenesulfonate group (-OTs) and another hydroxyl group protected by EE. After 12 h of reaction at 30°C, the reactor was opened. The reaction solution was concentrated, washed, and purified by column chromatography to obtain compound **S8-1** with two protected hydroxyl groups and one protected amino group (3.27 g).
Step b: Into a dried and clean container, **S8-1** (2.33 g, 6.0 mmol) was added and dissolved with dichloromethane. TFA was added until its concentration reached 0.1 M. After 4 h of reaction, the pH was adjusted to neutral. The reaction solution was concentrated, added with purified water, and extracted with dichloromethane. The extract was concentrated, precipitated, filtered, and dried to obtain the intermediate **S42-3** with a free amino group (1.42 g).
Step c: **S42-4** (2.97 g, 6.6 mmol, prepared via the condensation reaction of **S5-3** and **S4-1**) was dissolved with 50 mL DMF, added with **S42-3** (0.87 g, 3.0 mmol) and K₂CO₃ (1.09 g, 7.9 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and dissolved with methanol. The 1M hydrochloric acid was added to the solution until pH=3.5. After 4 h of reaction, the reaction solution was concentrated, precipitated, filtered, recrystallized, and dried to obtain the crude product of **E42-1** with a free hydroxyl group. The crude product was purified by silica gel column chromatography to obtain **E42-1** (2.34 g). The main data of the ¹H-NMR spectrum **of E42-1** are as follows: ¹H NMR (400 MHz, CDCl₃) δ: 5.04-4.96 (m, 2H), 3.89-3.83 (m, 1H), 3.71-3.33 (m, 20H), 2.74-2.24 (m, 23H), 1.77-1.16 (m, 48H), 0.85 (t, 12H). MS (ESI): m/z = 958.80 ([M+H]⁺).

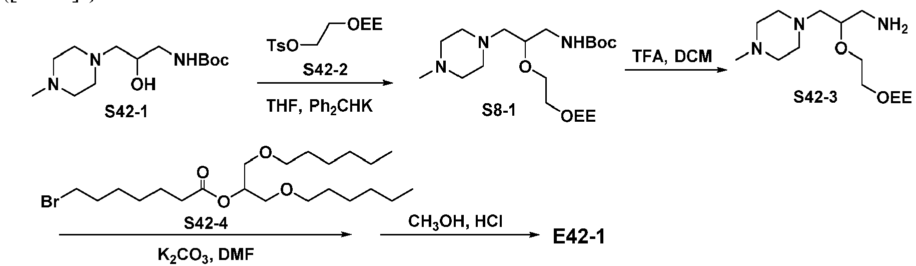

### Example 43: cationic lipid E43-1

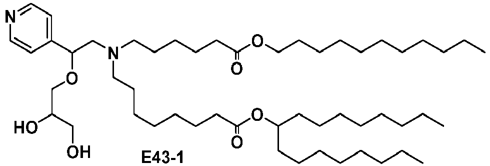

**E43-1** corresponds to the general formula (1); wherein, R₁ is an undecyl group, R₂ is 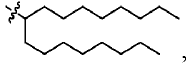 B₁ is a pentylene group, B₂ is a heptylene group, L₁ and L₂ are both ester groups (-OC(=O)-), -L₃(R₃)- is 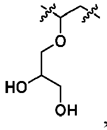 , R₃ is 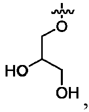 and R₄ is 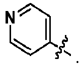 The molecular weight is approximately 861 Da.

The preparation process is as follows:
Step a: **S2-4** (3.31 g, 7.2 mmol) was dissolved with 50 mL DMF, added with **S11-3** (2.14 g, 6.0 mmol) and K₂CO₃ (1.19 g, 8.6 mmol), and reacted overnight at room temperature while stirring. After the reaction was over, the mixture was concentrated under reduced pressure and then added into 50 mL dichloromethane, followed by washing with 10% citric acid (25 mL*2) and brine (25 mL*2) successively. The organic phase was dried over anhydrous sodium sulfate, filtered, concentrated under reduced pressure, and purified by silica gel column chromatography to obtain **S43-5** (3.63 g).
Step b: The above described compound **S43-5** (2.95 g, 4.0 mmol) was dissolved with dried THF (50 mL), followed by slowly adding NaH (60%, 1.60 g, 40.0 mmol) in an ice bath and reacting for 1 h in an ice bath. After the reaction was over, **S2-6** (1.67 g, 4.8 mmol) was added and the reaction was continued for 1 h while stirring in an ice bath, followed by slowly restoring the temperature of reaction solution to room temperature and then reacting overnight. After the reaction was over, an ice bath was used and then 2 mL water was added slowly to quench the reaction. After stirring for 30 min, water (50 mL) was added and mixed while stirring. Then, extraction was performed twice using dichloromethane (25 mL*2). The organic phases were collected and combined, and then washed with a saturated solution of sodium chloride (50 mL). The organic phase was concentrated under reduced pressure and then dissolved with methanol. The 1M hydrochloric acid was added to the solution until pH=3.5. After 4 h of reaction, the reaction solution was concentrated, precipitated, filtered, recrystallized, and dried to obtain the crude product of **E43-1** with a free hydroxyl group. The crude product was purified by column chromatography to obtain **E43-1** (2.65 g). The main data of the ¹H-NMR spectrum of **E43-1** are as follows: ¹H NMR (400 MHz, CDCl₃) δ: 8.55 (d, 2H), 7.20 (d, 2H), 4.84-4.77 (m, 1H), 4.29-4.25 (m, 1H), 4.07 (t, 2H), 3.77-3.72 (m, 1H), 3.63-3.56 (m, 2H), 3.42-3.36 (m, 2H), 3.01-2.94 (m, 2H), 2.57-2.24 (m, 8H), 1.75-1.23 (m, 62H), 0.89 (t, 9H). MS (ESI): m/z = 861.76 ([M+H]⁺).

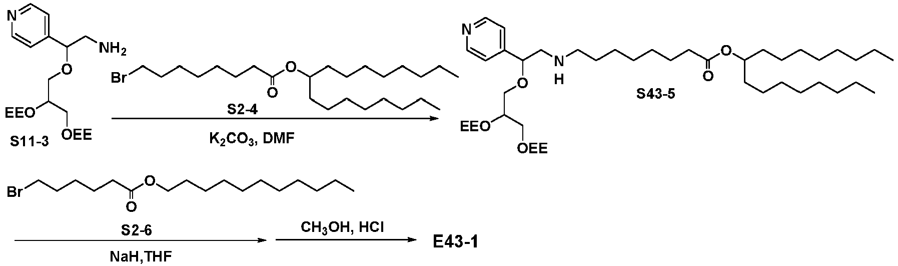

### Example 44: PEGylated lipid E44-1

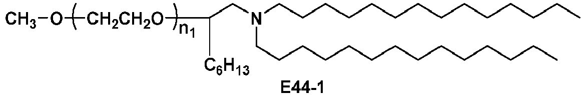

**E44-1** corresponds to the general formula (2); wherein, R₁ and R₂ are both tetradecyl groups, B₁ and B₂ are both linking bonds, L₁ and L₂ are both linking bonds, L₃ is 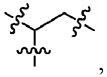 L_{A} is -(CH₂)ₕ- wherein h is 0, A₁ is -OCH₂CH₂-, n₁≈₄₅, R₃ is a methyl group, and R₅ is a hexyl group. The molecular weight is approximately 2.5 kDa.

The preparation process is as follows:
Step a: Into a sealed reactor under anhydrous and oxygen-free conditions, 120 mL tetrahydrofuran, excessive amounts of diphenylmethyl potassium (10.30 g, 50.0 mmol), compound **S44-2** (2.45 g, 10.0 mmol) and a sulfonate derivative of methoxypolyethylene glycol mPEG-CH₂CH₂OTs (**S44-1**, 12.96 g, 6.0 mmol, Mₙ≈2.2 kDa, n₁≈45, PDI=1.03) were added successively. After 12 h of reaction at 30°C, the reactor was opened. The reaction solution was concentrated and washed. After removing the Boc protecting group with a mixed solution of TFA/DCM (1:1 v/v), the reaction solution was washed with purified water and then extracted with dichloromethane. The extract was dried over anhydrous sodium sulfate, filtered, concentrated, and purified by column chromatography to obtain the PEGylated intermediate **S44-3** (5.91 g).
Step b: **S44-4** (1.21 g, 4.4 mmol) was dissolved with 30 mL DMF, added with **S44-3** (4.28 g, 2.0 mmol, Mₙ≈2.1 kDa, n₁≈45, PDI=1.03) and K₂CO₃ (0.73 g, 5.3 mmol), and reacted overnight at room temperature while stirring. After the reaction was over, the mixture was concentrated under reduced pressure and then added into 30 mL dichloromethane, followed by washing with 10% citric acid (15 mL*2) and brine (15 mL*2) successively. The organic phase was dried over anhydrous sodium sulfate, filtered, and concentrated under reduced pressure to obtain the crude product which was further purified by silica gel column chromatography to obtain **E44-1** (2.92 g). The main data of the ¹H-NMR spectrum of **E44-1** are as follows: ¹H NMR (400 MHz, CDCl₃) δ: 3.85-3.45 (m, PEG), 3.37 (s, 3H), 3.31-3.26 (m, 1H), 2.67-2.60 (m, 2H), 2.50-2.41 (m, 4H), 1.68-1.19 (m, 58H), 0.88 (t, 9H). The molecular weight of **E44-1** from GPC analysis was approximately 2.5 kDa, with PDI=1.03.

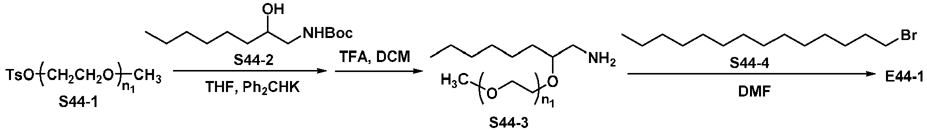

### 5.2. Lipid pharmaceutical compositions

### Example 45: preparation of LNP-mRNA pharmaceutical compositions

In this embodiment, LNP-mRNA pharmaceutical compositions containing Fluc-mRNA were prepared; wherein, the neutral lipids contained were all DSPC, the steroid lipids contained were all cholesterol, the cationic lipids contained were cationic lipids of the present invention, DLin-MC3-DMA (abbreviated as MC3) or reference compounds **d-1**~**d-4**, the PEGylated lipids contained were PEGylated lipids of the present invention or PEG2k-DMG (abbreviated as DMG), and the nucleic acid drugs contained were all Fluc-mRNA; wherein, the structures of **d-1**~**d-4** are as follows:

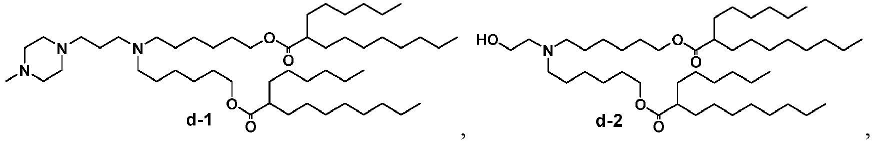

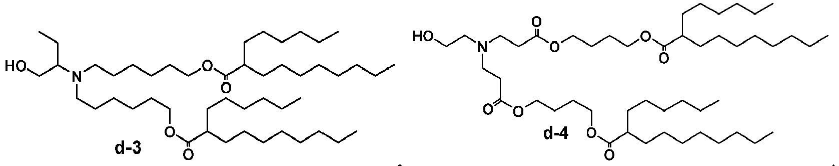

The method for the preparation of LNP-mRNA pharmaceutical compositions is as follows:
Step (1): Cationic lipid, DSPC, cholesterol, and PEGylated lipid (specifically as illustrated by Table 1 in **Example 46**) were dissolved with ethanol at the ratio of 50:10:38:1.5 to obtain the ethanol phase solution.
Step (2): The Fluc-mRNA was added into a 10-50 mM citrate buffer (pH=4) to obtain the aqueous phase solution.
Step (3): The ethanol phase solution and the aqueous phase solution were mixed (1:3 v/v) to prepare LNP/Fluc-mRNA. The DPBS ultrafiltration was performed multiple times to wash and remove ethanol and free molecules. Finally, the solution was passed through a 0.2 µm sterile filter, for further use.

Following the above steps (1-3), LNP/Fluc-mRNA was also prepared at the molar ratio of lipids modified as cationic lipid:DSPC:cholesterol:PEGylated lipid = 48:9:42:1.5, with the rest conditions unchanged; wherein, the cationic lipid was **E3-2**, and the PEGylated lipid was **E44-1.**

### Example 46: biological assays for lipid pharmaceutical compositions

### (1) Determination of nanoparticle size and nucleic acid complexation ability

Determination of nucleic acid complexation ability: The gel permeation electrophoresis experiment was carried out to examine the nucleic acid complexation ability of LNP/Fluc-mRNA. 0.8 g agarose was weighed and dissolved in 40 mL solution of TAE. The solution was heated in a microwave until the granules of agarose were completely dissolved, and then cooled. 5 µL nucleic acid dye (GelGreen) was added in the cooled agarose gel which was then added into the gel slot and dried with natural air. The mixed solution of LNP/Fluc-mRNA and 2 µL loading buffer was added into the agarose gel well, and the electrophoresis voltage was set to 90 V. The electrophoresis was carried out at room temperature for 10 min. The results showed that free Fluc-mRNA hardly existed in both the LNP/Fluc-mRNA experimental groups (**L-1**~**L-43**, **L-3-2-d**, **PL-0**, **PL-3-2**, **PL-4**, **PL-10-2**, **PL-16**, **PL-17**) and the LNP/Fluc-mRNA control group **D-0**, indicating that the LNP of the present invention has a good ability to complex with nucleic acid drugs.

Determination of encapsulation efficiency: The LNP/Fluc-mRNA was ultracentrifuged (4°C, 60000 rpm, 1 h) with an ultracentrifuge. The concentration of unencapsulated Fluc-mRNA in the supernatant was determined by a nucleic acid quantifier. The encapsulation efficiency of the liposomes for Fluc-mRNA was calculated. The results are summarized in **Table 1**, which indicate that the liposomes of the present invention have higher encapsulation efficiency for nucleic acid drugs; wherein, the encapsulation efficiency of the experimental groups are all above 80%. Particularly, compared with **E16-1**, the polar head of **d-1** does not have a side-chain hydroxyl group, the polar head of **d-2** does not have a branched structure, and the polar head of **d-3** does not have an azacycle, while accordingly, the encapsulation efficiency of the experimental group **L-16** (95%) is higher than those of **D-1**, **D-2**, and **D-3** (81%, 91%, 83%). **E1-1** and **d-4** both have multiple ester bonds, while the encapsulation efficiency of **L-1** (87%) is higher than that of **D-4** (81%). Comparing **L3-2** with **L3-2-d**, both have the same types of lipids, except with the ratios of lipids varied to a certain degree, and both have higher encapsulation efficiency.

Determination of particle size: According to the literature (Hassett et al., J. Controlled Release 2021, 335, 237-246), a formulation of LNP loaded with nucleic acid drugs can exhibit better pharmaceutical efficacy when its particle size is in the range of 60-150 nm. In this embodiment, the particle size of LNP/Fluc-mRNA was determined by dynamic light scattering (DLS). The measured sizes of LNP/Fluc-mRNA were relatively uniform, and the PDI values were all smaller than 0.3. The results showed that the particle sizes of LNP/Fluc-mRNA prepared with the cationic lipids of the present invention were in the range of approximately 70 nm to approximately 113 nm, which were all within the range of the particle size that can achieve better pharmaceutical efficacy.

**Table 1: Determination of Particle Size and Encapsulation Efficiency of LNP/Fluc-mRNA**

| **LNP/Fluc-mRNA** | **D-0** | **D-1** | **D-2** | **D-3** | **D-4** | |
|---|---|---|---|---|---|---|
| **Cationic Lipid** | MC3 | d-1 | d-2 | d-3 | d-4 | |
| **PEGylated Lipid** | DMG | | | | | |
| **Encapsulation Efficiency/%** | 91 | 81 | 91 | 83 | 81 | |
| **Particle Size/nm** | 95 | 78 | 97 | 102 | 96 | |

| **LNP/Fluc-mRNA** | **L-1** | **L-2** | **L-3-1** | **L-3-2** | **L-4** | **L-5** |
|---|---|---|---|---|---|---|
| **Cationic Lipid** | E1-1 | E2-1 | E3-1 | E3-2 | E4-1 | E5-1 |
| **PEGylated Lipid** | DMG | | | | | |
| **Encapsulation Efficiency/%** | 87 | 96 | 89 | 98 | 92 | 95 |
| **Particle Size/nm** | 91 | 81 | 85 | 86 | 79 | 78 |

| **LNP/Fluc-mRNA** | **L-6** | **L-7** | **L-8** | **L-9** | **L-10-1** | **L-10-2** |
|---|---|---|---|---|---|---|
| **Cationic Lipid** | E6-1 | E7-1 | E8-1 | E9-1 | E10-1 | E 10-2 |
| **PEGylated Lipid** | DMG | | | | | |
| **Encapsulation Efficiency/%** | 85 | 88 | 92 | 87 | 86 | 94 |
| **Particle Size/nm** | 89 | 94 | 101 | 85 | 113 | 76 |

| **LNP/Fluc-mRNA** | **L-11** | **L-12** | **L-13** | **L-14** | **L-15** | **L-16** |
|---|---|---|---|---|---|---|
| **Cationic Lipid** | E11-1 | E12-1 | E13-1 | E14-1 | E15-1 | E16-1 |
| **PEGylated Lipid** | | | DMG | | | |
| **Encapsulation Efficiency/%** | 87 | 90 | 81 | 87 | 88 | 95 |
| **Particle Size/nm** | 96 | 70 | 99 | 85 | 90 | 80 |

| **LNP/Fluc-mRNA** | **L-17** | **L-18** | **L-19** | **L-20** | **L-21** | **L-22** |
|---|---|---|---|---|---|---|
| **Cationic Lipid** | E17-1 | E18-1 | E19-1 | E20-1 | E21-1 | E22-1 |
| **PEGylated Lipid** | DMG | | | | | |
| **Encapsulation Efficiency/%** | 86 | 93 | 87 | 89 | 88 | 94 |
| **Particle Size/nm** | 79 | 86 | 72 | 78 | 77 | 73 |

| **LNP/Fluc-mRNA** | **L-23** | **L-24** | **L-25** | **L-26** | **L-27** | **L-28** |
|---|---|---|---|---|---|---|
| **Cationic Lipid** | E23-1 | E24-1 | E25-1 | E26-1 | E27-1 | E28-1 |
| **PEGylated Lipid** | DMG | | | | | |
| **Encapsulation Efficiency/%** | 88 | 86 | 95 | 96 | 91 | 82 |
| **Particle Size/nm** | 99 | 89 | 73 | 84 | 91 | 100 |

| **LNP/Fluc-mRNA** | **L-29** | **L-30** | **L-31** | **L-32** | **L-33** | **L-34** |
|---|---|---|---|---|---|---|
| **Cationic Lipid** | E29-1 | E30-1 | E31-1 | E32-1 | E33-1 | E34-1 |
| **PEGylated Lipid** | DMG | | | | | |
| **Encapsulation Efficiency/%** | 93 | 90 | 89 | 87 | 99 | 81 |
| **Particle Size/nm** | 77 | 75 | 79 | 90 | 93 | 82 |

| **LNP/Fluc-mRNA** | **L-35** | **L-36** | **L-37** | **L-38** | **L-39** | **L-40** |
|---|---|---|---|---|---|---|
| **Cationic Lipid** | E35-1 | E36-1 | E37-1 | E38-1 | E39-1 | E40-1 |
| **PEGylated Lipid** | DMG | | | | | |
| **Encapsulation Efficiency/%** | 90 | 93 | 91 | 86 | 94 | 99 |
| **Particle Size/nm** | 98 | 89 | 75 | 97 | 78 | 83 |

| **LNP/Fluc-mRNA** | **L-41** | **L-42** | **L-43** | **L-3-2-d** | | |
|---|---|---|---|---|---|---|
| **Cationic Lipid** | E41-1 | E42-1 | E43-1 | E3-2 | | |
| **PEGylated Lipid** | DMG | | | | | |
| **Encapsulation Efficiency/%** | 82 | 85 | 83 | 93 | | |
| **Particle Size/nm** | 80 | 83 | 84 | 88 | | |

| **LNP/Fluc-mRNA** | **PL-0** | **PL-3-2** | **PL-4** | **PL-10-2** | **PL-16** | **PL-17** |
|---|---|---|---|---|---|---|
| **Cationic Lipid** | MC3 | E3-2 | E4-1 | E10-2 | E16-1 | E17-1 |
| **PEGylated Lipid** | E44-1 | | | | | |
| **Encapsulation Efficiency/%** | 94 | 98 | 95 | 96 | 97 | 90 |
| **Particle Size/nm** | 91 | 82 | 91 | 72 | 76 | 75 |

### (2) Evaluation of stability in serum

The above described LNP/Fluc-mRNA (control groups **D-0, D-1, D-2, D-3, D-4;** experimental groups **L-1, L-3-2, L-4, L-10-2, L-16, L-17, PL-0, PL-3-2, PL-4, PL-10-2, PL-16, PL-17**) were respectively added to the culture media containing 10% fetal bovine serum (FBS), stirred at 37°C, and sampled at a regular interval to determine the change of particle size of LNP/Fluc-mRNA to analyze the stability of nucleic acid pharmaceutical formulation in serum. The results (**Table 2**) showed that, in 7 days, **PL-3-2, PL-4, PL-16,** and **PL-10-2** containing both the cationic lipids of the present invention and the PEGylated lipids of the present invention had the smallest changes of particle size (0~3%); whereas, the changes of particle size of the rest experimental groups and the control groups were 5-16%, wherein **D-4, L-1, L-17,** and **PL-17** containing the cationic lipids having carbonate groups or multiple ester groups had relatively bigger changes of particle size. These results indicate that the lipid nanoparticles obtained from the preparation of lipid pharmaceutical compositions containing the cationic lipids of the present invention can have sufficient stability under physiological conditions, and can achieve better efficacy if the PEGylated lipids of the present invention are also used.

**Table 2: Evaluation of the Stability of LNP/Fluc-mRNA in Serum**

| **LNP/Fluc-mRNA** | **D-0** | **D-1** | **D-2** | **D-3** | **D-4** | |
|---|---|---|---|---|---|---|
| **Change of Particle Size/%** | 5 | 7 | 6 | 7 | 16 | |

| **LNP/Fluc-mRNA** | **L-1** | **L3-2** | **L-4** | **L10-2** | **L-16** | **L-17** |
|---|---|---|---|---|---|---|
| **Change of Particle Size/%** | 14 | 8 | 10 | 8 | 7 | 11 |

| **LNP/Fluc-mRNA** | **PL-0** | **PL3-2** | **PL-4** | **PL10-2** | **PL-16** | **PL-17** |
|---|---|---|---|---|---|---|
| **Change of Particle Size/%** | 5 | 0 | 3 | 1 | 0 | 9 |

### (3) Evaluation of cytotoxicity

The cells used were HeLa cells, and the method used was using CCK-8 kit to measure cell viability.

The commercial transfection reagent Lipofectamine 2000 (L2K) was used to prepare the L2K/Fluc-mRNA complex according to the method in **Example 45.**

HeLa cells were inoculated onto a 96-well plate at 1×10⁴ cells/well, 100 µL per well, and then divided into the control group (blank control group), the L2K/Fluc-mRNA group (positive control group) and the LNP/Fluc-mRNA groups (experimental groups in Table 1), and then incubated at 37°C, 5% CO₂. After 24 hours of cell incubation, 3.3 µg/mL L2K/Fluc-mRNA and 3.3 µg/mL LNP/Fluc-mRNA were respectively added to the positive control group and the experimental groups for further incubation at 37°C, 5% CO₂. After another 24 hours of incubation, the 96-well plate was retrieved while protected from light. The culture medium was aspirated, and then a diluted solution of CCK-8 was added 120 µL per well. Then, the incubation was continued for 1-4 hours at 37°C, 5% CO₂.

The 96-well plate was retrieved, and a microplate reader was used to measure the absorbance of each well at a wavelength of 450 nm.

The results of three repeated assays were averaged, and showed that the cell viability of the positive control group L2K/Fluc-mRNA was 92%, those of the experimental groups were all above 91%, and those of **L3-2, L-16, PL-3-2, PL-4,** and **PL-16** were 96% and higher.

These results indicate that, compared with the L2K/Fluc-mRNA which contains the commercial transfection reagent Lipofectamine 2000, the LNP/Fluc-mRNA of the present invention also has no obvious cytotoxicity.

### (4) Evaluation of transfection activity at cell level

**Table 3: Results of Cell Transfection Assays**

| **No.** | **Relative Value of Fluorescence Intensity** | **No.** | **Relative Value of Fluorescence Intensity** | **No.** | **Relative Value of Fluorescence Intensity** |
|---|---|---|---|---|---|
| **Blank** | 1 | **L-1** | 3.8 | **L-12** | 2.9 |
| **D-0** | 2.2 | **L-2** | 3.3 | **L-13** | 2.5 |
| **D-1** | 2.5 | **L-3-2** | 3.5 | **L-14** | 2.8 |
| **D-2** | 3.0 | **L-4** | 3.0 | **L-15** | 2.3 |
| **D-3** | 2.7 | **L-5** | 3.2 | **L-16** | 3.6 |
| **D-4** | 2.6 | **L-6** | 3.1 | **L-17** | 3.1 |
| | | **L-7** | 2.7 | **L-22** | 3.2 |
| | | **L-8** | 3.0 | **L-36** | 3.1 |
| | | **L-9** | 2.5 | **PL-0** | 2.1 |
| | | **L-10-2** | 3.7 | **PL-1** | 3.9 |
| | | **L-11** | 2.4 | **PL-3-2** | 3.7 |

In order to examine the cell-level transfection efficiency of mRNA of each group of LNP/Fluc-mRNA composition prepared in Example 45, assays based on luciferase bioluminescence were performed. The formulation of LNP/Fluc-mRNA composition was dissolved in the culture medium to the required dose. HeLa cells as a cell model were inoculated at a density of 6,000 cells/well. The cell suspension was inoculated at 100 µL/well onto a 96-well black frame plate with clear wells. After the inoculation, the cells were incubated for 24 h in a cell culture incubator. Then, a dose of 0.2 ug mRNA per well was given, while the blank control group was added with free Fluc-mRNA of the corresponding dose. After 24 hours of transfection, the old culture medium was removed and replaced with a new medium containing the D-fluorescein sodium substrate (1.5 mg/mL). After 5 minutes of incubation, bioluminescence was detected using a microplate reader. Stronger fluorescence meant more Fluc-mRNA were transported into the cytoplasm and translated into the corresponding fluorescent protein. The results are shown in Table 3, wherein, the relative value of fluorescence intensity is the ratio of the fluorescence intensity of each group to that of the blank control group. The results indicate that the LNP/Fluc-mRNA compositions prepared in the present invention all have excellent transfection efficiency in vitro, which means the LNPs of the control group and the experimental groups are all effective carriers for delivery and most of the experimental groups have higher transfection efficiency than the control group. When the PEGylated lipids contained are DMG, the transfection efficiency of LNP/Fluc-mRNA compositions is higher when the cationic lipids have azacycle-containing polar head groups such as those in **L-1, L-3-2, L-10-2** and **L-16;** wherein, **L-1** has the highest transfection efficiency, which may due to the multiple ester groups contained that are more conducive to the degradation of cationic lipids in cells which further promotes the release of the nucleic acid drug Fluc-mRNA into the cytoplasm. When the PEGylated lipid in an LNP/Fluc-mRNA composition is **E44-1** of the present invention, the transfection efficiency will not have an obvious compromise (e.g., comparable to the transfection efficiency **of L-0** and **PL-0**), and will be increased to a certain degree when a cationic lipid of the present invention is also used (e.g., **PL-1** and **PL-3-2**).

Those described above are only embodiments of the present invention, not limiting the patent scope of the present invention. Any transformation of equivalent structure or equivalent route based on the specification content of the present invention, directly or indirectly applied in other related arts, should be included in the protected patent scope of the present invention in the same way.

For those skilled in the art, without departing from the purpose and the scope of the present invention, and without unnecessary experimentation, the present invention can be implemented in a wider range with equivalent parameters, concentrations, and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A cationic lipid of the general formula (1): 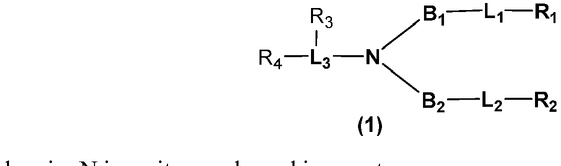
wherein, N is a nitrogen rancing center;
L₁ and L₂ are each independently a linking bond or a divalent linking group;
L₃ is a trivalent linking group; L₃ and R₃ form a divalent linking group -L₃(R₃)- with R₃ as the side chain;
B₁ and B₂ are each independently a linking bond or a C₁₋₃₀ alkylene group;
R₁ and R₂ are each independently a C₂₋₃₀ aliphatic hydrocarbon group or a C₂₋₃₀ residue of aliphatic hydrocarbon derivative containing one or two oxygen atoms;
R₃ is selected from the group consisting of -OR_{d}, -NR_{d}R_{d}, -SR_{d}, -C(=O)R_{d}, -C(=O)OR_{d}, -OC(=O)R_{d}, -OC(=O)OR_{d}, and 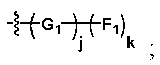 wherein, R_{d} is, at each occurrence, independently a C₁₋₁₂ alkyl group; G₁ is a branching group having a valence of k+1; j is 0 or 1; F₁ contains the functional group R₀₁; when j is 0, G₁ is absent; when j is 1, G₁ is connected to k F₁, and k F₁ are each independently of the same or different structures, wherein k is an integer in the range of 2 to 8;
R₄ is selected from the group consisting of a carbocyclyl group, a heterocyclyl group, and R'N(R')-R"-N(R')-; wherein, a heterocyclyl group is a cyclic group having 1, 2, or more heteroatoms in its ring atoms, said heteroatom being B, O, N, Si, P, or S; wherein, R' is, at each occurrence, independently H or a C₁₋₃ alkyl group; R" is a C₂₋₄ alkylene group;
said alkylene group, aliphatic hydrocarbon group, residue of aliphatic hydrocarbon derivative, carbocyclyl group, and heterocyclyl group are each independently substituted or unsubstituted;
or a salt, tautomer, stereoisomer, or solvate thereof.

2. The cationic lipid of claim **1,** wherein said L₁ and L₂ belong to any of the following cases:
Case (1): one of L₁ and L₂ is a linking bond, and the other is a divalent linking group;
Case (2): both L₁ and L₂ are linking bonds;
Case (3): both L₁ and L₂ are divalent linking groups, and L₁ and L₂ are of the same or different structures;
in any said case, the divalent linking group is selected from the group consisting of -O-, -S-, -S-S-, -OC(=O)-, -C(=O)O-, -OC(=O)O-, -C(=O)-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, -C(R_{c})=N-NR_{c}-, -NR_{c}-N=C(R_{c})-, and a combination of any two thereof and a C₁₋₁₀ alkylene group; wherein, R_{c} is, at each occurrence, independently H or methyl, preferably H;
preferably, L₁ and L₂ are each independently selected from the group consisting of -O-, -OC(=O)-, -C(=O)O-, -OC(=O)O-, -C(=O)-, -NHC(=O)-, -C(=O)NH-, -C(=O)O(CH₂)_{y}OC(=O)-, -OC(=O)(CH₂)_{y}OC(=O)-, -C(=O)O(CH₂)_{y}C(=O)O-, and -OC(=O)(CH₂)_{y}C(=O)O-, wherein y is an integer in the range of 2 to 8.

3. The cationic lipid of claim **1,** wherein said -L₃(R₃)- is selected from the group consisting of -L₄-, -Z-L₄-, -L₅-Z-L₄-, -L₄-Z-L₅-, -Z-L₄-Z-L₅-, -L₅-Z-L₄-Z-L₅-, -L₄-Z-L₅-Z-L₅-, -Z-L₅-Z-L₄-, and -L₅-Z-L₅-Z-L₄-, and its right end is connected to the nitrogen branching center; wherein, L₄ is -(CH₂)ₓ-CR₃H-(CH₂)ₓ-, L₅ is -(CH₂)ₓ-, and x is, at each occurrence, independently an integer in the range of 0 to 3; wherein, Z is, at each occurrence, independently selected from the group consisting of -C(=O)-, -OC(=O)-, -C(=O)O-, -OC(=O)O-, -O-, -S-, -S-S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; and wherein, R_{c} is, at each occurrence, independently H or methyl, preferably H;
-L₃(R₃)- is preferably -(CH₂)ₓ-CR₃H-(CH₂)ₓ-.

4. The cationic lipid of claim **3,** wherein the structure of said -L₃(R₃)- is 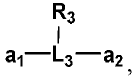 wherein a₁ is a linking bond connected to R₄, and a₂ is a linking bond connected to the nitrogen branching center of the general formula (1); said 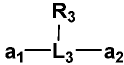 is preferably selected from the group consisting of the following structures:

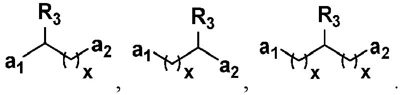

5. The cationic lipid of claim **4,** wherein said 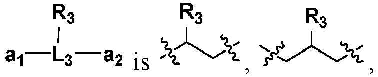 or 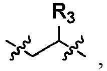 preferably

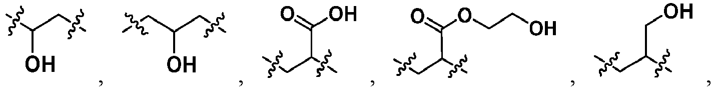

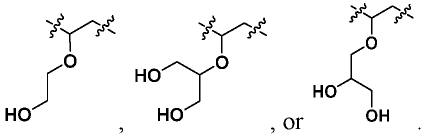

6. The cationic lipid of claim **1,** wherein said B₁ and B₂ are each independently a linking bond or C₁₋₂₀ alkylene group, preferably belonging to any of the following cases:
Case (1): one of B₁ and B₂ is a linking bond, and the other is a C₁₋₂₀ alkylene group;
Case (2): both B₁ and B₂ are linking bonds;
Case (3): B₁ and B₂ are each independently a C₁₋₂₀ alkylene group;
said C₁₋₂₀ alkylene group is substituted or unsubstituted, and is selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, an eicosylene group, a divalent linking group consisting of 1 -CR_{q}H- and 0 to 19 -CH₂-, and a divalent linking group consisting of 2 -CR_{q}H- and 0 to 18 -CH₂-; said R_{q} is, at each occurrence, independently selected from the group consisting of -OH, -(CH₂)_{tq}OH, -(CH₂)_{tq}CH₃, -(CH₂)_{tq}OCH₃, -SH, -(CH₂)_{tq}SH, -(CH₂)_{tq}SCH₃, -NH₂, -N((CH₂)_{tq}CH₃)₂, -(CH₂)_{tq}NH₂, and -(CH₂)_{tq}N(CH₃)₂, wherein tq is an integer in the range of 0 to 4; R_{q} is preferably -OH.

7. The cationic lipid of claim **6,** wherein said B₁ and B₂ are each independently selected from the group consisting of a linking bond, an ethylene group, a propylene group, a butylene group, a pentylene group, a hexylene group, a heptylene group, an octylene group, a nonylene group, a decylene group, a -OH substituted butylene group, a -OH substituted pentylene group, a -OH substituted hexylene group, a -OH substituted heptylene group, and a -OH substituted octylene group;
it is preferred that both B₁ and B₂ are ethylene groups, both B₁ and B₂ are butylene groups, both B₁ and B₂ are hexylene groups, both B₁ and B₂ are heptylene groups, B₁ is a butylene group while B₂ is a heptylene group, B₁ is a pentylene group while B₂ is a hexylene group, B₁ is a pentylene group while B₂ is a heptylene group, B₁ is a linking bond while B₂ is a hexylene group, B₁ is a pentylene group while B₂ is a -OH substituted hexylene group, both B₁ and B₂ are -OH substituted hexylene groups, or B₁ is a hexylene group while B₂ is a -OH substituted hexylene group.

8. The cationic lipid of claim **1,** wherein said R₁ and R₂ are each independently R_{L}, R_{B}, or Rr; said R_{L}, R_{B}, and Rr each independently contains 0 to 4 Rₘ substituents; said Rₘ is, at each occurrence, independently a linear C₁₋₁₂ hydrocarbon group or a branched C₁₋₃ alkyl group, preferably a methyl group;
said R_{L} is a linear C₂₋₃₀ aliphatic hydrocarbon group containing 0 to 4 carbon-carbon double bonds, preferably any of the following structures or any *cis-*/*trans-* isomer thereof: 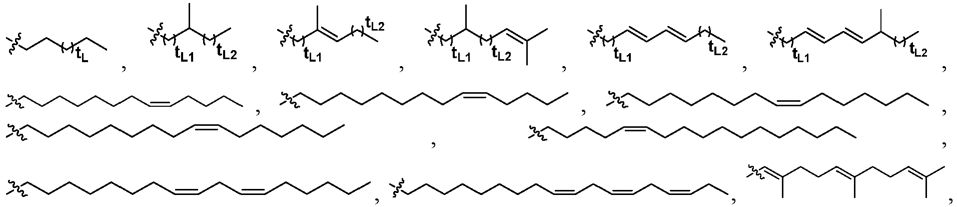 and 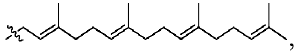 wherein t_{L} is an integer in the range of 0 to 20, and wherein t_{L1} and t_{L2} are each independently an integer in the range of 2 to 10;
said R_{B} has the structure of 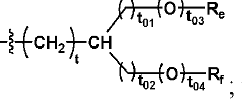 wherein, t is 0, 1, or 2, and t₀₁, t₀₂, t₀₃, and t₀₄ are each independently 0 or 1; Rₑ and R_{f} are each independently selected from the group consisting of C₁₋₁₂ alkyl, C₃₋₁₂ alkenyl, and C₃₋₁₂ alkynyl; R_{B} is preferably selected from the group consisting of the following structures: 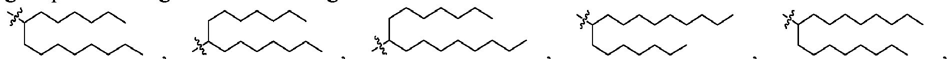 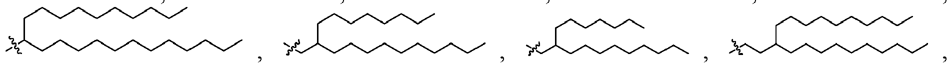 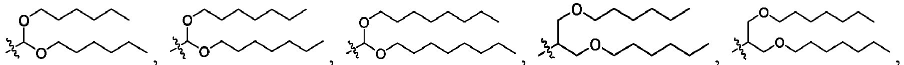 and 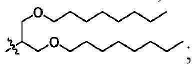
said Rr is a C₃₋₃₀ aliphatic hydrocarbon group containing cyclic structures; said cyclic structure is a C₃₋₂₀ carbocycle, preferably a benzene ring or a residue of C₃₋₂₀ cycloalkane; Rr is preferably selected from the group consisting of the following structures: 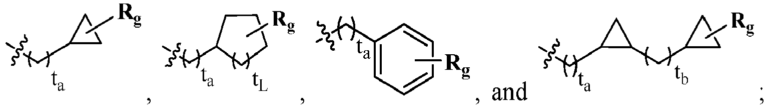 wherein, tₐ is, at each occurrence, independently an integer in the range of 0 to 12, t_{b} is an integer in the range of 1 to 12, t_{L} is an integer in the range of 0 to 20, and R_{g} is, at each occurrence, independently a substituent at any position on the ring, said substituent being selected from the group consisting of H, C₁₋₁₂ alkyl, C₂₋₁₂ alkenyl, and C₂₋₁₂ alkynyl;
Rr is more preferably selected from the group consisting of the following structures:

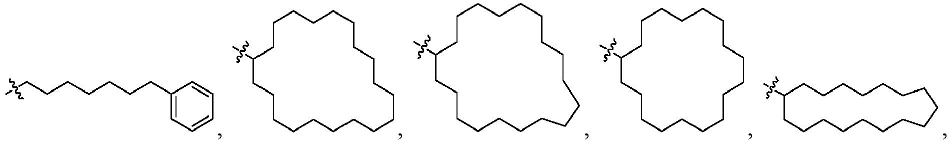

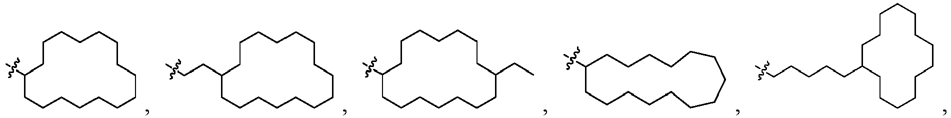

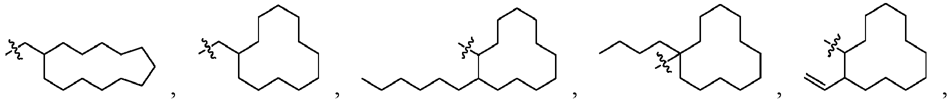

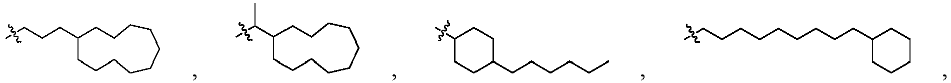

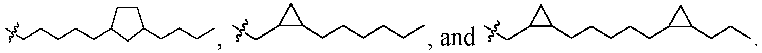

9. The cationic lipid of claim **1,** wherein said R₃ is selected from the group consisting of -O(CH₂)ᵣCH₃, -S(CH₂)ᵣCH₃, -C(=O)(CH₂)ᵣCH₃, -C(=O)O(CH₂)ᵣCH₃, -OC(=O)(CH₂)ᵣCH₃, -OC(=O)O(CH₂)ᵣCH₃, and -(CH₂)ᵣN(CH₃)₂, wherein r is an integer in the range of 0 to 3 and preferably 0 or 1.

10. The cationic lipid of claim **1,** wherein said R₃ is 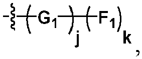 and F₁ has the structure of -(CH₂)ₕ-R₀₁ or -(CH₂)_{f}-Z₃-(CH₂)_{g}-R₀₁; wherein, h is an integer in the range of 0 to 6, f is 0, 1, or 2, and g is 2, 3, or 4; wherein, Z₃ is selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; and wherein, R_{c} is, at each occurrence, independently H or methyl, preferably H;
F₁ is preferably selected from the group consisting of -R₀₁, -CH₂-R₀₁, -(CH₂)₂-R₀₁, -O-(CH₂)₂-R₀₁, -C(=O)O-(CH₂)₂-R₀₁, -OC(=O)-(CH₂)₂-R₀₁, and -OC(=O)O-(CH₂)₂-R₀₁.

11. The cationic lipid of claim **1,** wherein said j is 1, R₃ is 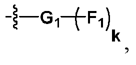 and G₁ has the structure of -(CH₂)_{f}-(Z₀)_{q}-(CH₂)_{f}-G₀₁; wherein, q is 0 or 1; wherein, f is, at each occurrence, independently 0, 1, or 2; wherein, G₀₁ is a trivalent or quadrivalent branching core selected from the group consisting of -OCH<, -N<, and 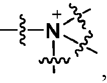 and its left end is connected to -(CH₂)_{f}-; wherein, Z₀ is Z₁, Z₂, or -(Z₂)_{q}-(CH₂)_{g}-(Z₁)_{q}-, wherein Z₁ and Z₂ are each independently selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is, at each occurrence, independently H or methyl, preferably H; and wherein, g is 2, 3, or 4;
G₁ is preferably -(CH₂)_{f}O(CH₂)_{f}CH<, more preferably -OCH₂CH<.

12. The cationic lipid of claim **1,** wherein said R₄ is R'N(R')-R"-N(R')-, selected from the group consisting of 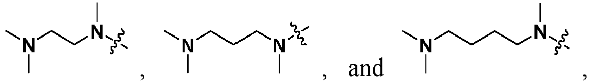 preferably

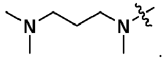

13. The cationic lipid of claim **1,** wherein said R₄ is a carbocyclyl or heterocyclyl group containing 0 to 5 substituents; said substituents are each independently selected from the group consisting of -F, -Cl, -Br, -I, -R_{d}', -OR_{d}', -NR_{d}'R_{d}', -SR_{d}', -C(=O)R_{d}', -C(=O)OR_{d}', -OC(=O)R_{d}', and -OC(=O)OR_{d}', wherein R_{d}' is a C₁₋₃ alkyl group;
R₄ is preferably selected from the group consisting of the following structures: 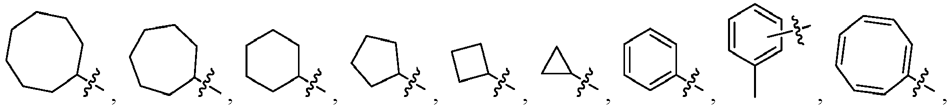 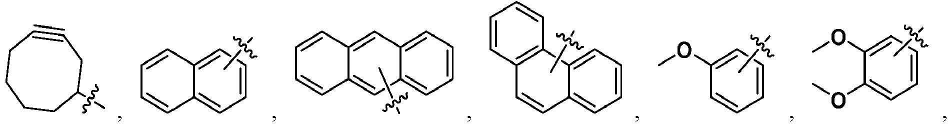 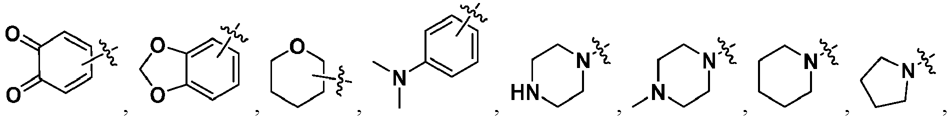 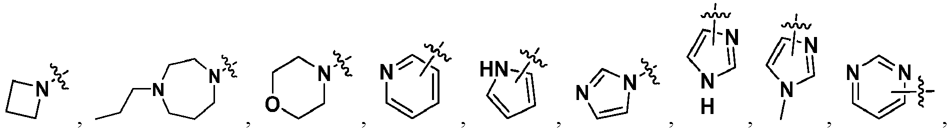 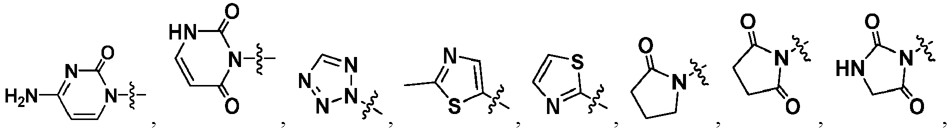 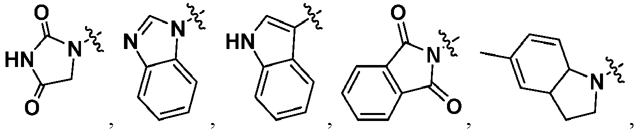
and more preferably selected from the group consisting of the following structures:

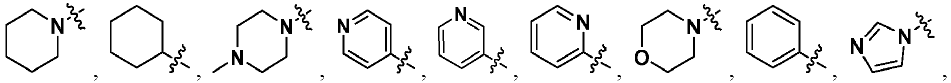

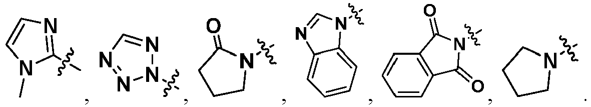

14. The cationic lipid of claim **1**, wherein said R₀₁ is selected from the group consisting of functional groups, modified forms of functional groups, therapeutically targeting functional groups, and fluorescent functional groups; wherein, said modified forms are selected from the group consisting of the precursors of functional groups, the active forms to which functional groups are precursors, the active forms in which functional groups are substituted, and the inactive forms in which functional groups are protected; wherein, said precursors of functional groups refer to the structures which can be transformed into said functional groups through at least one process among oxidation, reduction, hydration, dehydration, electronic rearrangement, structural rearrangement, salt complexation and decomplexation, ionization, protonation, and deprotonation.

15. The cationic lipid of claim **14**, wherein R₀₁ is selected from the group consisting of the functional groups in the following classes A~I and modified forms thereof:
Class A: active ester group, analogous structure of active ester group; wherein, said active ester group is selected from the group consisting of a succinimidyl active ester group, a *p*-nitrophenyl active ester group, an *o*-nitrophenyl active ester group, a 1,3,5-trifluorophenyl active ester group, a 1,3,5-trichlorophenyl active ester group, a 1,3,5-tribromophenyl active ester group, a 1,3,5-triiodophenyl active ester group, a pentafluorophenyl active ester group, an imidazole active ester group, a benzotriazole active ester group, a thiazolidine-2-thione active ester group, a pyrrolidine-2-thione active ester group, a 2-mercaptobenzothiazole active ester group, and a 1-oxo-3-thioxoisoindoline active ester group; and wherein, said analogous structure of active ester group is an active carboxylate group or active acyl group;
Class B: carboxyl group, protected carboxyl group, sulfonic acid group, sulfonate group, sulfinic acid group, sulfinate group, sulfenic acid group, ester group, thioester group, dithioester group, carbonate group, thiocarbonate group, dithiocarbonate group, trithiocarbonate group, xanthate group, tetrathiodiester group, sulfone group, sulfoxide group, methacryloyl group, hydroxamic acid group, thiohydroxamic acid group, sulfonyl halide group, thiocarboxy group;
Class C: aldehyde group, hydrated aldehyde group, thioaldehyde group, acyl halide group, ketone group, hydrated ketone group, thione group, hydrated thione group, glyoxal group, acetal group, monothioacetal group, dithioacetal group, ketal group, monothioketal group, dithioketal group, hemiacetal group, thiohemiacetal group, hemiketal group, orthoacid group, protected orthoacid group, orthoester group, cyanate group, thiocyanate group, isocyanate group, isothiocyanate group, oxazoline group, isoxazoline group;
Class D: primary amino group, secondary amino group, protected amino group, hydroxylamine group, thiol group, disulfide group, halogen atom, haloacetamide group, ammonium salt, hydrazino group, tetramethylpiperidinyloxy group, dioxapiperidinyloxy group, O-carbonyl hydroxylamine group, amide group, imide group, hydrazide group, sulfonyl hydrazide group, hydrazone group, imine group, enamino group, alkynylamine group, carbamate group, thiocarbamate group, dithiocarbamate group;
Class E: urea group, thiourea group, guanidino group and its protonated form, amidine group and its protonated form, anhydride group, squaric acid group, squarate group, semi-squaric acid group, semi-squarate group, imidazole-1-carboxamide group, imidate group, nitrone group, aldoxime group, ketoxime group;
Class F: maleimide group, furan-protected maleimide group, acrylate group, *N*-acrylamide group, *N*-methacrylamide group, methacrylate group, maleamic acid group, 1,2,4-triazoline-3,5-dione group, linear azo compound group, cyclic azo compound group;
Class G: alkenyl group, alkenylhydrocarbon group, cycloalkenyl group, alkynyl group, alkynylhydrocarbon group, protected alkynyl group, cycloalkynl group, linear conjugated diene group, cyclic conjugated diene group, heteroatom-containing cyclic conjugated diene group, epoxy group, 1,2,4,5-tetrazine group, azido group, nitrile oxide group, cyano group, isocyano group, diazo group, diazonium ion , azo oxide group, nitrilimine group, aldimine *N*-oxide group, tetrazolyl group, 4-acetyl-2-methoxy-5-nitrophenoxy group and its diazotized form, imidazole group, indolyl group; wherein, the cycloalkenyl group is selected from the group consisting of a cyclooctenyl group, a norbornenyl group, a norbornadienyl group, an oxa norbornenyl group, and an oxa norbornadienyl group;
Class H: hydroxyl group, protected hydroxyl group, protected dihydroxyl group, siloxy group, trihydroxysilyl group, protected trihydroxysilyl group; wherein, the hydroxyl group is selected from the group consisting of an alcoholic hydroxyl group, a phenolic hydroxyl group, an enolic hydroxyl group, and a hemiacetal hydroxyl group;
Class I: monosaccharide group, selected from the group consisting of the residues of allose, altrose, arabinose, cladinose, erythrose, erythrulose, fructose, fucitol, fucosamine, fucose, fuculose, galactosamine, galactosaminitol, *N-*acetyl-galactosamine, galactose, glucosamine, *N*-acetyl-glucosamine, glucosaminitol, glucose, glucose-6-phosphate, gulose glyceraldehyde, *L*-glycero-*D*-manno-heptose, glycerol, glyceraldehyde, dihydroxyacetone, gulose, idose, lyxose, mannosamine, mannose, mannose-6-phosphate, mannoheptulose, allulose, quinolose, quinosamine, rhamnitol, rhamnosamine, rhamnose, ribose, ribulose, deoxyribose, sedoheptulose, sorbose, tagatose, talose, tartaric acid, threose, xylose, xylulose, and functional derivatives thereof; said monosaccharide group is in *D*-configuration or *L*-configuration, cyclic or linear, substituted or unsubstituted.

16. The cationic lipid of claim **14**, wherein said R₀₁ is selected from the group consisting of the functional groups of the following classes A~I and modified forms thereof:
Class A: 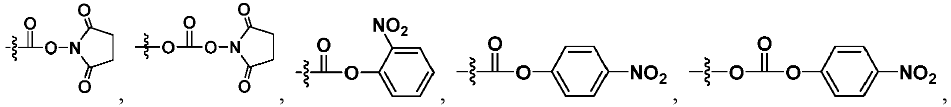 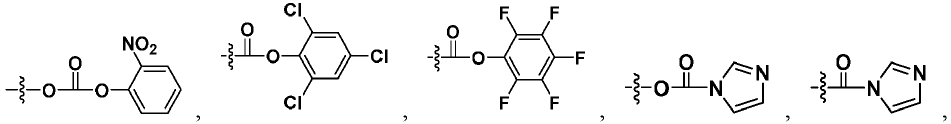 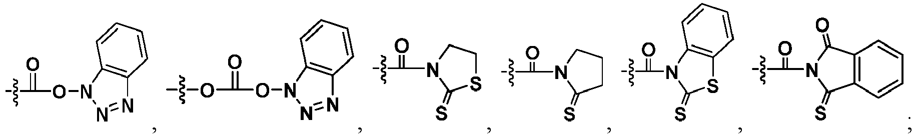
Class B: 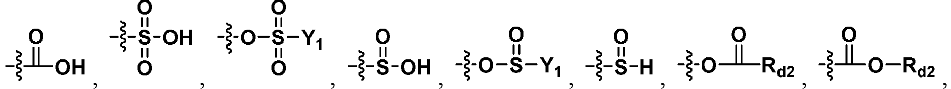 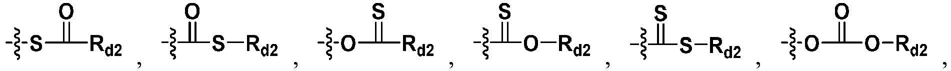 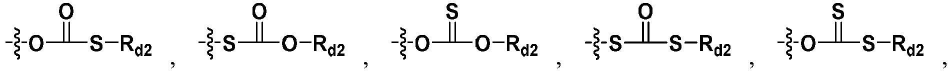 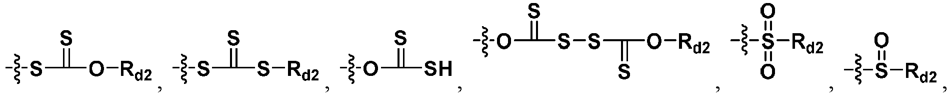 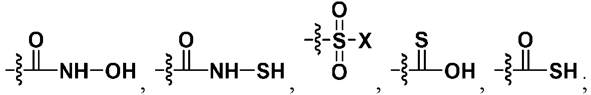
Class C: 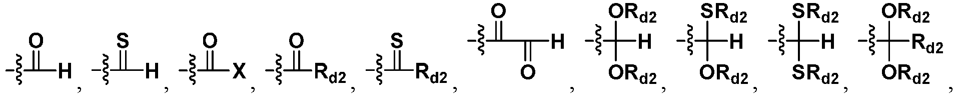 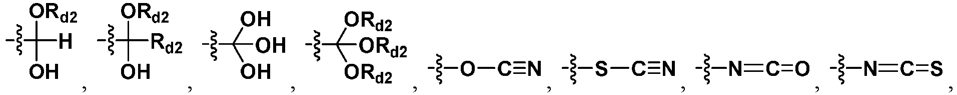 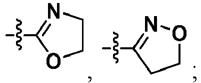
Class D: 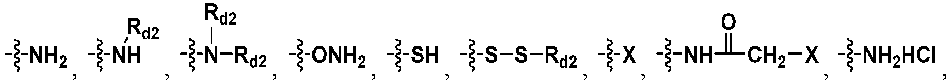 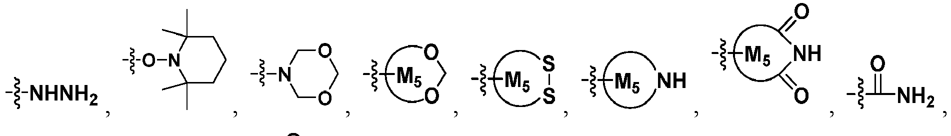 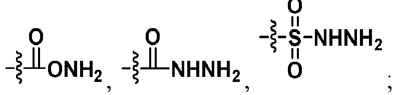
Class E: 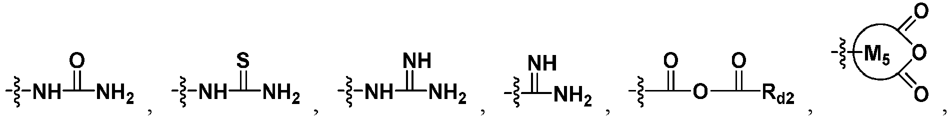 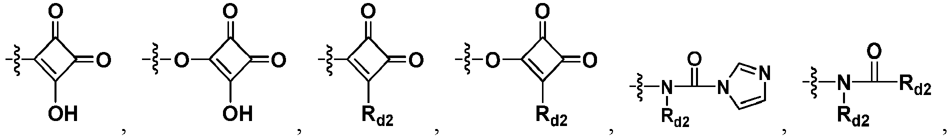 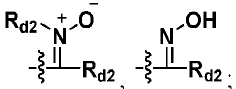
Class F: 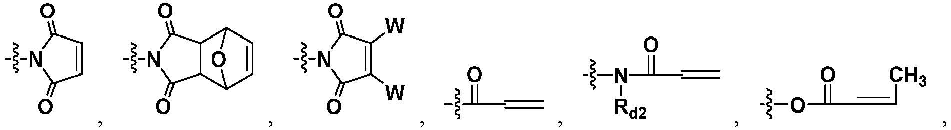 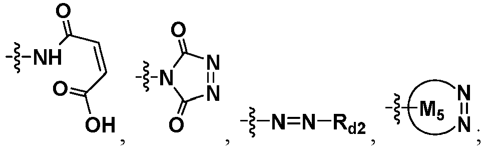
Class G: 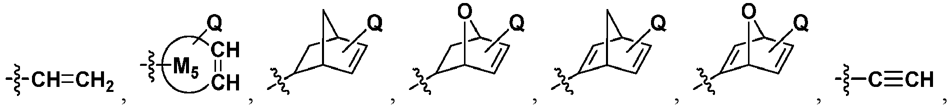 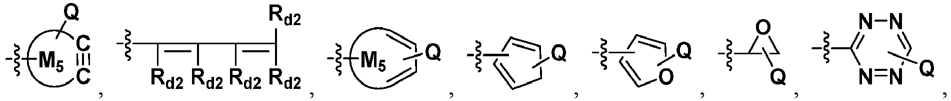 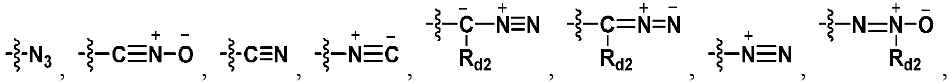 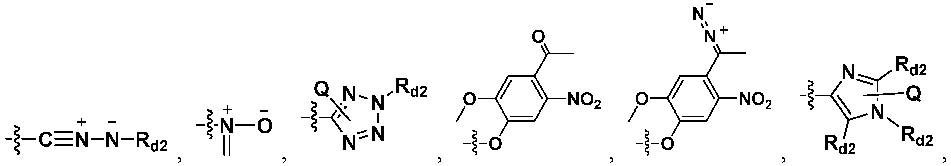 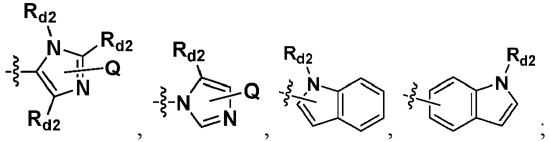
Class H: 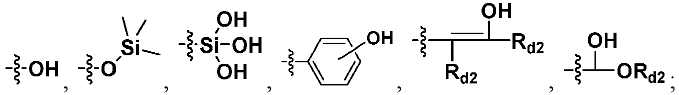
Class I: 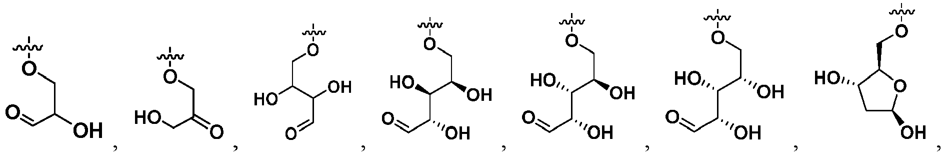 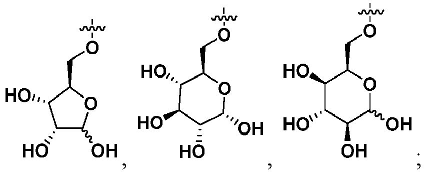
wherein, X is a halogen atom selected from the group consisting of a fluorine atom, a chlorine atom, a bromine atom, and an iodine atom;
wherein, Y₁ is selected from the group consisting of C₁₋₅ alkyl, vinyl, phenyl, benzyl, p-methylphenyl, 4-(trifluoromethoxy)phenyl, trifluoromethyl, and 2,2,2-trifluoroethyl groups;
wherein, R_{d2} is an organic group and is preferably, at each occurrence, independently selected from the group consisting of a C₁₋₅ alkyl group, a C₂₋₅ alkenyl group, a C₂₋₅ alkynyl group, and a phenyl group; said alkyl group, alkenyl group, alkynyl group and phenyl group are each independently substituted or unsubstituted;
wherein, W is a leaving group selected from the group consisting of -F, -Cl, -Br, -I, and -SPh;
wherein, M₅ is a ring atom selected from the group consisting of a carbon atom, a nitrogen atom, a phosphorus atom, and a silicon atom; the cyclic structure containing M₅ is a 3 to 30 membered ring, preferably a 3 to 20 membered ring, more preferably a 3 to 16 membered ring, and more preferably a 5 to16 membered ring; said cyclic structure is preferably selected from the group consisting of cyclohexane, furanose ring, pyranose ring, benzene, tetrahydrofuran, pyrrolidine, thiazolidine, cyclohexene, tetrahydropyran, piperidine, 1,4-dioxane, pyridine, pyridazine, pyrimidine, pyrazine, 1,3,5-triazine, 1,4,7-triazacyclononane, cyclotripeptide, indene, indane, indole, isoindole, purine, naphthalene, dihydroanthracene, xanthene, thioxanthene, dihydrophenanthrene, 10,11 -dihydro-5H-dibenzo[a,d]cycloheptane, dibenzocycloheptene, 5-dibenzosuberenone, quinoline, isoquinoline, fluorene, carbazole, iminodibenzyl, acenaphthene, dibenzocyclooctyne, aza-dibenzocyclooctyne, substituted forms thereof, and heterosubstituted forms thereof;
wherein, 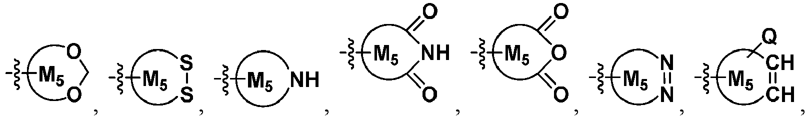 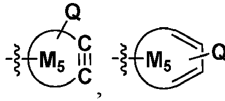 are cyclic structures of which the ring skeletons contain an acetal group, a disulfide bond, an amine group, an imide group, an anhydride group, an azo group, a carbon-carbon double bond, a carbon-carbon triple bond, and a conjugated diene, respectively; said cyclic structure is selected from the group consisting of a carbocycle, a heterocycle, a benzoheterocycle, a substituted carbocycle, a substituted heterocycle, and a substituted benzoheterocycle;
wherein, Q is an atom or substituent that promotes the inductive or conjugate effect of electrons of unsaturated bonds; when Q is connected to the ring, the number of Q may be one or greater; when said number is greater than one, the structures of Q may be the same or a combination of two or more different structures; when Q is a substituent, the structure of which is linear, branched with a pendant group, or ring-containing.

17. The cationic lipid of claim **14**, wherein said R₀₁ is selected from the group consisting of hydroxyl, carboxyl, aldehyde, amino, and thiol groups, and protected forms thereof; or, from the group consisting of a halogen atom, an ester group, a sulfonate group, and an active ester group; R₀₁ is preferably selected from the group consisting of -OH, -COOH, and -C(=O)OCH₃.

18. The cationic lipid of claim **1,** which is selected from the group consisting of the following structures:

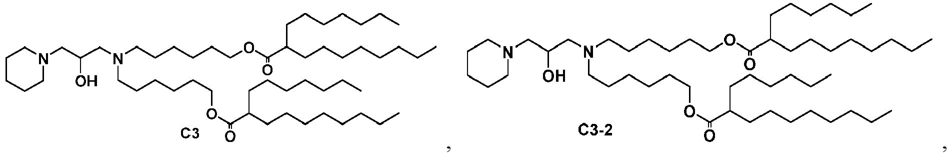

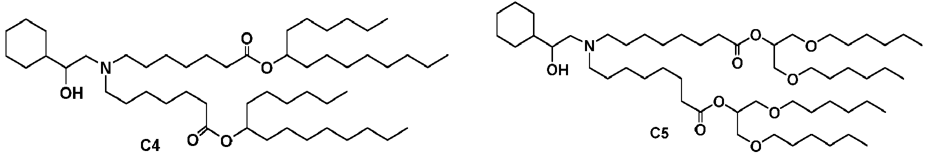

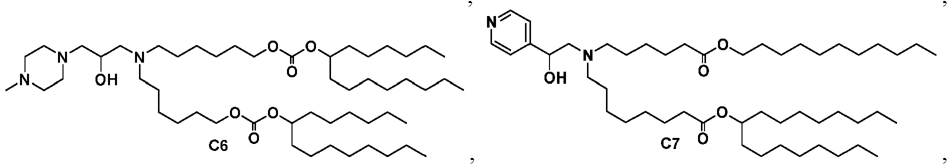

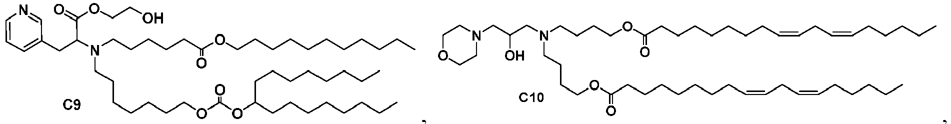

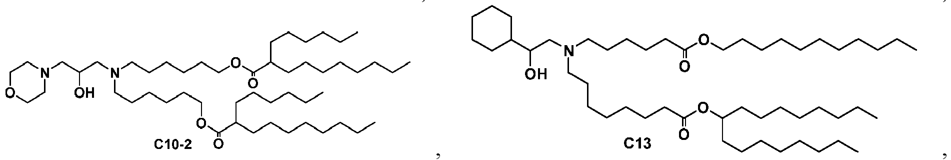

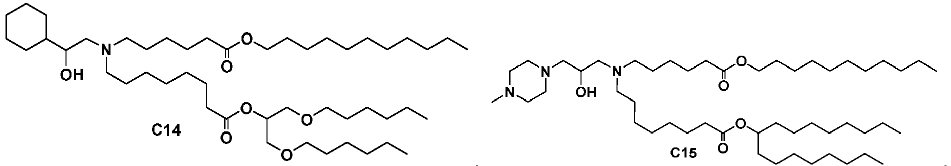

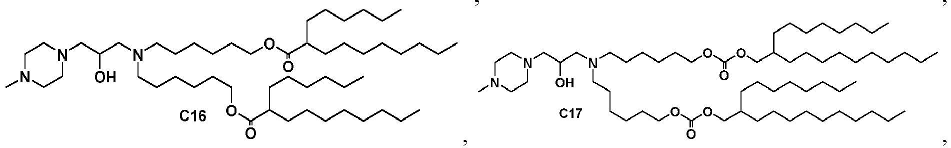

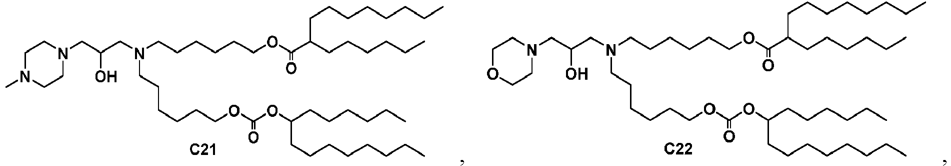

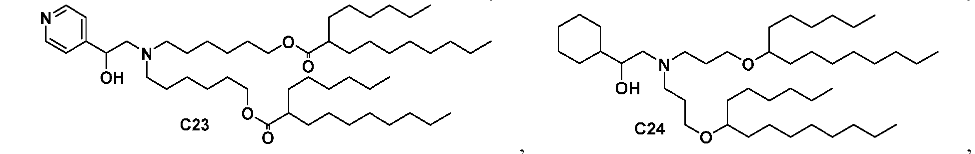

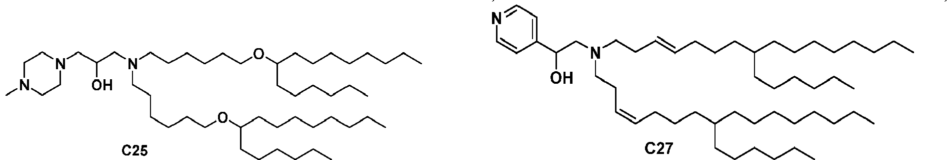

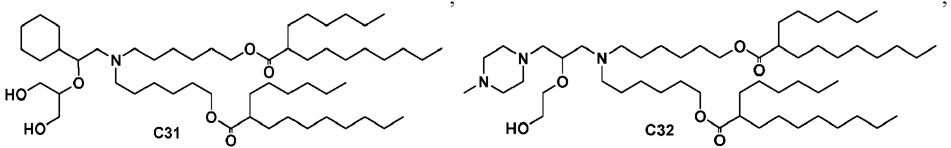

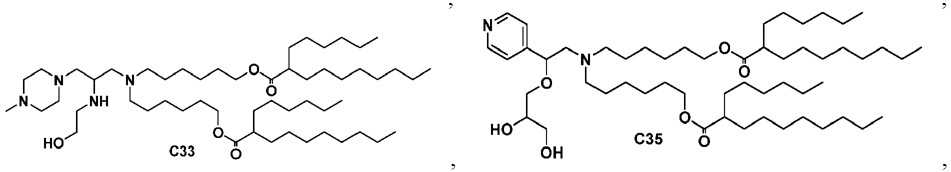

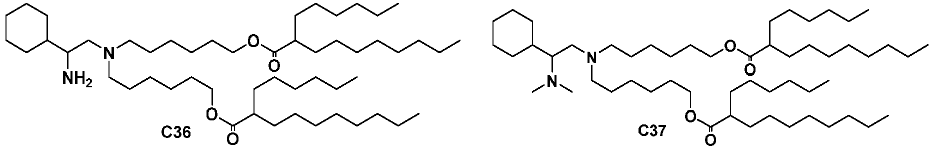

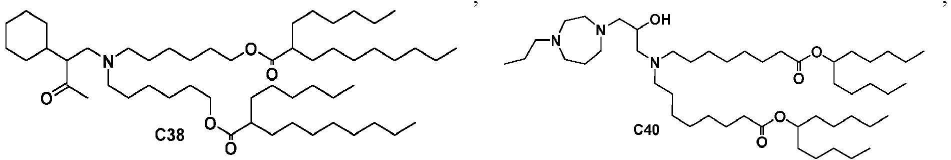

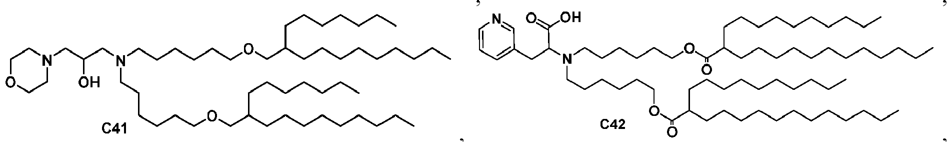

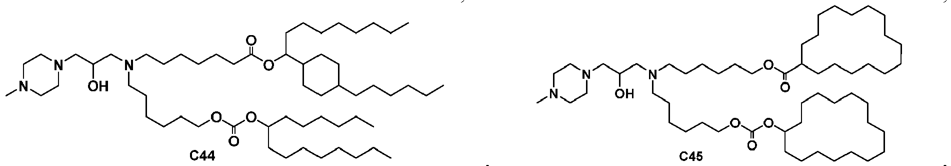

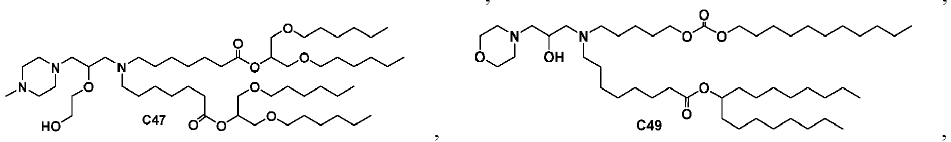

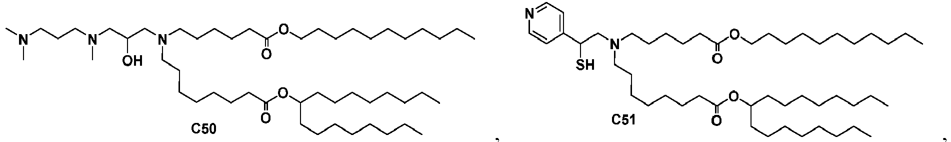

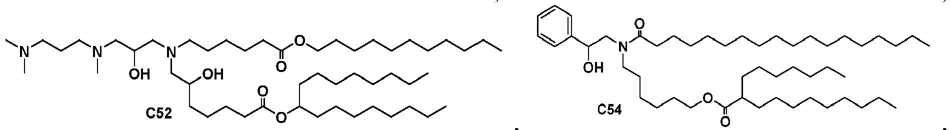

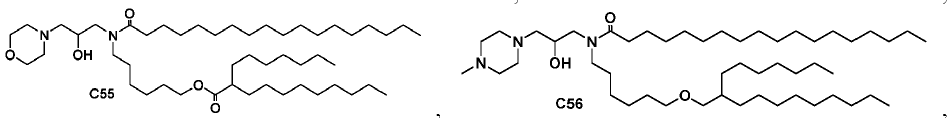

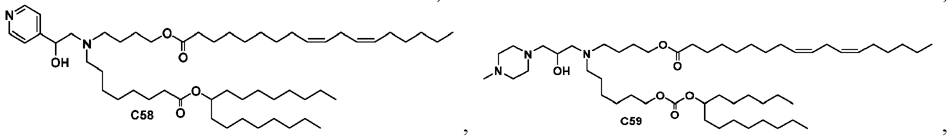

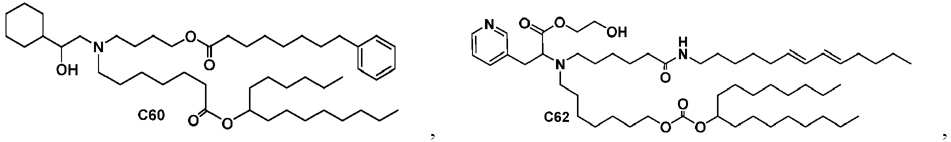

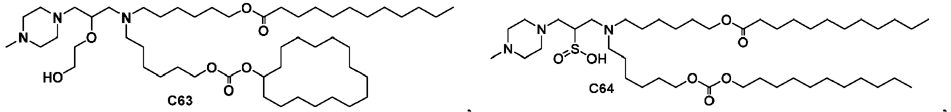

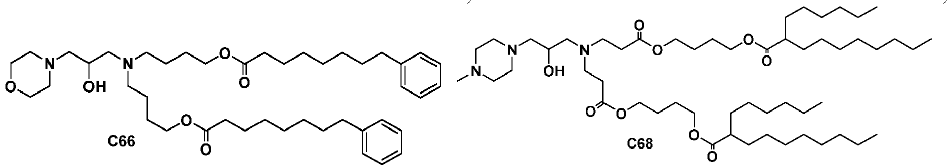

19. A PEGylated lipid derived from the cationic lipid of claim **1,** the structure of which is represented by the general formula (2): 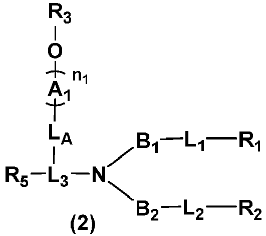
wherein, N is the nitrogen branching center;
L₁ and L₂ are each independently a linking bond or a divalent linking group;
L₃ is a trivalent linking group; L₃ and -L_{A}(A₁)ₙ₁OR₃ form a divalent linking group -L₃[L_{A}(A₁)ₙ₁OR₃]- with -L_{A}(A₁)ₙ₁OR₃ as the side chain;
B₁ and B₂ are each independently a linking bond or a C₁₋₃₀ alkylene group;
R₁ and R₂ are each independently a C₂₋₃₀ aliphatic hydrocarbon group or a C₂₋₃₀ residue of aliphatic hydrocarbon derivative containing one or two oxygen atoms;
R₃ is selected from the group consisting of H, -R_{d}, -(CH₂)ₕNR_{d}R_{d}, -(CH₂)ₕSR_{d}, -C(=O)R_{d}, -C(=O)OR_{d}, and 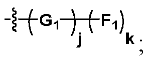 wherein, R_{d} is, at each occurrence, independently a C₁₋₁₂ alkyl group; h is an integer in the range of 0 to 6; G₁ is a branching group having a valence of k+1; j is 0 or 1; F₁ contains the functional group R₀₁; when j is 0, G₁ is absent; when j is 1, G₁ is connected to k F₁, and k F₁ are each independently of the same or different structures, wherein k is an integer from 2 to 8;
R₅ is selected from the group consisting of a C₃₋₁₄ alkyl group, a carbocyclyl group, a heterocyclyl group, and R'N(R')-R"-N(R')-; wherein, a heterocyclyl group is a cyclic group having 1, 2, or more heteroatoms in its ring atoms, said heteroatom being B, O, N, Si, P, or S; wherein, R' is, at each occurrence, independently H or a C₁₋₃ alkyl group; R" is a C₂₋₄ alkylene group;
L_{A} is -(CH₂)ₕ- or -(CH₂)_{f}-Z₃-(CH₂)_{g}-; wherein, h is an integer in the range of 0 to 6, f is 0, 1, or 2, and g is 2, 3, or 4; wherein, Z is selected from the group consisting of -C(=O)-, -OC(=O)-, -C(=O)O-, -OC(=O)O-, -O-, -S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is, at each occurrence, independently H or methyl, preferably H;
A₁ is -OCH₂CH₂-, and is connected to L_{A} by its left end;
n₁ is the number of repetitions of A₁, and is an integer in the range of 20 to 250;
said alkyl group, alkylene group, aliphatic hydrocarbon group, residue of aliphatic hydrocarbon derivative, carbocyclyl group, and heterocyclyl group are each independently substituted or unsubstituted.

20. The PEGylated lipid of claim **19,** which is selected from the group consisting of the following structures:

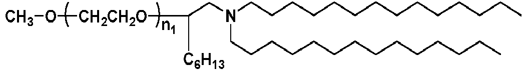

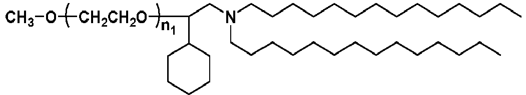

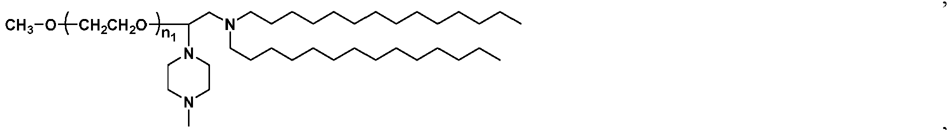

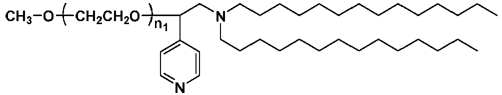

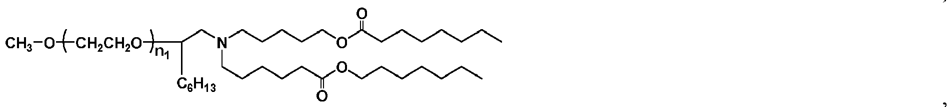

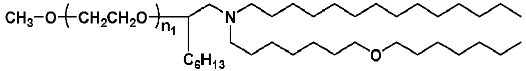

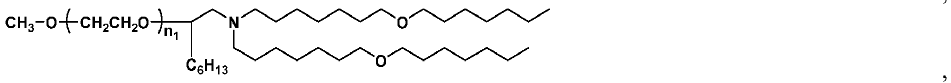

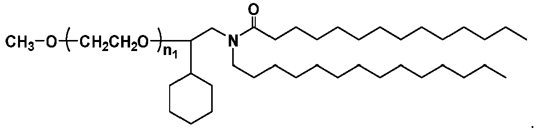

21. A lipid composition containing a cationic lipid of any one of claims **1** to **18.**

22. The lipid composition of claim **21,** which contains also one or more types of lipids selected from the group consisting of phospholipid, steroid lipid, and PEGylated lipid, belonging to any of the following cases:
Case (1): contains also a phospholipid;
Case (2): contains also a steroid lipid;
Case (3): contains also a PEGylated lipid;
Case (4): contains also a phospholipid and a steroid lipid;
Case (5): contains also a phospholipid and a PEGylated lipid;
Case (6): contains also a steroid lipid and a PEGylated lipid;
Case (7): contains also a phospholipid, a steroid lipid, and a PEGylated lipid;
and preferably contains also three types of lipids consisting of phospholipid, steroid lipid, and PEGylated lipid, simultaneously.

23. The lipid composition of claim **22,** wherein said phospholipid is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3 -phosphocholine, 1,2-distearoyl-sn-glycero-3-phosphatidylcholine, 1,2-diundecanoyl-sn-glycero-3 -phosphatidylcholine, 1-plamitoyl-2-oleoyl-sn-glycero-3 -phosphocholine, 1,2-di-*O*-octadecenyl-sn-glycero-3-phosphatidylcholine, 1-oleoyl-2-cholesterylhemisuccinyl-sn-glycero-3-phosphocholine, 1-*O*-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3 -phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3 -phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3 -phosphoethanolamine, 1,2-dioleoyl-sn-glycero-3-phospho-rac-(1-glycerol) sodium salt, dioleoyl phosphatidylserine, dipalmitoylphosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof.

24. The lipid composition of claim **22,** wherein said steroid lipid is selected from the group consisting of cholesterol, coprostanol, sitosterol, ergosterol, campesterol, stigmasterol, brassicasterol tomatidine, ursolic acid, α-tocopherol, and mixtures thereof.

25. The lipid composition of claim **22,** wherein said PEGylated lipid is selected from the group consisting of 1,2-dimyristoyl-sn-glycerol-methoxypolyethylene glycol, 1,2-distearoyl-sn-glycero-3-phosphoethanolamine-*N*-[amino(polyethylene glycol)], PEG-cholesterol, polyethylenglycol-diacylgricerol conjugate, and polytheylyene-dialkyloxypropyl conjugate, and specifically the group consisting of PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, and PEG2000-2,3-distearoylglycerol.

26. The lipid composition of claim **22,** wherein said PEGylated lipid is selected from PEGylated lipids of any one of claims **19** to **20.**

27. The lipid composition of claim **22,** wherein the structure of the PEGylated lipid is selected from the group consisting of the following: 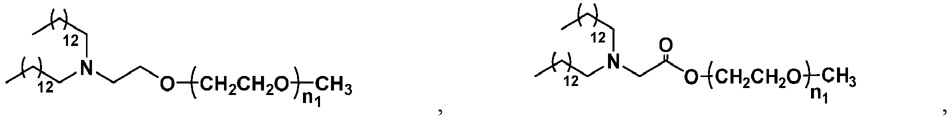 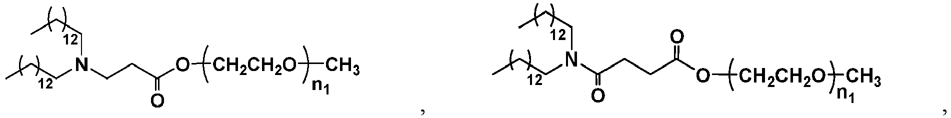 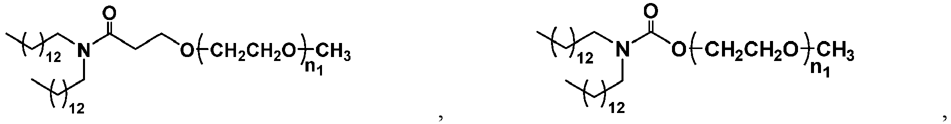 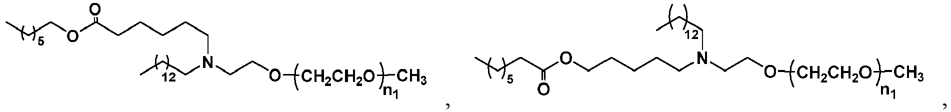 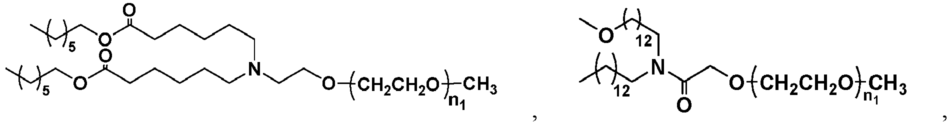 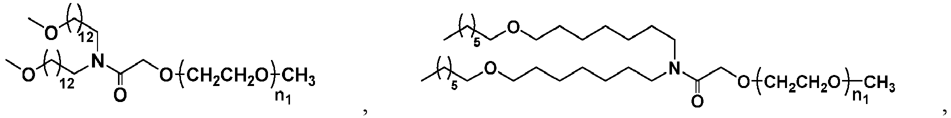 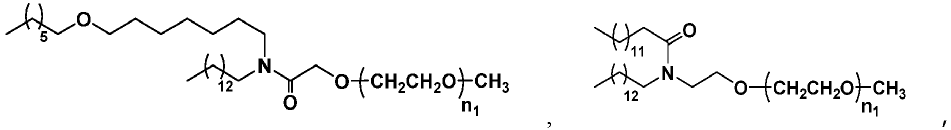 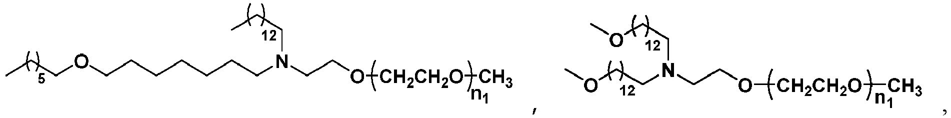 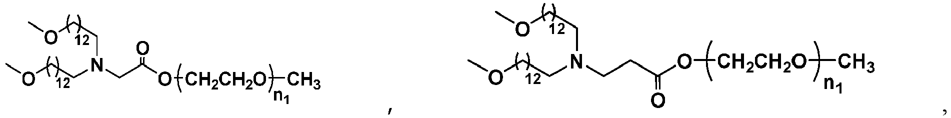 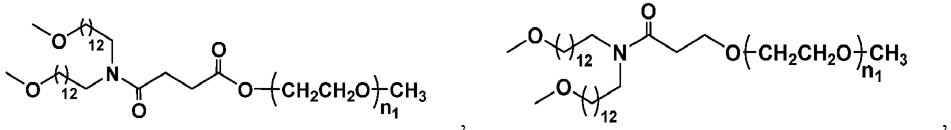 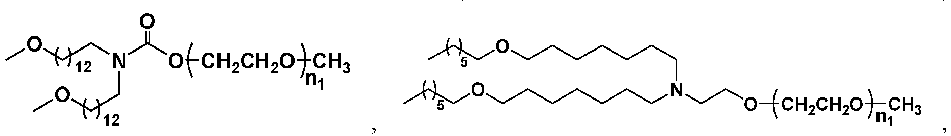 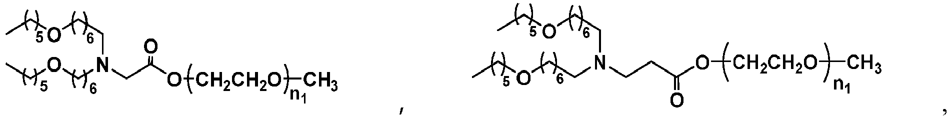 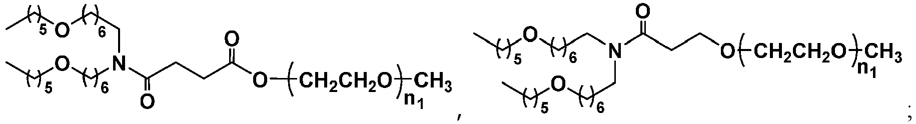 wherein, n₁ is an integer in the range of 20 to 250.

28. The lipid composition of claim **22,** wherein,
the molar percentage of PEGylated lipid in total lipids ranges from 0.5 to 5%, preferably ranges from 1 to 3%, and is more preferably 1.5%, 1.6%, 1.7%, 1.8%, or 1.9%;
the molar percentage of cationic lipid in total lipids ranges from 30 to 65%, and is preferably 35%, 40%, 45%, 46%, 47%, 48%, 49%, 50%, or 55%;
the molar percentage of phospholipid in total lipids ranges from 7.5 to 13%, and is preferably 8%, 9%, 10%, 11%, or 12%;
the molar percentage of steroid lipid in total lipids ranges from 35 to 50%, and is preferably 40%, 41%, 42%, 43%, 44%, 45%, 46%, 47%, 48%, 49%, or 50%.

29. A lipid pharmaceutical composition containing a lipid composition of any one of claims **21** to **28** and a drug selected from the group consisting of nucleic acid drug, genetic vaccine, anti-neoplastic drug, small molecule drug, peptide drug, and protein drug.

30. The lipid pharmaceutical composition of claim **29,** wherein said drug is a nucleic acid drug selected from the group consisting of DNA, RNA, antisense nucleic acid, plasmid, interfering nucleic acid, aptamer, antagomir, and ribozyme, wherein said RNA is selected from the group consisting of mRNA, saRNA, circRNA, miRNA, and siRNA; said nucleic acid drug is preferably selected from the group consisting of DNA, mRNA, miRNA, and siRNA.

31. The lipid pharmaceutical composition of claim **29,** which is used as a medicine selected from the group consisting of a drug for treating cancer, an anti-infective agent, an antibiotic agent, an antiviral agent, an antifungal agent, and a vaccine.

32. The lipid pharmaceutical composition of claim **29,** which is an LNP pharmaceutical composition, preferably an LNP-nucleic acid pharmaceutical composition, and more preferably an LNP-mRNA composition.

33. A formulation of lipid pharmaceutical composition which contains a lipid pharmaceutical composition of any one of claims **29**to **32** and a pharmaceutically acceptable diluent or excipient; said diluent or excipient is preferably deionized water, ultrapure water, phosphate buffer, or physiological saline, more preferably phosphate buffer or physiological saline, and most preferably physiological saline.
